(19) Europäisches Patentamt
European Patent Office
Office européen des brevets

(11) EP 1 500 658 A1

(12) **EUROPEAN PATENT APPLICATION**
published in accordance with Art. 158(3) EPC

(43) Date of publication:
**26.01.2005 Bulletin 2005/04**

(21) Application number: **03719194.7**

(22) Date of filing: **24.04.2003**

(51) Int Cl.7: **C07D 401/06**, C07D 401/14, A61K 31/4439, A61P 1/02, A61P 1/04, A61P 1/16, A61P 9/10, A61P 11/00, A61P 13/12, A61P 19/02, A61P 19/10, A61P 27/02, A61P 29/00, A61P 35/00, A61P 37/02, A61P 43/00

(86) International application number:
**PCT/JP2003/005255**

(87) International publication number:
**WO 2003/091242 (06.11.2003 Gazette 2003/45)**

(84) Designated Contracting States:
**AT BE BG CH CY CZ DE DK EE ES FI FR GB GR HU IE IT LI LU MC NL PT RO SE SI SK TR**

(30) Priority: **26.04.2002 JP 2002126943**

(71) Applicant: **Takeda Pharmaceutical Company Limited**
**Osaka 541-0045 (JP)**

(72) Inventors:
- **KAJINO, Masahiro**
  **Toyonaka-shi, Osaka 561-0881 (JP)**
- **TAKIZAWA, Masayuki**
  **Tsukuba-shi, Ibaraki 300-2648 (JP)**
- **NOTOYA, Kohei**
  **Ibaraki-shi, Osaka 567-0895 (JP)**
- **NARA, Hiroshi**
  **Suita-shi, Osaka 565-0823 (JP)**
- **IKEMOTO, Tomomi**
  **Takarazuka-shi, Hyogo 665-0815 (JP)**
- **NISHIGUCHI, Atsuko**
  **Itami-shi, Hyogo 664-0883 (JP)**

(74) Representative:
**Rickard, Timothy Mark Adrian et al**
**Takeda Euro IP Department,**
**11-12 Charles II Street**
**London SW1Y 4QU (GB)**

(54) **NOVEL THIOL DERIVATIVE, PROCESS FOR PRODUCING THE SAME AND USE THEREOF**

(57) The present invention provides a novel thiol derivative [I] which has an excellent matrix metalloprotease inhibiting activity and is useful as a pharmaceutical composition, particularly a therapeutic agent or prophylactic agent for osteoarthritis and rheumatoid arthritis and a salt thereof, the thiol derivative being a compound represented by the formula [I]:

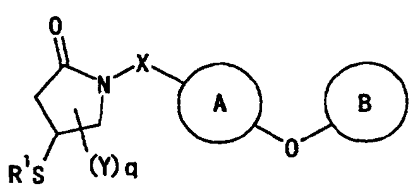

[I]

wherein ring A represents an optionally substituted aromatic heterocyclic ring;

ring B represents an optionally substituted homocyclic or heterocyclic ring;

$R^1$ represents a hydrogen atom, an optionally substituted hydrocarbon group, an acyl group, an optionally substituted heterocyclic group or $SR^2$ (wherein $R^2$ represents a hydrogen atom, an optionally substituted hydrocarbon group, an acyl group or an optionally substituted heterocyclic group);

X represents an optionally substituted divalent $C_{1-3}$ aliphatic hydrocarbon group;

Y represents an optionally substituted hydrocarbon group, a halogen atom, a carboxy group, an acyl group, an optionally substituted hydroxy group, an optionally substituted amino group, $SR^3$ (wherein $R^3$ represents a hydrogen atom, an optionally substituted hydrocarbon group, an acyl group or an optionally substituted heterocyclic group), an oxo group, a thioxo group, an optionally substituted imino group, a nitro group or a cyano group; and

q represents an integer of 0 to 5.

EP 1 500 658 A1

## Description

## Technical Field

The present invention relates to a novel thiol derivative which has an excellent matrix metalloprotease inhibiting activity, and is useful as a therapeutic agent or prophylactic agent for osteoarthritis and rheumatoid arthritis and also as an agent for inhibiting the metastasis, infiltration and proliferation of various cancers.

## Background Art

A matrix metalloprotease (MMP) is an endopeptidase playing a physiologically important role in tissue remodelling, and its protease activity is under strict control. However, such control is disturbed in a pathological state to induce an excessive degradation of an extracellular matrix, thus being involved pathogenically in many diseases, for example, an articular disease such as osteoarthritis and rheumatoid arthritis, a bone disease such as osteoporosis, periodontosis, tumor infiltration or metastasis, corneal ulceration, and the like.

At least 26 types of MMPs are presently known, and they are classified into 5 groups consisting of the collagenase group (MMP-1, 8, 13, 18), the gelatinase group (MMP-2, 9), the stromelysin group (MMP-3, 10, 11), the membrane-type MMP group (MMP-14, 15, 16, 17) and the miscellaneous group (MMP-7, 12), based on their primary structures and substrate specificities. Among these groups, MMP-13 in the collagenase group is reported to be expressed exclusively in the cartilage and bone tissues and produced at a higher level in articular diseases.

In addition, MMP-13 is assumed to be deeply involved in bone or articular diseases due to its higher collagen degrading activity when compared with other collagenases.

A large number of MMP inhibitors have been reported (Current Pharmaceutical Design, 2, 624-661 (1996)), Expert Opinion on Therapeutic Patents, 6, 1305-1315 (1996), Chem. Rev. 99, 2735-2776 (1999), Current Medicinal Chemistry, B, 425-474 (2001)). Those also widely reported are the compounds exhibiting inhibitory activities on MMP-13, which are classified broadly into (i) hydroxamic acid derivatives (British Journal of Pharmacology, 121, 540-546 (1997), WO97-31892, WO98-15525, WO98-16506, WO98-16520, Journal of Medicinal Chemistry, 43, 2293-2296 (2000), Bioorganic & Medicinal Chemistry Letters, 11, 1211-1213 (2001)), (ii) carboxylic acid derivatives (Journal of Clinical Investigation, 99, 1534-1545 (1997), WO98-6711, WO98-9934, WO98-17643) and (iii) thiol derivatives (WO97-48685, WO98-3164, WO98-3166, WO98-08814, Bioorganic & Medicinal Chemistry Letters, 9, 943-948 (1999), WO00-17162).

There is a need for the development of a novel compound which is excellent in efficacy, durability, safety, oral absorption or the like when compared with the conventional MMP inhibitors and is useful as a therapeutic agent or a prophylactic agent for articular diseases (osteoarthritis, rheumatoid arthritis, etc.), osteoporosis, cancers, periodontosis, corneal ulcer and other MMP-related diseases.

## Disclosure of Invention

The present inventors have conducted extensive research and discovered that, due to the chemical structure characterized substantially by the substitution of the nitrogen atom on a ring represented by the following formula:

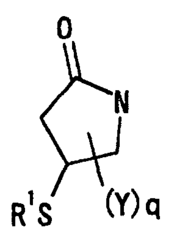

wherein $R^1$ represents a hydrogen atom, an optionally substituted hydrocarbon group, an acyl group, an optionally substituted heterocyclic group or $SR^2$ (wherein $R^2$ represents a hydrogen atom, an optionally substituted hydrocarbon group, an acyl group or an optionally substituted heterocyclic group);

each Y may be the same or different and represents an optionally substituted hydrocarbon group, a halogen atom, a carboxy group, an acyl group, an optionally substituted hydroxy group, an optionally substituted amino group, $SR^3$ (wherein $R^3$ represents a hydrogen atom, an optionally substituted hydrocarbon group, an acyl group or an optionally substituted heterocyclic group), an oxo group, a thioxo group, an optionally substituted imino group, a nitro group or a cyano group; and

q represents an integer of 0 to 5,

with a group represented by the following formula:

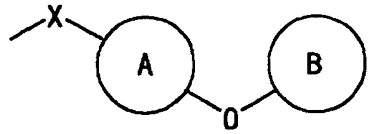

wherein ring A represents an optionally substituted aromatic heterocyclic ring;

ring B represents an optionally substituted homocyclic or heterocyclic ring; and

X represents an optionally substituted divalent $C_{1-3}$ aliphatic hydrocarbon group,

a compound represented by the following formula [I]:

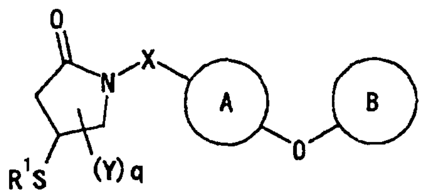

[I]

wherein ring A represents an optionally substituted aromatic heterocyclic ring;

ring B represents an optionally substituted homocyclic or heterocyclic ring;

$R^1$ represents a hydrogen atom, an optionally substituted hydrocarbon group, an acyl group, an optionally substituted heterocyclic group or $SR^2$ (wherein $R^2$ represents a hydrogen atom, an optionally substituted hydrocarbon group, an acyl group or an optionally substituted heterocyclic group);

X represents an optionally substituted divalent $C_{1-3}$ aliphatic hydrocarbon group;

each Y may be the same or different and represents an optionally substituted hydrocarbon group, a halogen atom, a carboxy group, an acyl group, an optionally substituted hydroxy group, an optionally substituted amino group, $SR^3$ (wherein $R^3$ represents a hydrogen atom, an optionally substituted hydrocarbon group, an acyl group or an optionally substituted heterocyclic group), an oxo group, a thioxo group, an optionally substituted imino group, a nitro group or a cyano group; and

q represents an integer of 0 to 5,

or a salt thereof unexpectedly exhibits an excellent MMP inhibiting effect (especially an MMP13 inhibiting effect) resulting from its specific chemical structure in combination with excellent durability, safety, and oral absorption, and such pharmacological effects are useful in a prophylactic and therapeutic agent for osteoarthritis, rheumatoid arthritis, osteoporosis, cancer, periodontosis or corneal ulcer, thus completing the invention.

That is, the present invention relates to:

[1] A compound represented by the formula [I]:

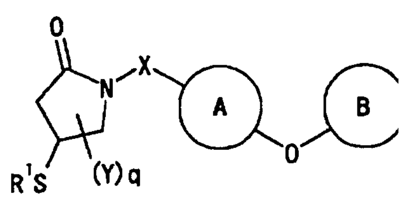

[I]

wherein ring A represents an optionally substituted aromatic heterocyclic ring;

ring B represents an optionally substituted homocyclic or heterocyclic ring;

$R^1$ represents a hydrogen atom, an optionally substituted hydrocarbon group, an acyl group, an optionally substituted heterocyclic group or $SR^2$ (wherein $R^2$ represents a hydrogen atom, an optionally substituted hydrocarbon group, an acyl group or an optionally substituted heterocyclic group);

X represents an optionally substituted divalent $C_{1-3}$ aliphatic hydrocarbon group;

each Y may be the same or different and represents an optionally substituted hydrocarbon group, a halogen atom, a carboxy group, an acyl group, an optionally substituted hydroxy group, an optionally substituted amino group, $SR^3$ (wherein $R^3$ represents a hydrogen atom, an optionally substituted hydrocarbon group, an acyl group or an optionally substituted heterocyclic group), an oxo group, a thioxo group, an optionally substituted imino group, a nitro group or a cyano group; and

q represents an integer of 0 to 5,

or a salt thereof,

[2] The compound according to the above [1], wherein X is an optionally substituted methylene group,

[3] The compound according to the above [1], wherein $R^1$ represents a hydrogen atom, an optionally substituted lower alkyl group, -(C=O)-$R^4$ or -(C=S)-$R^4$ (wherein $R^4$ represents a hydrogen atom, an optionally substituted hydrocarbon group, an optionally substituted amino group or an optionally substituted hydroxy group) or $SR^2$ (wherein $R^2$ has the same meaning as defined above),

[4] The compound according to the above [1], wherein $R^1$ is (1) a hydrogen atom, (2) a $C_{1-6}$ alkyl group optionally substituted by 1 to 3 substituents selected from (i) a $C_{1-6}$ alkoxy-carbonyl group, (ii) a $C_{6-14}$ aryl group, (iii) a carboxy group, (iv) an amino group, (v) a mono- or di-$C_{1-6}$ alkylamino group, (vi) a $C_{1-6}$ alkyl-carbonylamino group, (vii) a $C_{1-6}$ alkoxy-carbonylamino group, (viii) a $C_{1-6}$ alkyl-carbonyloxy group and (ix) a heterocyclic group, (3) -(C=O) -$R^{6a}$ or -(C=S)-$R^{6a}$ (wherein $R^{6a}$ represents a $C_{1-6}$ alkyl group, a $C_{2-6}$ alkenyl group or a $C_{6-14}$ aryl group each optionally substituted by 1 to 3 substituents selected from (i) a carboxy group, (ii) a $C_{1-6}$ alkyl-carbonylamino group, (iii) a $C_{1-6}$ alkyl-carbonyloxy group and (iv) a $C_{1-6}$ alkoxy-carbonyl group) , (4) - (C=O) -$NR^{6a}R^{27a}$ or - (C=S) -$NR^{6a}R^{27a}$ (wherein $R^{6a}$ has the same meaning as defined above and $R^{27a}$ represents a hydrogen atom or a $C_{1-6}$ alkyl group), or (5) $SR^{2a}$ (wherein $R^{2a}$ represents (i) a $C_{1-6}$ alkyl group or a $C_{6-14}$ aryl group each optionally substituted by 1 to 3 substituents selected from (a) a carboxy group, (b) an amino group and (c) a $C_{1-6}$ alkyl-carbonylamino group, or (ii) a $C_{1-6}$ alkoxy-carbonyl group),

[5] The compound according to the above [1], wherein ring A is an optionally substituted pyridine ring,

[6] The compound according to the above [1], wherein ring A is the following formula:

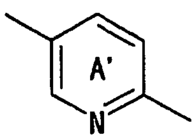

or

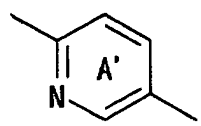

wherein ring A' represents an optionally substituted pyridine ring,

[7] The compound according to the above [1], wherein X is a methylene group optionally substituted by 1 or 2 substituents selected from (1) a halogen atom, (2) a $C_{1-4}$ alkyl group or $C_{1-4}$ alkoxy group each optionally substituted by 1 or 2 substituents selected from (i) a halogen atom, (ii) $C_{1-4}$ alkoxy group, (iii) a nitro group, (iv) a cyano group, (v) a hydroxy group, (vi) an amino group, (vii) a mono- or di-$C_{1-4}$ alkylamino group, (viii) a carboxy group, (ix) a $C_{1-4}$ alkoxy-carbonyl group and (x) $C_{1-4}$ alkyl-carbonyl group, (3) a nitro group, (4) a cyano group, (5) a hydroxy group, (6) an amino group, (7) a mono- or di-$C_{1-4}$ alkylamino group, (8) a carboxy group, (9) a $C_{1-4}$ alkoxy-carbonyl group and (10) a $C_{1-4}$ alkyl-carbonyl group, an oxo group or a thioxo group;

$R^1$ is (1) a hydrogen atom, (2) a $C_{1-6}$ alkyl group optionally substituted by 1 to 3 substituents selected from (i) a $C_{1-6}$ alkoxy-carbonyl group, (ii) a $C_{6-14}$ aryl group, (iii) a carboxy group, (iv) an amino group, (v) a mono- or di-$C_{1-6}$ alkylamino group, (vi) a $C_{1-6}$ alkyl-carbonylamino group, (vii) a $C_{1-6}$ alkoxy-carbonylamino group, (viii) a $C_{1-6}$ alkyl-carbonyloxy group and (ix) a heterocyclic group, (3) - (C=O) -$R^{6a}$ or - (C=S) -$R^{6a}$ (wherein $R^{6a}$ represents a $C_{1-6}$ alkyl group, a $C_{2-6}$ alkenyl group or a $C_{6-14}$ aryl group each optionally substituted by 1 to 3 substituents selected from (i) a carboxy group, (ii) a $C_{1-6}$ alkyl-carbonylamino group, (iii) a $C_{1-6}$ alkyl-carbonyloxy group and (iv) a $C_{1-6}$ alkoxy-carbonyl group, (4)-(C=O)-$NR^{6a}R^{27a}$ or - (C=S) -$NR^{6a}R^{27a}$ (wherein $R^{6a}$ has the same meaning as defined above and $R^{27a}$ represents a hydrogen atom or a $C_{1-6}$ alkyl group), or (5) $SR^{2a}$ (wherein $R^{2a}$ represents (i) a $C_{1-6}$ alkyl group or a $C_{6-14}$ aryl group each optionally substituted by 1 to 3 substituents selected from (a) a carboxy group, (b) an amino group and (c) a $C_{1-6}$ alkyl-carbonylamino group, or (ii) a $C_{1-6}$ alkoxy-carbonyl group) ;

ring A is a pyridine ring optionally substituted by 1 to 3 substituents selected from (i) a halogen atom, (ii) an

optionally halogenated $C_{1-4}$ alkyl group and (iii) an optionally halogenated $C_{1-4}$ alkoxy group;

ring B is a benzene ring optionally substituted by 1 to 3 substituents selected from (i) a halogen atom, (ii) a $C_{1-6}$ alkyl group and (iii) a $C_{1-6}$ alkylsulfonylamino group;

Y is (1) a $C_{1-6}$ alkyl group optionally substituted by (i) a hydroxy group or (ii) a $C_{1-6}$ alkoxy group, or (2) an oxo group; and

q is 0 or 1,

[8] The compound according to the above [1], wherein the formula [I] is represented by the following formula:

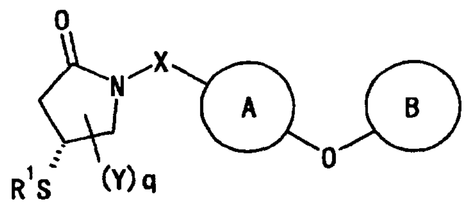

wherein each symbol has the same meaning as defined above,

[9] (4R)-1-{[6-(4-fluorophenoxy)pyridin-3-yl]methyl}-4-mercaptopyrrolidin-2-one or a salt thereof,

S-((3R)-1-{[6-(4-fluorophenoxy)pyridin-3-yl]methyl}-5-oxopyrrolidin-3-yl)methylthiocarbamate or a salt thereof,

(3R)-1-{[6-(4-fluorophenoxy)pyridin-3-yl]methyl}-5-oxopyrrolidin-3-yl methyl-(dithiocarbamate) or a salt thereof,

(4R)-1-{[6-(4-ethylphenoxy)pyridin-3-yl]methyl}-4-mercaptopyrrolidin-2-one or a salt thereof,

S-((3R)-1-{[6-(4-ethylphenoxy)pyridin-3-yl]methyl}-5-oxopyrrolidin-3-yl)methylthiocarbamate or a salt thereof,

N-{4-[(5-[(4R)-4-mercapto-2-oxopyrrolidin-1-yl]methyl)pyridin-2-yl]oxy}phenyl}methanesulfonamide or a salt thereof,

S-{(3R)-1-[(6-{4-[(methylsulfonyl)amino]phenoxy}pyridin-3-yl)methyl]-5-oxopyrrolidin-3-yl}methylthiocarbamate or a salt thereof, or

(3R)-1-((6-(4-fluorophenoxy)-3-pyridinyl)methyl)-5-oxo-3-pyrrolidinyl aryldithiocarbamate or a salt thereof,

[10] A method for producing a compound represented by the following formula:

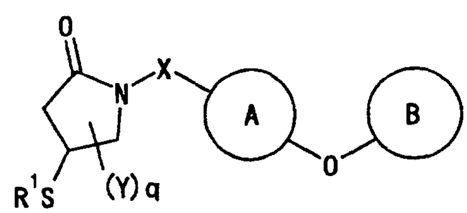

wherein each symbol has the same meaning as defined above,

or a salt thereof, comprising reacting a compound represented by the following formula:

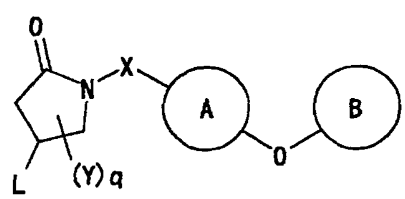

wherein L represents a leaving group and each of the other symbols has the same meaning as defined above,

or a salt thereof with a compound represented by the formula: $R^1SH$

wherein $R^1$ has the same meaning as defined above, or a salt thereof,

[11] A method for producing a compound represented by the following formula:

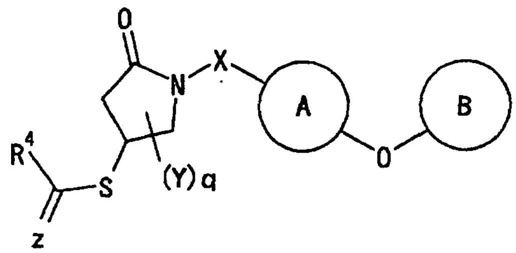

wherein Z represents O or S and each of the other symbols has the same meaning as defined above, or a salt thereof, comprising reacting a compound represented by the following formula:

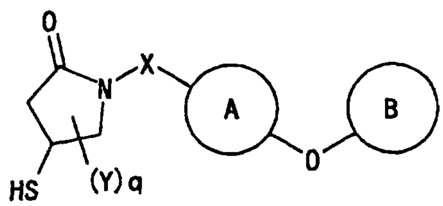

wherein each symbol has the same meaning as defined above, or a salt thereof with a compound represented by the formula:

$R^4$-$CO_2H$, $R^4$-COSH or $R^4$-CSOH

wherein $R^4$ has the same meaning as defined above, or a salt or a reactive derivative thereof,

[12] A method for producing a compound represented by the following formula:

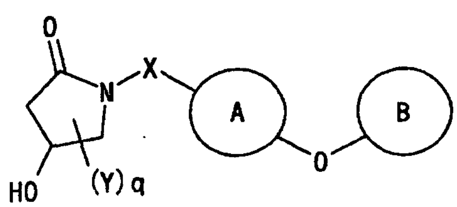

wherein each symbol has the same meaning as defined above, or a salt thereof, comprising reacting a compound represented by the following formula:

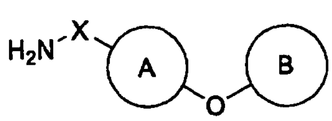

wherein each symbol has the same meaning as defined above, or a salt thereof with a compound represented by the following formula:

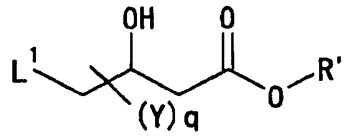

wherein $L^1$ represents a leaving group and R' represents an optionally substituted hydrocarbon group, an acyl group or an optionally substituted heterocyclic group,
or a salt thereof,
[13] A compound represented by the following formula:

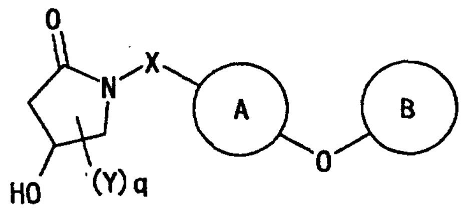

wherein each symbol has the same meaning as defined above,
or a salt thereof,
[14] A prodrug of the compound according to the above [1] ,
[15] A pharmaceutical composition comprising the compound according to the above [1] or a prodrug thereof,
[16] The pharmaceutical composition according to the above [15], which is a matrix metalloprotease inhibitor;
[17] The pharmaceutical composition according to the above [15], which is a prophylactic and therapeutic agent for matrix metalloprotease-related diseases,
[18] The pharmaceutical composition according to the above [15], which is a prophylactic and therapeutic agent for articular disease, osteoporosis, cancer, periodontosis, corneal ulcer, chronic ulcer, pathologic bone resorption, nephritis, angiogenesis, aneurysm, arteriosclerosis, pulmonary emphysema, chronic obstructive pulmonary disease (COPD), cirrhosis, autoimmune disease, or infiltration and metastasis of cancer, or a contraceptive,
[19] The pharmaceutical composition according to the above [18], wherein the articular disease include osteoarthritis or rheumatoid arthritis;
[20] A method for preventing and treating articular disease, osteoporosis, cancer, periodontosis, corneal ulcer, chronic ulcer, pathologic bone resorption, nephritis, angiogenesis, aneurysm, arteriosclerosis, pulmonary emphysema, chronic obstructive pulmonary disease (COPD), cirrhosis, autoimmune disease, or infiltration and metastasis of cancer, or for contraception, comprising administering an effective amount of the compound according to the above [1] or a prodrug thereof to a mammal; and
[21] Use of the compound according to the above [1] or a prodrug thereof for producing a prophylactic and therapeutic agent for articular disease, osteoporosis, cancer, periodontosis, corneal ulcer, chronic ulcer, pathologic bone resorption, nephritis, angiogenesis, aneurysm, arteriosclerosis, pulmonary emphysema, chronic obstructive pulmonary disease (COPD), cirrhosis, autoimmune disease, or infiltration and metastasis of cancer, or a contraceptive,

Furthermore, when the compound of the formula [I] or a salt thereof contains an asymmetric carbon in its structure, its optically active forms and racemates are also encompassed in the invention, and the compound of the formula [I] or a salt thereof may be any of a solvate, a hydrate, a non-solvate and an anhydride.
The present invention is further described below.

(1) Ring A

Ring A is an aromatic heterocyclic ring optionally having substituents.
An "aromatic heterocyclic ring" includes, for example, a 5- or 6-membered aromatic heterocyclic ring containing, in addition to carbon atoms, 1 to 3 hetero atoms consisting of 1 or 2 kinds of hetero atoms selected from a nitrogen atom, a sulfur atom and an oxygen atom (e.g., pyridine, pyrazine, pyrimidine, pyridazine, pyrrole, imidazole, pyrazole, triazole, thiophene, furan, thiazole, isothiazole, oxazole, isoxazole, etc.) and preferably includes a 6-membered nitrogen-containing aromatic heterocyclic ring containing, in addition to carbon atoms, 1 or 2 nitrogen atoms (e.g., pyridine, pyrazine, pyrimidine rings, etc.), a pyridine ring being the most preferred.
For an "aromatic heterocyclic ring" represented by ring A, a group represented by the following formula [I']:

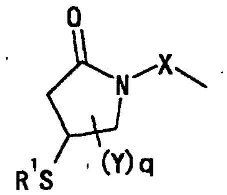

[I']

which is a partial structure of the compound represented by the formula [I] and a group represented by the following formula [I"]:

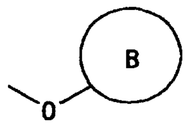

[I"]

which is a partial structure of the compound by represented by the formula [I] may be substituted on any position capable of being substituted on a ring A, respectively.

For example, when ring A is a pyridine ring, it may be bound to X in a partial structure represented by the formula [I'] and an oxygen atom in a partial structure represented by the formula [I"] at any of substitution positions represented by the following formulae:

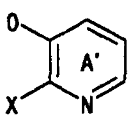
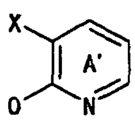
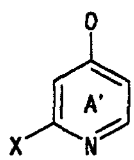
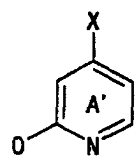
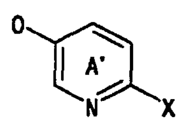

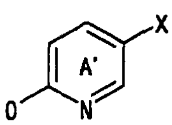
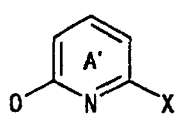
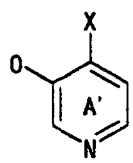
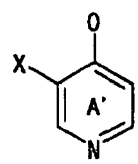
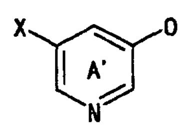

wherein ring A' represents an optionally substituted pyridine ring, X represents X in a partial structure represented by the formula [I'], and O represents an oxygen atom in a partial structure represented by the formula [I"], and preferred are substitution positions represented by the following formulae:

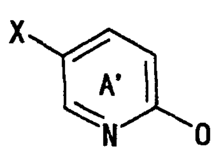
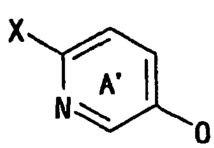

wherein each symbol has the same meaning as defined above, particularly preferred being a substitution position represented by the following formula:

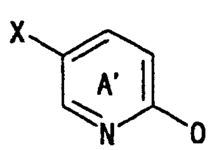

wherein each symbol has the same meaning as defined above.

The substituents that may be contained in the "optionally substituted aromatic heterocyclic ring" represented by ring A ("optionally substituted pyridine ring" represented by ring A') include, for example:

(i) a halogen atom (e.g., fluorine, chlorine, bromine, iodine, etc.);
(ii) an optionally substituted alkyl group;
(iii) an optionally halogenated alkoxy group (e.g., a $C_{1-6}$ alkoxy group which is substituted optionally with a halogen atom such as fluorine and chlorine, such as methoxy, difluoromethoxy, trichloromethoxy, tifluoromethoxy, ethoxy, 2,2,2-trifluoroethoxy, perfluoroethoxy, propoxy, isopropoxy, butoxy, isobutoxy, tert-butoxy, perfluorobutoxy, pentyloxy, hexyloxy, etc.);
(iv) an optionally halogenated alkylthio group (e.g., a $C_{1-6}$ alkylthio group (especially a $C_{1-4}$ alkylthio group) which is substituted optionally with a halogen atom such as fluorine and chlorine, such as methylthio, difluoromethylthio, trifluoromethylthio, ethylthio, propylthio, isopropylthio, butylthio, etc.);
(v) an aryl group (e.g., a $C_{6-14}$ aryl group such as phenyl, naphthyl, anthryl, phenanthryl, etc.);
(vi) an acyloxy group (e.g., a $C_{1-3}$ acyloxy group such as formyloxy, acetoxy, propionyloxy, etc.);
(vii) a hydroxy group;
(viii) a nitro group;
(ix) a cyano group;
(x) an amino group;
(xi) a mono- or dialkylamino group (e.g., a mono- or di-$C_{1-6}$ alkylamino group (especially a mono- or di-$C_{1-4}$ alkylamino group) such as methylamino, ethylamino,: propylamino, dimethylamino, diethylamino, etc.) ;
(xii) a cyclic amino group (e.g., a 5- to 9-membered cyclic amino group optionally containing, in addition to nitrogen atoms, 1 to 3 hetero atoms such as an oxygen atom and a sulfur atom (e.g., pyrrolidino, piperidino, morpholino, etc.), etc.);
(xiii) an acylamino group (e.g., a $C_{1-6}$ alkylcarbonylamino group such as formylamino, or acetylamino, propionylamino, butyloylamino, etc.);
(xiv) a lower alkyl-substituted carbamoylamino group (e.g., an ethylcarbamoylamino group, etc.);
(xv) an alkylsulfonylamino group (e.g., a $C_{1-6}$ alkylsulfonylamino group such as methylsulfonylamino, ethylsulfonylamino, propylsulfonylamino, etc.);
(xvi) an alkoxycarbonyl group (e.g., a $C_{1-6}$ alkoxycarbonyl group such as methoxycarbonyl, ethoxycarbonyl, propoxycarbonyl, butoxycarbonyl, isobutoxycarbonyl, etc.);
(xvii) a carboxy group;
(xviii) an alkylcarbonyl group (e.g., a $C_{1-6}$ alkylcarbonyl group such as methylcarbonyl, ethylcarbonyl, butylcarbonyl, etc.);
(xix) a carbamoyl group;
(xx) a mono- or dialkylcarbamoyl group (e.g., a mono- or di-$C_{1-6}$ alkylcarbamoyl group such as methylcarbamoyl, ethylcarbamoyl, propylcarbamoyl, butylcarbamoyl, diehylcarbamoyl, dibutylcarbamoyl, etc.);
(xxi) an alkylsulfonyl group (e.g., a $C_{1-6}$ alkylsulfonyl group such as methylsulfonyl, ethylsulfonyl, propylsulfonyl, etc.);
(xxii) an oxo group; and
(xxiii) a thioxo group.

The above-mentioned "optionally substituted alkyl group" includes, for example,

(a) in addition to a straight or branched alkyl group having 1 to 6 carbon atoms, such as methyl, ethyl, propyl, isopropyl, butyl, isobutyl, sec-butyl, and tert-butyl;
(b) a halogenated $C_{1-6}$ alkyl group (e.g., chloromethyl, difluoromethyl, trichloromethyl, trifluoromethyl, 2-bromoethyl, 2,2,2-trifluoroethyl, perfluoroethyl, 3,3,3-trifluoropropyl, perfluorobutyl, etc.);
(c) an amino group-substituted $C_{1-6}$ alkyl group (e.g., aminomethyl, 2-aminoethyl, etc.);
(d) a mono- or di-$C_{1-6}$ alkylamino group-substituted $C_{1-6}$ alkyl group (e.g., methylaminomethyl, dimethylaminomethyl, 2-methylaminoethyl, 2-dimethylaminoethyl, etc.);
(e) a carboxy group-substituted $C_{1-6}$ alkyl group(e.g., carboxymethyl, carboxyethyl, etc.);
(f) a $C_{1-6}$ alkoxycarbonyl group-substituted $C_{1-6}$ alkyl group (e.g., methoxycarbonylethyl, ethoxycarbonylethyl, etc.);
(g) a hydroxy group-substituted $C_{1-6}$ alkyl group (e.g., hydroxymethyl, hydroxyethyl, etc.);
(h) a $C_{6-14}$ aryl group-substituted $C_{1-6}$ alkyl group (e.g., benzyl, etc.);
(i) a $C_{1-6}$ alkoxy group-substituted $C_{1-6}$ alkyl group (e.g., methoxymethyl, methoxyethyl, etc.); or
(j) a $C_{7-15}$ aralkyloxy group-substituted $C_{1-6}$ alkyl group (e.g., benzyloxymethyl, etc.).

That is, mention may be made of a $C_{1-6}$ alkyl group optionally substituted by 1 to 4 substituents selected from halogen, amino, mono- or di-$C_{1-6}$ alkylamino, carboxy, $C_{1-6}$ alkoxycarbonyl, hydroxy, $C_{6-14}$ aryl, $C_{1-6}$ alkoxy and $C_{7-15}$ aralkyloxy.

A preferred substituent that may be contained in ring A (ring A') includes, for example, a halogen atom, an optionally substituted alkyl group, an optionally halogenated alkoxy group, a mono- or dialkylamino group, an optionally halogenated alkylthio group, a nitro group, and a cyano group.

A more preferred substituent that may be contained in ring A (ring A') includes, for example, a halogen atom, an optionally substituted $C_{1-4}$ alkyl group, an optionally halogenated $C_{1-4}$ alkoxy group, a mono- or di-$C_{1-4}$ alkylamino group, an optionally halogenated $C_{1-4}$ alkylthio group, a nitro group, and a cyano group.

Among these, particularly preferred are 1 to 3 substituents selected from a halogen atom, an optionally halogenated $C_{1-4}$ alkyl group and an optionally halogenated $C_{1-4}$ alkoxy group.

A substituent on ring A (ring A') may be on any position capable of being substituted on the ring, and when two or more substituents are present, they may be the same or different. The number of the substituents may be 1 to about 4, preferably 1 to about 3.

When ring A (ring A') contains a nitrogen atom, it may form a quaternary ammonium salt, for example, a salt together with an anion such as a halogen ion (e.g., $Cl^-$, $Br^-$, $I^-$, etc.), a sulfate ion, a hydroxy ion or the like.

As ring A, most preferred is the ring represented by the following formula:

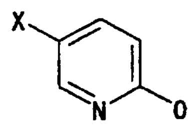

wherein the symbol has the same meaning as defined above.

(2) Ring B

Ring B represents an optionally substituted homocyclic or heterocyclic ring.

The above-mentioned "homocyclic or heterocyclic ring" includes, for example, (i) an aromatic heterocyclic ring or a non-aromatic heterocyclic ring containing, in addition to carbon atoms, preferably 1 to 3 hetero atoms consisting of 1 or 2 kinds of hetero atoms selected from a nitrogen atom, a sulfur atom and an oxygen atom or (ii) a cyclic hydrocarbon consisting of carbon atoms (homocyclic ring).

The above-mentioned "aromatic heterocyclic ring" includes, for example, those exemplified for ring A in the above.

A preferred aromatic heterocyclic ring includes, for example, (i) a 6-membered nitrogen-containing heterocyclic ring containing, in addition to carbon atoms, 1 or 2 nitrogen atoms (e.g., pyridine ring, pyrazine ring, etc.) or (ii) a 5-membered aromatic heterocyclic ring containing, in addition to carbon atoms, a sulfur atom (e.g., thiophene ring, etc.). In particular, a preferred aromatic heterocyclic ring includes, for example, pyrrole and thiazole rings, in addition to pyridine, pyrazine and thiophene rings.

The above-mentioned "non-aromatic heterocyclic ring" includes, for example, a 5- to 9-membered non-aromatic heterocyclic ring, preferably a 5- or 6-membered non-aromatic heterocyclic ring containing, in addition to carbon atoms, 1 to 3 hetero atoms selected from a nitrogen atom, an oxygen atom and a sulfur atom.

Specific examples of the "non-aromatic heterocyclic ring" include tetrahydropyridine, dihydropyridine, tetrahydropyrazine, tetrahydropyrimidine, tetrahydropyridazine, dihydropyran, dihydropyrrole, dihydroimidazole, dihydropyrazole, dihydrothiophene, dihydrofuran, dihydrothiazole, dihydroisothiazole, dihydrooxazole, dihydroisoxazole, piperidine, piperazine, hexahydropyrimidine, hexahydropyridazine, tetrahydropyran, morpholine, pyrrolidine, imidazolidine, pyrazolidine, tetrahydrothiophene, tetrahydrofuran, tetrahydrothiazole, tetrahydroisothiazole, tetrahydroxazole, and tetrahydroisoxazole rings.

The above-mentioned "cyclic hydrocarbon (homocyclic ring) consisting of carbon atoms" includes, for example, a 3- to 10-membered (preferably 5- to 9-membered) cyclic hydrocarbon, more preferably a 5- or 6-membered cyclic hydrocarbon. Mention may be made of, for example, benzene, a $C_{3-10}$ cycloalkene (e.g., cyclobutene, cyclopentene, cyclohexene, cycloheptene, cyclooctene, etc.), a $C_{3-10}$ cycloalkane (e.g., cyclobutane, cyclopentane, cyclohexane, cycloheptane, cyclooctane, etc.) or the like. A cycloalkene is preferably a $C_{5-6}$ cycloalkene (e.g., cyclopentene, cyclohexene, etc.), while a cycloalkane is preferably a $C_{5-6}$ cycloalkane (e.g., cyclohexane, cyclopentane, etc.). For ring B, preferred is, for example, a 6-membered homocyclic ring such as benzene ring and cyclohexene ring, and particularly preferred is a benzene ring.

For a substituent that may be contained in a "homocyclic or heterocyclic ring" represented by ring B, mention may be made of those exemplified as the substituents for ring A in the above.

A substituent on ring B may be on any position capable of being substituted on the ring, and when two or more substituents are present, they may be the same or different and the number of the substituents may be 1 to about 4. The number of the substituents is preferably 1 to about 3.

Ring B is a preferably benzene ring optionally substituted by 1 to 3 substituents selected from a halogen atom, a $C_{1-6}$ alkyl group and a $C_{1-6}$ alkylsulfonylamino group, and more preferably a ring represented by the following formula:

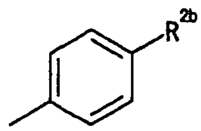

wherein $R^{2b}$ represents a hydrogen atom, a halogen atom, a $C_{1-6}$ alkyl group or a $C_{1-6}$ alkylsulfonylamino group.

(3) "Group $R^1$"

$R^1$ represents a hydrogen atom, an optionally substituted hydrocarbon group, an acyl group, an optionally substituted heterocyclic group or $SR^2$ (wherein $R^2$ represents a hydrogen atom, an optionally substituted hydrocarbon group, an acyl group or an optionally substituted heterocyclic group).

A "hydrocarbon group" in an "optionally substituted hydrocarbon group" represented by $R^1$, includes, for example, an alkyl group, an alkenyl group, an alkynyl group, a cycloalkyl group and an aryl group, and preferably an alkyl group, a cycloalkyl group and an aryl group, and particularly an alkyl group is widely used.

As for an "alkyl group", a straight or branched alkyl group having 1 to 6 carbon atoms, preferably a straight or branched alkyl group having 1 to 4 carbon atoms such as methyl, ethyl, propyl, isopropyl, butyl, isobutyl, sec-butyl and tert-butyl, may be used.

As for an "alkenyl group", an alkenyl group having 2 to 6 carbon atoms such as ethenyl, propenyl, isopropenyl, butenyl, isobutenyl, and sec-butenyl, preferably an alkenyl group having 2 to 4 carbon atoms such as ethenyl, propenyl, and isopropenyl, may be used.

As for an "alkynyl group", an alkynyl group having 2 to 6 carbon atoms such as ethynyl, propynyl, butynyl, and isobutynyl, preferably an alkynyl group having 2 to 4 carbon atoms such as ethynyl and propynyl, may be used.

As for a "cycloalkyl group", a $C_{3-8}$ cycloalkyl group such as cyclopropyl, cyclobutyl, cyclopentyl, cyclohexyl, cycloheptyl, and cyclooctyl, preferably a $C_{3-6}$ cycloalkyl group such as cyclopropyl, cyclobutyl, cyclopentyl, and cyclohexyl, may be used.

As for an "aryl group", an aryl group having 6 to 14 carbon atoms such as phenyl, naphthyl, anthryl, and phenanthryl, preferably an aryl group having 6 to 10 carbon atoms such as phenyl and naphthyl, may be used.

A "substituent" on an "optionally substituted hydrocarbon group" represented by $R^1$ include, for example:

(i) a halogen atom (e.g., fluorine, chlorine, bromine, iodine, etc.);
(ii) an optionally substituted alkyl group;
(iii) a cycloalkyl group (e.g., a $C_{3-8}$ cycloalkyl group such as cyclopropyl, cyclobutyl, cyclopentyl, cyclohexyl, etc.);
(iv) an optionally substituted aryl group;
(v) an optionally halogenated alkoxy group (e.g., a $C_{1-6}$ alkoxy group which is substituted optionally with a halogen atom such as fluorine and chlorine, such as methoxy, difluoromethoxy, trichloromethoxy, trifluoromethoxy, ethoxy, 2,2,2-trifluoroethoxy, perfluoroethoxy, propoxy, isopropoxy, butoxy, isobutoxy, tert-butoxy, perfluorobutoxy, pentyloxy, hexyloxy, $C_{3-8}$ cycloalkyloxy, etc.);
(vi) a nitro group;
(vii) a cyano group;
(viii) a hydroxy group;
(ix) an aryloxy group (e.g., a $C_{6-14}$ aryloxy group such as phenoxy, naphthyloxy, etc.);
(x) an aralkyloxy group (e.g., a $C_{6-14}$ aryl group-$C_{1-4}$ alkyloxy group such as benzyloxy, phenethyloxy, etc.);
(xi) an optionally halogenated alkylthio group (e.g., $C_{1-6}$ alkylthio group (especially a $C_{1-4}$ alkylthio group) which is substituted optionally with a halogen atom such as fluorine and chlorine, such as methylthio, difluoromethylthio, trifluoromethylthio, ethylthio, propylthio, isopropylthio, butylthio, etc.);
(xii) an amino group;
(xiii) an amino group which is a mono- or disubstituted by a formyl group or an optionally substituted alkyl group (e.g., a mono- or di-$C_{1-6}$ alkylamino group (especially a mono- or di-$C_{1-4}$ alkylamino group) such as methylamino,

ethylamino, propylamino, dimethylamino, and diethylamino, a formylamino group, a pyrimidinylmethylamino group, etc.);
(xiv) a cyclic amino group (e.g., a 5- or 9-membered cyclic amino group optionally containing, in addition to nitrogen atoms, 1 to 3 hetero atoms such as an oxygen atom and a sulfur atom, such as pyrrolidino, piperidino, morpholino, etc.);
(xv) an alkylcarbonylamino group (e.g., a $C_{1-6}$ alkylcarbonylamino group such as acetylamino, propionylamino, butyloylamino, etc.);
(xvi) an alkoxycarbonylamino group (e.g., a $C_{1-6}$ alkoxycarbonylamino group such as ethoxycarbonylamino, etc.);
(xvii) an arylcarbonylamino group (e.g., a $C_{6-14}$ arylcarbonylamino group such as benzoylamino, etc.);
(xviii) an acyloxy group (e.g., a $C_{1-3}$ acyloxy group such as formyloxy, acetoxy, propionyloxy, etc.);
(xix) an aminocarbonyloxy group;
(xx) a mono- or dialkylaminocarbonyloxy group (e.g., a mono- or di-$C_{1-6}$ alkylaminocarbonyloxy group such as methylaminocarbonyloxy, ethylaminocarbonyloxy, dimethylaminocarbonyloxy, diethylaminocarbonyloxy, etc.);
(xxi) an alkylsulfonylamino group (e.g., a $C_{1-6}$ alkylsulfonylamino group such as methylsulfonylamino, ethylsulfonylamino, propylsulfonylamino, etc.);
(xxii) an alkoxycarbonyl group (e.g., a $C_{1-6}$ alkoxycarbonyl group such as methoxycarbonyl, ethoxycarbonyl, propoxycarbonyl, butoxycarbonyl, isobutoxycarbonyl, etc.);
(xxiii) an aralkyloxycarbonyl group (e.g., a $C_{7-15}$ aralkyloxycarbonyl group such as benzyloxycarbonyl, etc.);
(xxiv) an aryloxycarbonyl group (e.g., a $C_{6-14}$ aryloxycarbonyl group such as phenoxycarbonyl, etc.);
(xxv) a carboxy group;
(xxvi) an alkylcarbonyl group (e.g., a $C_{1-6}$ alkylcarbonyl group such as methylcarbonyl, ethylcarbonyl, butylcarbonyl, etc.);
(xxvii) a cycloalkylcarbonyl group (e.g., a $C_{3-8}$ cycloalkylcarbonyl group such as cyclopentylcarbonyl, cyclohexylcarbonyl, etc.);
(xxviii) an arylcarbonyl group (e.g., a $C_{6-14}$ arylcarbonyl group such as benzoyl, etc.);
(xxix) a carbamoyl group;
(xxx) a thiocarbamoyl group;
(xxxi) a mono- or dialkylcarbamoyl group (e.g., a mono- or di-$C_{1-6}$ alkylcarbamoyl group such as methylcarbamoyl, ethylcarbamoyl, propylcarbamoyl, butylcarbamoyl, diethylcarbamoyl, dibutylcarbamoyl, etc.);
(xxxii) an alkylsulfonyl group (e.g., a $C_{1-6}$ alkylsulfonyl group such as methylsulfonyl, ethylsulfonyl, propylsulfonyl, etc.);
(xxxiii) a cycloalkylsulfonyl group (e.g., a $C_{3-8}$ cycloalkylsulfonyl group such as cyclopentylsulfonyl, cyclohexylsulfonyl, etc.);
(xxxiv) an arylsulfonyl group (e.g., a $C_{6-14}$ arylsulfonyl group such as phenylsulfonyl, naphthylsulfonyl, etc.);
(xxxv) an aralkylsulfonyl group (e.g., a $C_{7-15}$ aralkylsulforyl group such as benzylsulfonyl, etc.);
(xxxvi) an optionally substituted 5- or 6-membered heterocyclic group; and
(xxxvii) an alkylcarbonyloxy group (e.g., a $C_{1-6}$ alkylcarbonyloxy group such as acetyloxy, propionyloxy, butyloyloxy, etc.).

An "optionally substituted 5- or 6-membered heterocyclic group" as a "substituent" on an "optionally substituted hydrocarbon group" represented by $R^1$ includes, for example, a 5- or 6-membered aromatic heterocyclic group, a saturated or unsaturated 5- or 6-membered non-aromatic heterocyclic group.

The "5- or 6-membered aromatic heterocyclic group" includes, for example, furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl; 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, and triazinyl.

The "5- or 6-membered non-aromatic heterocyclic group" includes, for example, pyrrolidinyl, tetrahydrofuryl, tetrahydrothienyl, piperidyl, tetrahydropyranyl, morpholinyl, thiomorpholinyl, and piperazinyl.

The non-aromatic heterocyclic group may be further condensed with other aromatic or non-aromatic homocyclic or heterocyclic ring.

A "substituent" on an "optionally substituted 5- or 6-membered heterocyclic group" represented as a "substituent" on an "optionally substituted hydrocarbon group" represented by $R^1$ includes, for example, one exemplified as the "substituent" on an "optionally substituted aromatic heterocyclic ring" represented by ring A described above.

Further, a group represented by the following formula:

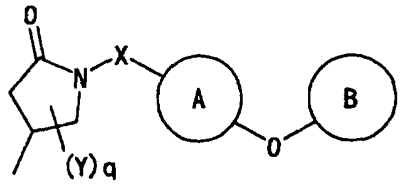

wherein each symbol has the same meaning as defined above, may also be exemplified as a "substituent" on an "optionally substituted hydrocarbon group" represented by $R^1$.

The number of substituents on an "optionally substituted hydrocarbon group" represented by $R^1$ is 1 to 5 (preferably 1 to 2), and when two or more substituents are present, they may be the same or different.

An "optionally substituted alkyl group" described as a "substituent" on an "optionally substituted hydrocarbon group" represented by $R^1$, includes the same that as the "optionally substituted alkyl group" exemplified as a substituent that may be contained in an "optionally substituted aromatic heterocyclic ring" represented by ring A described above.

As for an "aryl group" in an "optionally substituted aryl group" described as a "substituent" on an "optionally substituted hydrocarbon group" represented by $R^1$, for example, an aryl group having 6 to 14 carbon atoms such as phenyl, naphthyl, anthryl, and phenanthryl, preferably an aryl group having 6 to 10 carbon atoms such as phenyl and naphthyl, may be used.

A "substituent" on an "optionally substituted aryl group" described as a "substituent" on an "optionally substituted hydrocarbon group" represented by $R^1$ includes, for example, (i) a halogen atom such as fluorine, chlorine or the like, (ii) an optionally halogenated $C_{1-4}$ alkyl group such as methyl, ethyl, trifluoromethyl or the like, or (iii) a $C_{1-4}$ alkoxy group such as methoxy, ethoxy or the like.

An "acyl group" represented by $R^1$ includes, for example, -(C=O)-$R^4$,-(C=S)-$R^4$,-$SO_2$-$R^4$,-SO-$R^4$, and -(P=O)($OR^{4a}$) ($OR^{4b}$) (wherein $R^4$ represents a hydrogen atom, an optionally substituted hydrocarbon group, an optionally substituted amino group or an optionally substituted hydroxy group, and $R^{4a}$ and $R^{4b}$ may be the same or different and represent a hydrogen atom or an optionally substituted hydrocarbon group).

An "optionally substituted hydrocarbon group" represented by $R^4$, $R^{4a}$ and $R^{4b}$ includes, for example, one exemplified above as an "optionally substituted hydrocarbon group" represented by $R^1$.

An "optionally substituted amino group" represented by $R^4$ includes, for example, a group represented by -$NR^{4a}R^{4b}$ (wherein $R^{4a}$ and $R^{4b}$ have the same meaning as defined above) . Herein, $R^{4a}$ and $R^{4b}$ may be taken together to form a cyclic amino group (for example, a 5- to 9-membered cyclic amino group which may contain 1 to 3 hetero atoms such as an oxygen and a sulfur atom, in addition to nitrogen atoms (e.g., pyrrolidino, piperidino, morpholino, etc.), etc.).

An "optionally substituted hydroxy group" represented by $R^4$ includes, for example, a group represented by -$OR^{4a}$ (wherein $R^{4a}$ has the same meaning as defined above).

An "acyl group" represented by $R^1$ is preferably -(C=O) -$R^4$ or - (C=S) -$R^4$, wherein the symbols have the same meaning as defined above.

A "heterocyclic group" in an "optionally substituted heterocyclic group" represented by $R^1$ includes, for example, an aromatic heterocyclic group, a saturated or unsaturated non-aromatic heterocyclic group or the like containing, in addition to carbon atoms, at least one (preferably 1 to 4, more preferably 1 to 2) atom of hetero atoms consisting of 1 to 3 (preferably 1 to 2) kinds of hetero atoms selected from an oxygen atom, a sulfur atom and a nitrogen atom, as atoms (ring atoms) constituting a ring system.

The "aromatic heterocyclic group" includes a 5- or 6-membered aromatic monocyclic heterocyclic group such as an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl, etc.), and a 8- to 12-membered aromatic condensed heterocyclic group such as an aromatic condensed heterocyclic group (e.g., benzofuranyl, isobenzofuranyl, benzothienyl, indolyl, isoindolyl, 1H-indazolyl, benzindazolyl, benzoxazolyl, 1,2-benzoisoxazolyl, benzothiazolyl, benzopyranyl, 1,2-benzoisothiazolyl, 1H-benzotriazolyl, quinolyl, isoquinolyl, cinnolinyl, quinazolyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolydinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl, etc.) (preferably a heterocyclic ring formed by condensing the above-mentioned 5- or 6-membered aromatic monocyclic heterocyclic group with a benzene ring, or a heterocyclic ring formed by condensing same or different 2 heterocyclic rings of the above-mentioned 5- or 6-membered aromatic monocyclic heterocyclic groups) .

The "non-aromatic heterocyclic group" includes, for example, a 3- to 8-membered (preferably 5- to 6-membered) saturated or unsaturated (preferably saturated) non-aromatic heterocyclic group such as oxiranyl, azetidinyl, oxetanyl, thietanyl, pyrrolidinyl, tetrahydrofuryl, tetrahydrothienyl, piperidyl, tetrahydropyranyl, morpholinyl, thiomorpholinyl and piperazinyl.

A "substituent" on an "optionally substituted heterocyclic group" represented by $R^1$ includes, for example, one exemplified as a substituent which may be contained in an "optionally substituted aromatic heterocyclic ring" represented by ring A, as well as a group represented by Y (wherein Y has the same meaning as defined above) in the formula [I] shown above, or a group represented by the following formula:

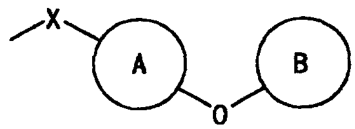

wherein each symbol has the same meaning as defined above.

A "substituent" on an "optionally substituted heterocyclic group" represented by $R^1$ may be on any position capable of being substituted on a ring, and when two or more substituents are present, they may be the same or different. The number of substituents may be about 1 to 3, preferably about 1 to 2.

An "optionally substituted hydrocarbon group" represented by $R^2$ in "$SR^2$" (wherein $R^2$ represents a hydrogen atom, an optionally substituted hydrocarbon group, an acyl group or an optionally substituted heterocyclic group) represented by $R^1$ includes, for example, one exemplified as an "optionally substituted hydrocarbon group" represented by $R^1$ described above.

An "acyl group" represented by $R^2$ in "$SR^2$" (wherein $R^2$ represents a hydrogen atom, an optionally substituted hydrocarbon group, an acyl group or an optionally substituted heterocyclic group) represented by $R^1$ includes, for example, one exemplified as an "acyl group" represented by $R^1$.

An "optionally substituted heterocyclic group" represented by $R^2$ in "$SR^2$" (wherein $R^2$ represents a hydrogen atom, an optionally substituted hydrocarbon group, an acyl group or an optionally substituted heterocyclic group) represented by $R^1$ includes, for example, one exemplified as an "optionally substituted heterocyclic group" represented by $R^1$.

As for $R^1$, for example, a hydrogen atom, an optionally substituted lower alkyl group, $-(C=O)-R^4$, $-(C=S)-R^4$ (wherein the symbol has the same meaning as defined above) or $SR^2$ (wherein $R^2$ has the same meaning as defined above) may be preferably used.

An "optionally substituted lower alkyl group" as a preferred example of $R^1$ includes:

(a) in addition to a straight or branched alkyl group having 1 to 6 carbon atoms such as methyl, ethyl, propyl, isopropyl, butyl, isobutyl, sec-butyl, and tert-butyl;
(b) a halogenated $C_{1-6}$ alkyl group (e.g., chloromethyl, difluoromethyl, trichloromethyl, trifluoromethyl, 2-bromoethyl, 2,2,2-trifluoroethyl, perfluoroethyl, 3,3,3-trifluoropropyl, perfluorobutyl, etc.);
(c) an amino group-substituted $C_{1-6}$ alkyl group (e.g., aminomethyl, 2-aminoethyl, etc.);
(d) a mono- or di-$C_{1-6}$ alkylamino group- substituted $C_{1-6}$ alkyl group (e.g., methylaminomethyl, dimethylaminomethyl, 2-methylaminoethyl, 2-dimethylaminoethyl, etc.);
(e) a carboxy group-substituted $C_{1-6}$ alkyl group (e.g., carboxymethyl, carboxyethyl, etc.);
(f) a $C_{1-6}$ alkoxycarbonyl group-substituted $C_{1-6}$ alkyl group (e.g., methoxycarbonylethyl, ethoxycarbonylethyl, tert-butoxycarbonylmethyl, etc.);
(g) a hydroxy group-substituted $C_{1-6}$ alkyl group (e.g., hydroxymethyl, hydroxyethyl, etc.);
(h) a $C_{6-14}$ aryl group-substituted $C_{1-6}$ alkyl group (e.g. , benzyl, etc.);
(i) a $C_{1-6}$ alkoxy group-substituted $C_{1-6}$ alkyl group (e.g., methoxymethyl, methoxyethyl, etc.);
(j) a $C_{7-15}$ aralkyloxy group-substituted $C_{1-6}$ alkyl group (e.g., benzyloxymethyl, etc.);
(k) a $C_{1-6}$ alkylcarbonylamino group-substituted $C_{1-6}$ alkyl group (e.g., acetylaminomethyl, acetylaminoethyl, etc.);
(l) a $C_{1-6}$ alkoxycarbonylamino group-substituted $C_{1-6}$ alkyl group (e.g., ethoxycarbonylaminoethyl, tert-butoxycarbonylaminoethyl, etc.);
(m) a $C_{1-6}$ alkylcarbonyloxy group-substituted $C_{1-6}$ alkyl group (e.g., acetyloxymethyl, etc.); or
(n) a heterocyclic group (e.g., a 5- or 6-membered aromatic heterocyclic group containing 1 or 2 hetero atoms selected from an oxygen atom, a sulfur atom and a nitrogen atom, such as furyl, thienyl, pyrrolyl, etc.).

More preferably, $R^1$ is (1) a hydrogen atom, (2) a $C_{1-6}$ alkyl group optionally substituted by 1 to 3 (preferably 1 or 2) substituents selected from (i) a $C_{1-6}$ alkoxycarbonyl group (especially, ethoxycarbonyl, tert-butoxycarbonyl, etc.), (ii) a $C_{6-14}$ aryl group (especially, phenyl, etc.), (iii) a carboxy group, (iv) an amino group, (v) a mono- or di-$C_{1-6}$

alkylamino group, (vi) a $C_{1-6}$ alkylcarbonylamino group, (vii) a $C_{1-6}$ alkoxycarbonylamino group, (viii) a $C_{1-6}$ alkylcarbonyloxy group, and (ix) a heterocyclic group (e.g., a 5- or 6-membered aromatic heterocyclic group containing 1 or 2 hetero atoms selected from an oxygen atom, a sulfur atom and a nitrogen atom, such as furyl, thienyl, pyrrolyl, etc.), (3) - (C=O) -$R^{6a}$ or - (C=O) -$R^{6a}$ (wherein $R^{6a}$ represents a $C_{1-6}$ alkyl group, $C_{2-6}$ alkenyl group (especially allyl) or a $C_{6-14}$ aryl group (especially phenyl, etc.) each optionally substituted by 1 to 3 (preferably 1 or 2) substituents selected from (i) a carboxy group, (ii) a $C_{1-6}$ alkylcarbonylamino group, (iii) a $C_{1-6}$ alkylcarbonyloxy group and (iv) a $C_{1-6}$ alkoxycarbonyl group), (4) -(C=O)-$NR^{6a}R^{27a}$ or - (C=S) -$NR^{6a}R^{27a}$ (wherein $R^{6a}$ has the same meaning as defined above and $R^{27a}$ represents a hydrogen atom or a $C_{1-6}$ alkyl group (especially methyl, ethyl, etc.)), or (5) $SR^{2a}$ (wherein $R^{2a}$ represents (i) a $C_{1-6}$ alkyl group or a $C_{6-14}$ aryl group each optionally substituted by 1 to 3 (preferably 1 or 2) substituents selected from (a) a carboxy group, (b) an amino group and (c) a $C_{1-6}$ alkylcarbonylamino group, or (ii) a $C_{1-6}$ alkoxycarbonyl group), and in particular, -(C=O) -$NR^{6a}R^{27a}$ or -(C=S)-$NR^{6a}R^{27a}$ (wherein $R^{6a}$ and $R^{27a}$ have the same meaning as defined above) is preferably used.

Furthermore, $R^1$ preferably includes a group represented by the following formula:

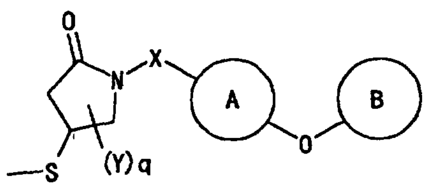

wherein each symbol has the same meaning as defined above,

or a group by the following formula:

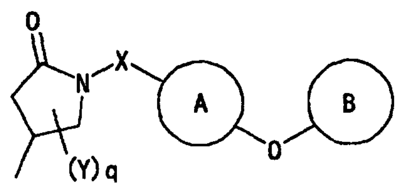

wherein each symbol has the same meaning as defined above.

The configuration for $R^1S$- is preferably an absolute configuration represented by the following structure in the formula [I]:

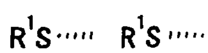

(4) Group "X"

X represents an optionally substituted divalent $C_{1-3}$ aliphatic hydrocarbon group.

A "divalent $C_{1-3}$ aliphatic hydrocarbon group" in an "optionally substituted divalent $C_{1-3}$ aliphatic hydrocarbon group" represented by X, represents a group obtained by removing (2 in total of) hydrogen atoms each one of which is binding to the same or different carbon atoms in a saturated or unsaturated $C_{1-3}$ aliphatic hydrocarbon.

Specific examples thereof include the following:

(i) a $C_{1-3}$ alkylene group (e.g., $-CH_2-$, $-CH_2CH_2-$, $-CH_2CH_2CH_2-$, $-CH(CH_3)-CH_2-$, etc.);
(ii) a $C_{2-3}$ alkenylene group (e.g., $-CH=CH-$, $-CH=CH-CH_2-$, etc.);
(iii) a $C_{2-3}$ alkynylene group (e.g., $-C\equiv C-$, $-C\equiv C-CH_2-$, etc.).

Among these, a $C_{1-3}$ alkylene group (e.g., $-CH_2-$, $-CH_2CH_2-$, $-CH_2CH_2CH_2-$ or $-CH(CH_3)-CH_2-$, etc.) is preferred and $-CH_2-$ is particularly preferred.

A "substituent" on an "optionally substituted divalent $C_{1-3}$ aliphatic hydrocarbon group" represented by X includes, for example, in addition to one exemplified as a "substituent" on an "optionally substituted hydrocarbon group" represented by $R^1$ described above, as well as an oxo group and a thioxo group.

As for X, an optionally substituted methylene group is preferably used.
The "substituent" on an "optionally substituted methylene group" includes, for example, one exemplified as a "substituent" on an "optionally substituted divalent $C_{1-3}$ aliphatic hydrocarbon group" represented by X.
As for X, preferred is a methylene group optionally substituted by 1 or 2 substituents selected from (1) a halogen atom, (2) a $C_{1-4}$ alkyl group or a $C_{1-4}$ alkoxy group each optionally substituted by 1 or 2 substituents selected from (i) a halogen atom, (ii) a $C_{1-4}$ alkoxy group, (iii) a nitro group, (iv) a cyano group, (v) a hydroxy group, (vi) an amino group, (vii) a mono- or di-$C_{1-4}$ alkylamino group, (viii) a carboxy group, (ix) a $C_{1-4}$ alkoxycarbonyl group, and (x) a $C_{1-4}$ alkylcarbonyl group, (3) a nitro group, (4) a cyano group, (5) a hydroxy group, (6) an amino group, (7) a mono- or di-$C_{1-4}$ alkylamino group, (8) a carboxy group, (9) a $C_{1-4}$ alkoxycarbonyl group, and (10) a $C_{1-4}$ alkylcarbonyl group, an oxo group or a thioxo group, and an unsubstituted methylene group is particularly preferred.

(5) Group "Y"

Each Y may be the same or different and represents an optionally substituted hydrocarbon group, a halogen atom, a carboxy group, an acyl group, an optionally substituted hydroxy group, an optionally substituted amino group, $SR^3$ (wherein $R^3$ represents a hydrogen atom, an optionally substituted hydrocarbon group, an acyl group or an optionally substituted heterocyclic group), an oxo group, a thioxo group, an optionally substituted imino group, a nitro group or a cyano group.
An "optionally substituted hydrocarbon group" represented by Y includes, for example, one exemplified as an "optionally substituted hydrocarbon group" represented by $R^1$ described above.
A "halogen atom" represented by Y includes, for example, fluorine, chlorine, bromine and iodine.
An "acyl group" represented by Y includes, for example, one exemplified as an "acyl group" represented by $R^1$ described above.
An "optionally substituted hydroxy group" represented by Y includes, for example, one exemplified as an "optionally substituted hydroxy group" as a "substituent" on an "optionally substituted amino group" represented $R^4$ described above.
An "optionally substituted amino group" represented by Y includes, for example, one exemplified as an "optionally substituted amino group" represented by $R^4$ described above.
An "optionally substituted hydrocarbon group" as "$R^3$" in "$SR^3$" represented by Y includes, for example, one exemplified as an "optionally substituted hydrocarbon group" represented by $R^1$ described above.
An "acyl group" as "$R^3$" in "$SR^3$" represented by Y includes, for example, one exemplified as an "acyl group" represented by $R^1$ described above.
An "optionally substituted heterocyclic group" as "$R^3$" in "$SR^3$" represented by Y includes, for example, one exemplified as an "optionally substituted heterocyclic group" represented by $R^1$ described above (provided that, among the examples of as a "substituent" on the "optionally substituted heterocyclic group" represented by $R^1$ described above, a group represented by Y (wherein Y has the same meaning as defined above) and a group represented by the following formula:

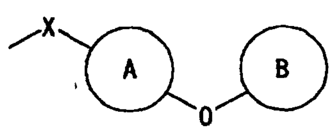

wherein each symbol has the same meaning as defined above, are excluded from the substituents on the "optionally substituted heterocyclic group" represented by $R^3$).
A substituent on an "optionally substituted imino group" represented by Y includes, for example, one exemplified as an "optionally substituted hydrocarbon group" and an "acyl group" represented by $R^1$ described above.
Preferred as Y is an optionally substituted lower alkyl group, an optionally substituted hydroxy group, an optionally substituted amino group, $SR^{12}$ (wherein $R^{12}$ represents a hydrogen atom, an optionally substituted hydrocarbon group, an acyl group or an optionally substituted heterocyclic group) or an oxo group.
The "optionally substituted lower alkyl group" described above as a preferred example of Y includes, for example:

(a) in addition to a straight or branched alkyl group having 1 to 6 carbon atoms such as methyl, ethyl, propyl, isopropyl, butyl, isobutyl, sec-butyl, and tert-butyl;
(b) a halogenated $C_{1-6}$ alkyl group (e.g., chloromethyl, difluoromethyl, trichloromethyl, trifluoromethyl, 2-bromoethyl, 2,2,2-trifluoroethyl, perfluoroethyl, 3,3,3-trifluoropropyl, perfluorobutyl, etc.);

(c) an amino group-substituted $C_{1-6}$ alkyl group (e.g., aminomethyl, 2-aminoethyl, etc.);
(d) a mono- or di-$C_{1-6}$ alkylamino group-substituted $C_{1-6}$ alkyl group (e.g., methylaminomethyl, dimethylaminomethyl, 2-methylaminoethyl, 2-dimethylaminoethyl, etc.);
(e) a carboxy group-substituted $C_{1-6}$ alkyl group (e.g., carboxymethyl, carboxyethyl, etc.);
(f) a $C_{1-6}$ alkoxycarbonyl group-substituted $C_{1-6}$ alkyl group (e.g., methoxycarbonylethyl, ethoxycarbonylethyl, tert-butoxycarbonylmethyl, etc.);
(g) a hydroxy group-substituted $C_{1-6}$ alkyl group (e.g., hydroxymethyl, hydroxyethyl, etc.);
(h) a $C_{6-14}$ aryl group-substituted $C_{1-6}$ alkyl group (e.g. , benzyl, etc.);
(i) a $C_{1-6}$ alkoxy group-substituted $C_{1-6}$ alkyl group (e.g., methoxymethyl, methoxyethyl, etc.); or
(j) a $C_{7-15}$ aralkyloxy group-substituted $C_{1-6}$ alkyl group (e.g., benzyloxymethyl, etc.).

The "optionally substituted hydroxy group" described above as a preferred example of Y includes, for example, one exemplified as an "optionally substituted hydroxy group" as a "substituent" on an "optionally substituted amino group" represented by $R^4$ described above.
The "optionally substituted amino group" described above as a preferred example of Y includes, for example, one exemplified as an "optionally substituted amino group" represented by $R^4$ described above.
An "optionally substituted hydrocarbon group" as "$R^{12}$" in "$SR^{12}$" as a preferred example of Y includes, for example, one exemplified as an "optionally substituted hydrocarbon group" represented by $R^1$ described above (provided that a group represented by the following formula:

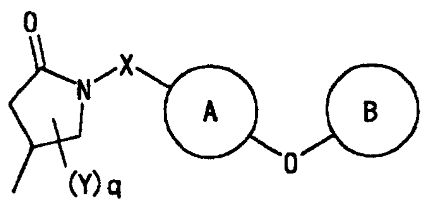

wherein each symbol has the same meaning as defined above, as a substituent on an "optionally substituted hydrocarbon group" represented by $R^1$, is excluded from the substituents on an "optionally substituted hydrocarbon").
An "acyl group" as "$R^{12}$" in "$SR^{12}$" represented by Y may be, for example, one exemplified above as an "acyl group" represented by $R^1$ described above.
An "optionally substituted heterocyclic group" as "$R^{12}$" in "$SR^{12}$" represented by Y may be, for example, one exemplified as an "optionally substituted heterocyclic group" represented by $R^1$ described above (provided that among the examples of a "substituent" on an "optionally substituted heterocyclic group" represented by $R^1$ described above, a group represented by Y (wherein Y has the same meaning as defined above) and a group represented by the following formula:

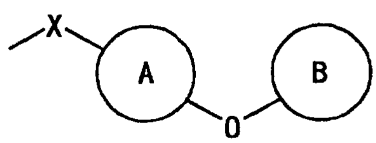

wherein each symbol has the same meaning as defined above,
are excluded from the substituents on an "optionally substituted heterocyclic group" represented by $R^{12}$) .
More preferred examples of Y include, for example, an unsubstituted $C_{1-6}$ alkyl group (e.g., methyl, ethyl, propyl, isopropyl, butyl, isobutyl, sec-butyl, tert-butyl, etc.), a hydroxy group-substituted $C_{1-6}$ alkyl group (e.g., hydroxymethyl, hydroxyethyl, etc.), a $C_{1-6}$ alkoxy group-substituted $C_{1-6}$ alkyl group (e.g., benzyloxymethyl, methoxymethyl, etc.) or an oxo group.

(6) Number "q"

q represents an integer of 0 to 5.
q is preferably 0 or 1 and more preferably 0.

(7) Partial structure of compound represented by the formula [I]

A preferred group represented by the following formula:

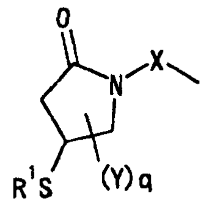

which is a partial structure of the formula [I] may be, for example, a group represented by the following formula:

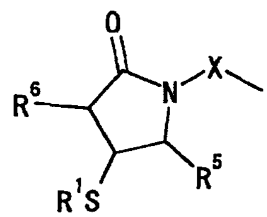

wherein each of $R^5$ and $R^6$ may be the same or different and represents a hydrogen atom, an optionally substituted lower alkyl group, an optionally substituted hydroxy group, an optionally substituted amino group or $SR^7$ (wherein $R^7$ represents a hydrogen atom, an optionally substituted hydrocarbon group, an acyl group or an optionally substituted heterocyclic group) and each of other symbols has the same meaning as defined above.

An "optionally substituted lower alkyl group" represented by $R^5$ and $R^6$ includes, for example:

(a) in addition to a straight or branched alkyl group having 1 to 6 carbon atoms such as methyl, ethyl, propyl, isopropyl, butyl, isobutyl, sec-butyl, and tert-butyl;
(b) a halogenated $C_{1-6}$ alkyl group (e.g., chloromethyl, difluoromethyl, trichloromethyl, trifluoromethyl, 2-bromoethyl, 2,2,2-trifluoroethyl, perfluoroethyl, 3,3,3-trifluoropropyl, perfluorobutyl, etc.);
(c) an amino group-substituted $C_{1-6}$ alkyl group (e.g., aminomethyl, 2-aminoethyl, etc.);
(d) a mono- or di-$C_{1-6}$ alkylamino group-substituted $C_{1-6}$ alkyl group (e.g., methylaminomethyl, dimethylaminomethyl, 2-methylaminoethyl, 2-dimethylaminoethyl, etc.);
(e) a carboxy group-substituted $C_{1-6}$ alkyl group (e.g., carboxymethyl, carboxyethyl, etc.);
(f) a $C_{1-6}$ alkoxycarbonyl group-substituted $C_{1-6}$ alkyl group (e.g., methoxycarbonylethyl, ethoxycarbonylethyl, tert-butoxycarbonylmethyl, etc.);
(g) a hydroxy group-substituted $C_{1-6}$ alkyl group (e.g., hydroxymethyl, hydroxyethyl, etc.);
(h) a $C_{6-14}$ aryl group-substituted $C_{1-6}$ alkyl group (e.g., benzyl, etc.);
(i) a $C_{1-6}$ alkoxy group-substituted $C_{1-6}$ alkyl group (e.g., methoxymethyl, methoxyethyl, etc.); or
(j) a $C_{7-15}$ aralkyloxy group-substituted $C_{1-6}$ alkyl group (e.g., benzyloxymethyl, etc.).

An "optionally substituted hydroxy group" represented by $R^5$ and $R^6$ may be, for example, one exemplified as an "optionally substituted hydroxy group" as a "substituent" on an "optionally substituted amino group" represented by $R^4$ described above.

An "optionally substituted amino group" represented by $R^5$ and $R^6$ may be, for example, one exemplified as an "optionally substituted amino group" represented by $R^4$ described above.

More preferred examples of $R^5$ and $R^6$ include, for example, a hydrogen atom, an unsubstituted $C_{1-6}$ alkyl group (e.g., methyl, ethyl, propyl, isopropyl, butyl, isobutyl, sec-butyl, tert-butyl, etc.), a hydroxyl group-substituted $C_{1-6}$ alkyl group (e.g., hydroxymethyl, hydroxyethyl, etc.)., an alkoxy group-substituted $C_{1-6}$ alkyl group (e.g., benzyloxymethyl, methoxymethyl, etc.) or an oxo group. In particular, $R^5$ is preferably a $C_{1-6}$ alkyl group optionally substituted by a hydroxy group or a $C_{1-6}$ alkoxy group, or an oxo group, and $R^6$ is preferably a hydrogen atom.

Among compounds represented by the formula [I], particularly preferred is a compound in which ring A is a pyridine ring, ring B is a benzene ring, $R^1$ is an acyl group, and X is a methylene group.

More specifically, among the compounds represented by the formula [I], particularly preferred is a compound in which:

X is a methylene group optionally substituted by 1 or 2 substituents selected from (1) a halogen atom, (2) a $C_{1-4}$ alkyl group or a $C_{1-4}$ alkoxy group each optionally substituted by 1 or 2 substituents selected from (i) a halogen atom, (ii) a $C_{1-4}$ alkoxy group, (iii) a nitro group, (iv) a cyano group, (v) a hydroxy group, (vi) an amino group, (vii) a mono- or di-$C_{1-4}$ alkylamino group, (viii) a carboxy group, (ix) a $C_{1-4}$ alkoxycarbonyl group and (x) a $C_{1-4}$ alkylcarbonyl group, (3) a nitro group, (4) a cyano group, (5) a hydroxy group, (6) an amino group, (7) a mono- or di-$C_{1-4}$ alkylamino group, (8) a carboxy group, (9) a $C_{1-4}$ alkoxycarbonyl group, and (10) a $C_{1-4}$ alkylcarbonyl group, an oxo group or thioxo group;

$R^1$ is (1) a hydrogen atom, (2) a $C_{1-6}$ alkyl group optionally substituted by 1 to 3 substituents selected from (i) a $C_{1-6}$ alkoxycarbonyl group, (ii) a $C_{6-14}$ aryl group, (iii) a carboxy group, (iv) an amino group, (v) a mono- or di-$C_{1-6}$ alkylamino group, (vi) a $C_{1-6}$ alkylcarbonylamino group, (vii) a $C_{1-6}$ alkoxycarbonylamino group, (viii) a $C_{1-6}$ alkylcarbonyloxy group and (ix) a heterocyclic group (e.g., a 5- or 6-membered aromatic heterocyclic group containing 1 or 2 hetero atoms selected from an oxygen atom, a sulfur atom and a nitrogen atom, such as furyl, thienyl, and pyrrolyl), (3) -(C=O)-$R^{6a}$ or -(C=S)-$R^{6a}$ (wherein $R^{6a}$ has the same meaning as defined above), (4) - (C=O) -$NR^{6a}R^{27a}$ or -(C=S)-$NR^{6a}R^{27a}$ (wherein $R^{6a}$ and $R^{27a}$ have the same meaning as defined above), or (5) $SR^{2a}$ (wherein $R^{2a}$ has the same meaning as defined above);

ring A is a pyridine ring optionally substituted by 1 to 3 substituents selected from (i) a halogen atom, (ii) an optionally halogenated $C_{1-4}$ alkyl group and (iii) an optionally halogenated $C_{1-4}$ alkoxy group;

ring B is a benzene ring optionally substituted by 1 to 3 substituents selected from (i) a halogen atom, (ii) a $C_{1-6}$ alkyl group and (iii) a $C_{1-6}$ alkylsulfonylamino group;

Y is (1) a $C_{1-6}$ alkyl group optionally substituted by (i) hydroxy or (ii) $C_{1-6}$ alkoxy, or (2) an oxo group; and

q is 0 or 1.

(8) Optically active substances of a compound represented by the formula [I]

While a compound represented by the formula [I] or a salt thereof can exist as an optically active substance when it contains an asymmetric carbon in its structure, a preferred optically active substance may be, for example, a compound represented by the following formula:

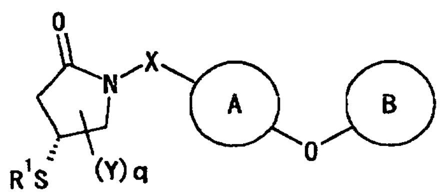

wherein each symbol has the same meaning as defined above, or a salt thereof.

The present invention further provides a method for producing a compound represented by the formula [I] or a salt thereof.

A compound represented by the formula [I] (hereinafter, referred to as "compound [I]") or a salt thereof and starting compounds thereof can be produced by the methods described below.

(Method A) A compound represented by the formula [I] of the invention or a salt thereof can be produced, for example, by reacting a compound represented by the following formula [II] :

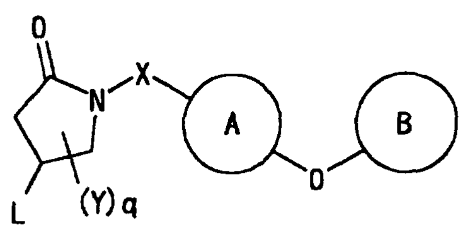

[II]

wherein L represents a leaving group, and each of other symbols has the same meaning as defined above, or a salt thereof with a compound of the following formula [III] :

$R^1SH$ (III)

wherein $R^1$ has the same meaning as defined above,
or a salt thereof in the presence of a base.

Herein, a leaving group represented by L in a compound of the formula [II] includes, for example, a hydroxyl group, a halogen atom (e.g., chlorine, bromine, iodine, etc.), a substituted sulfonyloxy group (e.g., methanesulfonyloxy, p-toluenesulfonyloxy, etc.), an acyloxy group (e.g., acetoxy, benzoyloxy, etc.), and an oxy group substituted by a heterocyclic ring or an aryl group (such as succinimide, benzotriazole, quinoline and 4-nitrophenyl, etc.).

In this reaction, 1 to 10 moles, preferably 1 to 5 moles of a compound of the formula [III] or a salt thereof is used with respect to 1 mole of a compound of the formula [II] or a salt thereof.

As for $R^1SH$, for example, an inorganic sulfide such as hydrogen sulfide, sodium hydrogen sulfide, sodium sulfide or the like, and their salts, an organic sulfur-containing acid such as thioacetic acid, thiobenzoic acid or the like, and their salts, an aliphatic mercaptan such as methylmercaptan, benzylmercaptan, triphenylmethylmercaptan, 3-mercaptopropionic acid derivatives or the like, and an aromatic mercaptan such as thiophenol, or a thiourea may be used.

As for the reaction solvent, for example, an alcohol such as methanol, ethanol or the like, an ether such as dioxane, tetrahydrofuran or the like, an aromatic hydrocarbon such as benzene, toluene, xylene or the like, an ester such as ethyl acetate or the like, a halogenated hydrocarbon such as chloroform, dichloromethane or the like, a nitrile such as acetonitrile or the like, an amide such as N,N-dimethylformamide, N,N-dimethylacetamide or the like, a sulfoxide such as dimethylsulfoxide or the like, or a mixture thereof may be used.

Furthermore, the addition of a base in this reaction allows the reaction to proceed advantageously. As for the base, for example, an inorganic base (e.g., an alkali metal hydroxide such as sodium hydroxide, potassium hydroxide, etc.; an alkali metal hydrogen carbonate such as sodium hydrogen carbonate, potassium hydrogen carbonate, etc.; an alkali metal carbonate such as sodium carbonate, potassium carbonate, cesium carbonate, etc.; an alkali metal hydride such as sodium hydride, potassium hydride, etc.; an alkali metal amide such as sodium amide, etc.; an alkoxide such as sodium methoxide, sodium ethoxide, etc.), an organic base (e.g., an aliphatic amine such as trimethylamine, triethylamine, diisopropylethylamine, etc.; an aromatic base such as pyridine, etc.) may be used.

Instead of using a base in this reaction, a compound represented by the formula [III] or a salt thereof may be converted for example into an alkaline metal salt or an alkaline earth metal salt, which may then be reacted with a compound represented by the formula [II] or a salt thereof.

While the amount of the base used may vary depending on the types of the compound and the solvent used as well as other reaction conditions, it is usually 0.1 to 10 moles, preferably 0,2 to 5 moles per 1 mole of a compound represented by $R^1SH$ (wherein $R^1$ has the same meaning as defined above) or a salt thereof. The reaction temperature is usually in the range of about -50 to 200°C, preferably -20 to 100°C. The reaction time may vary depending on the type of the compound and the reaction temperature, and is usually 1 to 96 hours, preferably 1 to 48 hours.

A compound represented by the formula [II] or a salt thereof may be produced from, for example, a compound represented by the following formula [IV]:

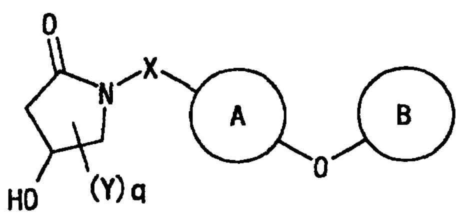

[IV]

wherein each symbol has the same meaning as defined above, or a salt thereof.

Thus, a compound represented by the formula [II] can be produced by converting a hydroxy group of a compound represented by the formula [IV] into a variety of leaving groups.

Conversion of the hydroxy group to a halogen atom can be carried out using, for example, an inorganic acid halide such as thionyl chloride, thionyl bromide, phosphorus trichloride, phosphorus pentachloride, phosphorus oxychloride or the like, or a halide acid such as hydrochloric acid, hydrobromic acid or the like. Other leaving groups may be obtained from a corresponding hydroxy product by converting into an appropriate leaving group according to a method known in a literature such as an acylation or alkylation described for example in Organic Functional Group Preparations (Academic Press, Inc.), or any equivalent method.

For example, a reaction using an acylation employs 1 to 5 moles, preferably 1 to 2 moles of a corresponding acylating agent and 1 to 5 moles, preferably 1 to 3 moles of a base per 1 mole of a hydroxy product or a salt thereof. For example, depending on the type of a base, some base, such as pyridine, can serve also as a solvent.

The reaction solvent may be, for example, an ether such as dioxane, tetrahydrofuran or the like, an aromatic hydrocarbon such as benzene, toluene, xylene or the like, an ester such as ethyl acetate, a halogenated hydrocarbon such as chloroform, dichloromethane or the like, a nitrile such as acetonitrile or the like, an amide such as N,N-dimethylformamide, N,N-dimethylacetamide or the like, a sulfoxide such as dimethylsulfoxide or the like, tert-butanol, water, or a mixture thereof.

For the base, an alkali metal hydroxide such as sodium hydroxide, potassium hydroxide or the like, an alkali metal hydrogen carbonate such as sodium hydrogen carbonate, potassium hydrogen carbonate or the like, an alkali metal carbonate such as sodium carbonate, potassium carbonate, cesium carbonate or the like, an alkali metal hydride such as sodium hydride, potassium hydride or the like, an alkali metal amide such as sodium amide or the like, an alkoxide such as sodium methoxide, sodium ethoxide or the like, an aliphatic amine such as trimethylamine, triethylamine, diisopropylethylamine or the like, or an aromatic base such as pyridine or the like, may be used.

While the reaction temperature may vary depending on the substrate employed as well as other conditions, it is -20 to 200°C, preferably 0 to 100°C. The reaction time is 5 minutes to 24 hours, preferably 10 minutes to 12 hours.

Thus obtained compound [II] can be subjected to the above-described method to react with any of various sulfur-containing nucleophilic agents to produce a compound [I] wherein $R^1$ is H or a salt thereof, or to produce a compound [I] wherein $R^1$ is not H or a salt thereof and also to be deprotected to produce a compound [I] wherein $R^1$ is H or a salt thereof.

Among compounds [I] , a compound (wherein $R^1$ is H) represented by the following formula [V]:

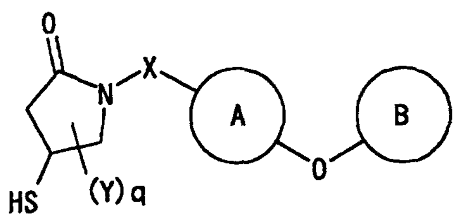

[V]

wherein each symbol has the same meaning as defined above,

or a salt thereof can be produced by a solvolysis of a compound [Ia], which is a compound [I] wherein $R^1$ is an acyl group, by means of, for example, an acid or a base. In this reaction, an acyl group may be, for example, preferably -(C=O)-$R^4$ wherein $R^4$ is a hydrogen atom, an optionally substituted hydrocarbon group, an optionally substituted amino group or an optionally substituted hydroxy group, and among these, particularly preferred is one wherein $R^4$ is an optionally substituted hydrocarbon group, thus acetyl, benzoyl, trifluoroacetyl or the like being particularly preferred.

An acid used in the reaction may be, for example, an inorganic acid such as hydrochloric acid, sulfuric acid, nitric acid or the like, and a base used may be, for example, an alkali metal hydroxide such as sodium hydroxide, potassium hydroxide, etc.; an alkali metal hydrogen carbonate such as sodium hydrogen carbonate, potassium hydrogen carbonate, etc.; an alkaline metal carbonate such as sodium carbonate and potassium carbonate, etc.; an alkali metal amide such as sodium amide, etc.; an alkoxide such as sodium methoxide, sodium ethoxide, etc.; a thioalkoxide such as sodium thiomethoxide, sodium thioethoxide, etc.; ammonia; and an organic amine such as methylamine, ethylamine, etc.

This reaction is carried out in an aqueous solution of the inorganic acid described above (usually at 10 to 30%) in an amount of 5 to 50 volumes per 1 mole of a compound [Ia], or in an aqueous solution containing 3 to 10 moles of the base described above per 1 mole of a compound [Ia]. Further, in view of the solubility of the compound, the reaction may be carried out in an aqueous solution described above added with an organic solvent, or in an organic solvent. An organic solvent that can be used may include, for example, an alcohol such as methanol, ethanol, etc; an organic acid such as acetic acid, trifluoroacetic acid, etc.; an ether such as dioxane, tetrahydrofuran, etc.; a nitrile such as acetonitrile, etc.; an amide such as N,N-dimethylformamide, N,N-dimethylacetamide, etc.; a sulfoxide such as dimethylsulfoxide, etc.; or a mixture thereof.

While the reaction temperature may vary depending on the compound [Ia] employed as well as other conditions, it is 0 to 200°C, preferably 20 to 150°C. The reaction time is 20 minutes to 48 hours, preferably 1 hour to 24 hours.

(Method B) A compound represented by the following formula [Ib]:

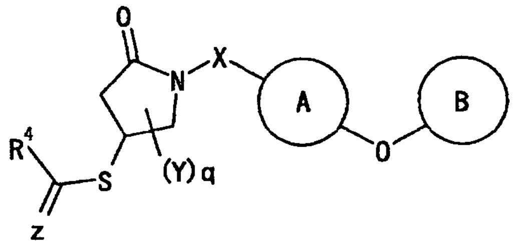

[Ib]

wherein Z represents O or S and each of other symbols has the same meaning as defined above, or a salt thereof, which is encompassed within the compound represented by the formula [I] of the invention or a salt thereof, can be produced by, for example, reacting a compound represented by the formula [V]:

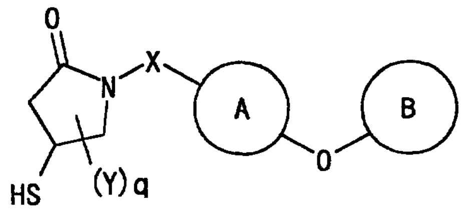

[V]

wherein each symbol has the same meaning as defined above, or a salt thereof with a compound represented by any of the following formulae:

$$R^4\text{-}CO_2H \qquad [VIa],$$

$$R^4\text{-}COSH \qquad [VIb],$$

and

$$R^4\text{-}CSOH \qquad [VIc]$$

wherein each symbol has the same meaning as defined above, or a salt thereof or a reactive derivative thereof.

In this reaction, a compound represented by the formula [VIa], [VIb] or [VIc] or a salt thereof or a reactive derivative thereof is typically used in an amount of 1 to 5 moles, preferably 1 to 3 moles, with respect to 1 mole of a compound of the formula [V] or a salt thereof. A reactive derivative of a compound of the formula [VIa], [VIb] or [VIc] or a salt thereof may include, for example, (thio)carboxylic acid halide, (thio)carboxylic acid anhydride, activated (thio) ester and the like. Further, when $R^4$ is a monosubstituted amino group, use may be made of, for example, an isocyanate compound represented by the following formula:

$$R^{30}\text{-}N{=}C{=}O \qquad [VId]$$

or

$$R^{30}\text{-}N{=}C{=}S \qquad [VIe]$$

wherein $R^{30}$ represents an optionally substituted hydrocarbon group or an acyl group, or an isothiocyanate compound, or a (thio)carbamic acid halide represented by the following formula:

$$R^{30}\text{-NH-(C=O)-L'} \qquad [VIf]$$

or

$$R^{30}\text{-NH- (C=S) -L'} \qquad [VIg]$$

wherein L' represents a halogen atom, and other symbols respectively have the same meaning as defined above.

This reaction is advantageously carried out in a solvent. An aprotic solvent is preferred for the solvent, and for example, an ether such as dioxane, tetrahydrofuran, etc.; an aromatic hydrocarbon such as benzene, toluene, xylene, etc.; an ester such as ethyl acetate, etc.; a halogenated hydrocarbon such as chloroform, dichloromethane, etc.; a nitrile such as acetonitrile, etc.; an amide such as N,N-dimethylformamide, N,N-dimethylacetamide, etc.; a sulfoxide such as dimethylsulfoxide, etc.; pyridine, or a mixture thereof is used.

The reaction may be facilitated favorably by addition of acid and/or base. Such base is preferably an organic base, which may be preferably, for example, an aliphatic amine such as diisopropyl ethylamine, triethylamine, tri-n-octylamine, tetramethyl ethylene diamine, etc.; an aromatic base such as pyridine (usable as a solvent), 2,4,6-trimethylpyridine, picoline, 4-dimethylaminopyridine, 2,6-lutidine, etc.; or 1,8-diazabicyclo[5.4.0]undec-7-ene or the like. An acid may be preferably, for example, an inorganic acid such as hydrochloric acid, sulfuric acid, phosphoric acid, polyphosphoric acid, etc., or an organic acid such as acetic acid, trifluoroacetic acid, methanesulfonic acid, benzenesulfonic acid, toluenesulfonic acid, oxalic acid, fumaric acid, maleic acid or the like.

The reaction can be facilitated favorably by addition of a reducing agent. Such reducing agent may include a phosphine such as trimethylphosphine, tri-n-butylphosphine or triphenylphosphine, zinc, tin, palladium, sodium borohydride, dithioerythritol or the like, and preference is given to tributylphosphine and dithioerythritol. The reducing agent is used in an amount of 0.5 to 10 moles, preferably 0.5 to 3 moles with respect to 1 mole of a compound of the formula [V] or a salt thereof.

An acid and/or a base is used respectively in an amount of 0.01 to 5 equivalents, preferably 0.05 to 3 equivalents with respect to compound [V], and the reaction temperature is typically 0 to 100°C, and preferably 0 to 50°C. The reaction time is typically 1 minute to 15 hours, and preferably 10 minutes to 2 hours.

Compound [V] can be produced by Method A as described above or the like. Compound [VI] or a reactive derivative thereof may be produced by a method known in the art per se, or a commercially available product thereof may be used.

(Method B') A compound represented by the following formula [Ic]:

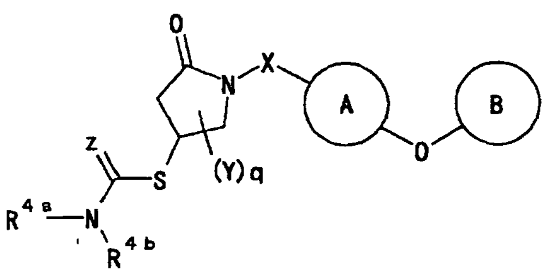

[Ic]

wherein each symbol has the same meaning as defined above,

or a salt thereof, which is included in the compounds of the formula [I] or [Ib] or salts thereof, can be produced, for example, by reacting a compound represented by the formula [V]:

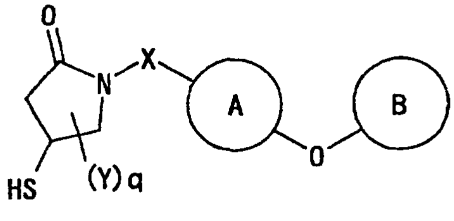

[V]

wherein each symbol has the same meaning as defined above,
or a salt thereof with a compound represented by the following formula [XIII]:

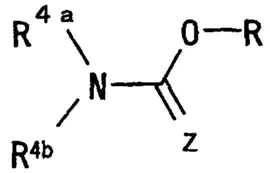

[XIII]

wherein R represents an optionally substituted hydrocarbon group, an acyl group or an optionally substituted heterocyclic group, and other symbols respectively have the same meaning as defined above, or a salt thereof.

An "optionally substituted hydrocarbon group", an "acyl group" or an "optionally substituted heterocyclic group" represented by R herein may be one exemplified above as a group represented by $R^1$. R is preferably an aryl group (a $C_{6-10}$ aryl group such as phenyl, etc.).

In this reaction, a compound represented by the formula [XIII] or a salt thereof is typically used in an amount of 0.1 to 10 moles, preferably 0.5 to 5 moles, with respect to 1 mole of a compound of the formula [V] or a salt thereof.

For the reaction solvent, for example, a halogen-based solvent such as dichloromethane, chloroform, etc.; an ether-based solvent such as tetrahydrofuran, diethyl ether, dimethoxyethane, etc.; an ester-based solvent such as ethyl acetate, isobutyl acetate, etc.; a ketone-based solvent such as acetone, methyl ethyl ketone, etc.; a hydrocarbon-based solvent such as benzene, toluene, n-hexane, etc.; an alcohol-based solvent such as methanol, ethanol, isopropanol, etc.; an aprotic polar solvent such as acetonitrile, N,N-dimethylformamide, dimethylsulfoxide, etc.; water, or a mixture thereof may be used, and preferably acetonitrile or isopropanol is used.

The reaction is facilitated favorably by addition of a reducing agent. Such reducing agent may include a phosphine such as trimethylphosphine, tri-n-butylphosphine or triphenylphosphine, zinc, tin, palladium, sodium borohydride, dithioerythritol or the like, tributylphosphine and dithioerythritol being preferred. The amount of the reducing agent used is 0.5 to 10 moles, preferably 0.5 to 3 moles, with respect to 1 mole of a compound of the formula [V] or a salt thereof.

Further, the reaction is facilitated favorably by addition of a base. Such base may include an amine base such as triethylamine, diisopropylethylamine, etc.; a metal hydroxide such as sodium hydroxide, potassium hydroxide or the like; or a metal carbonate such as sodium carbonate, potassium carbonate, sodium hydrogen carbonate, potassium hydrogen carbonate or the like. Preferred is triethylamine, sodium carbonate or potassium carbonate. The base is used in an amount of 0.1 to 10 moles, preferably 0.5 to 5 moles, with respect to 1 mole of a compound of the formula [V] or a salt thereof.

The reaction temperature is -70 to 200°C, and preferably -20 to 100°C. The reaction time is typically from 30 minutes to 24 hours, and preferably 30 minutes to 12 hours.

(Method C) A compound represented by the following formula [VII]:

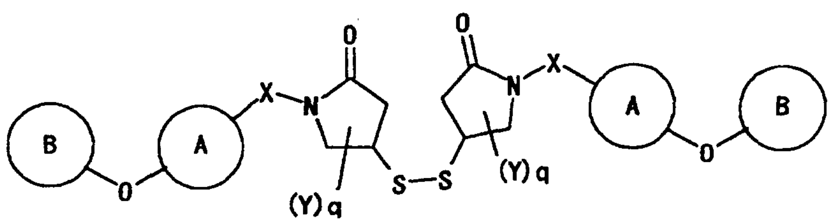

[VII]

wherein each symbol has the same meaning as defined above,
or a salt thereof, which is encompassed within the compound of the formula [I] of the invention, can be produced, for example, by reacting a compound represented by the following formula [V]:

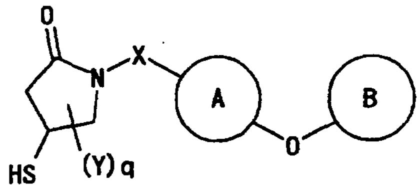

[V]

wherein each symbol has the same meaning as defined above,

or a salt thereof with an oxidizing agent.

An oxidizing agent employed in this reaction may be an ordinary disulfide bond-forming reagent found in "SHINJIKKENKAGAKUKOZA", Vol. 15, Oxidation and Reduction (MARUZEN), for example, a halogen such as chlorine, bromine, iodine, etc., an N-halogen carboxylimide such as N-chlorosuccinimide, etc. or a sulfonamide, a metallic oxidizing agent such as a chromic acid, lead tetraacetate, potassium permanganate, iron chloride, etc., an organic peroxide such as m-chloroperbenzoic acid, peracetic acid, etc. or hydrogen peroxide or an air oxidation, etc.

In the reaction, an oxidizing agent is typically used in an amount of 1 to 5 moles, preferably 1 to 2 moles, with respect to 1 mole of a compound of the formula [V] or a salt thereof.

This reaction is advantageously carried out in a solvent. The reaction solvent may include an ether such as dioxane, tetrahydrofuran, etc.; an aromatic hydrocarbon such as benzene, toluene, xylene, etc.; an ester such as ethyl acetate, etc.; a halogenated hydrocarbon such as chloroform, dichloromethane, etc.; a nitrile such as acetonitrile, etc.; an amide such as N,N-dimethylformamide, N,N-dimethylacetamide, etc.; a sulfoxide such as dimethylsulfoxide, etc.; or a mixture thereof.

Further, the reaction is facilitated favorably by addition of a base. Such base may be an inorganic base including an alkali metal hydroxide such as sodium hydroxide, potassium hydroxide or the like, an alkali metal hydrogen carbonate such as sodium hydrogen carbonate, potassium hydrogen carbonate or the like, an alkali metal carbonate such as sodium carbonate, potassium carbonate or the like, a halide such as sodium iodide, potassium iodide or the like, an alkali metal hydride such as sodium hydride, potassium hydride or the like, an alkali metal amide such as sodium amide or the like, an alkoxide such as sodium methoxide, sodium ethoxide or the like; or an organic base including an aliphatic amine such as trimethylamine, triethylamine, diisopropylethylamine or the like, or an aromatic amine such as pyridine or the like. The amount of the base used may vary depending on the types of compounds and solvent employed and other reaction conditions, but it is typically 0.1 to 20 moles, preferably 1 to 2 moles, with respect to 1 mole of a compound represented by the formula [V] or a salt thereof.

The reaction temperature is -20 to 200°C, and preferably 0 to 100°C. The reaction time is typically from 1 minute to 24 hours, and preferably 1 minute to 5 hours.

(Method D) A compound represented by the formula [IV] or a salt thereof can be produced, for example, from a compound represented by the following formula [VIII]:

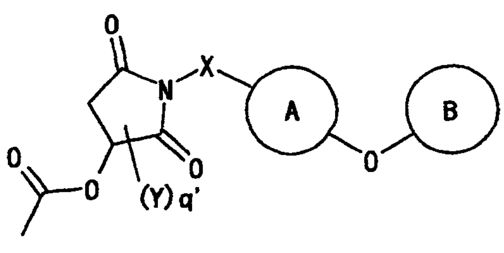

[VIII]

wherein q' represents an integer of 0 to 3, and each symbol has the same meaning as defined above,

or a salt thereof. That is to say, a compound of the formula [IV] or a salt thereof is obtained by reducing compound [VIII] (Reduction Reaction 1) to obtain a compound represented by;the following formula [IX]:

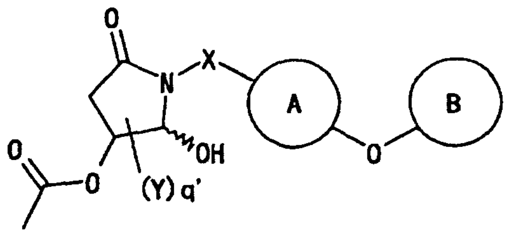

[IX]

wherein each symbol has the same meaning as defined above,
or a salt thereof, subsequently reducing the above-obtained compound [IX] or a salt thereof (Reduction Reaction 2) to obtain a compound represented by the following formula [X]:

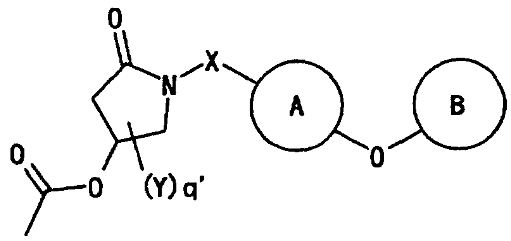

[X]

wherein each symbol has the same meaning as defined above,
or a salt thereof, and then deprotecting the resulting compound [X] or a salt thereof.

A reducing agent employed in Reduction Reaction 1 may be preferably, for example, a hydrogen complex such as sodium borohydride, lithium borohydride, sodium cyanoborohydride, sodium triacetoxyborohydride, aluminum diisobutylhydride, lithium aluminum hydride or the like.

In Reduction Reaction 1, a reducing agent is used in an amount of 0.5 to 10 moles, preferably 0.5 to 3 moles, with respect to 1 mole of a compound of the formula [VIII] or a salt thereof. The reaction solvent may be an alcohol such as methanol, ethanol, etc.; an ether such as dioxane, tetrahydrofuran, etc.; an aromatic hydrocarbon such as benzene, toluene, xylene, etc.; an ester such as ethyl acetate, etc.; a halogenated hydrocarbon such as chloroform, dichloromethane, etc.; a nitrile such as acetonitrile, etc.; an amide such as N,N-dimethylformamide, N,N-dimethylacetamide, etc.; a sulfoxide such as dimethylsulfoxide, etc.; or a mixture thereof.

In Reduction Reaction 1, the reaction temperature is -50 to 50°C, and preferably -30 to 20°C. The reaction time is 30 minutes to 24 hours, and preferably 30 minutes to 12 hours.

A reducing agent employed in Reduction Reaction 2 is preferably, for example, hydrosilane such as triethylsilane, trichlorosilane or the like.

In Reduction Reaction 2, a reducing agent is used in an amount of 0.5 to 10 moles, preferably 0.5 to 3 moles, with respect to 1 mole of a compound of the formula [IX] or a salt thereof. The reaction solvent may include, for example, an alcohol such as methanol, ethanol, etc.; an ether such as dioxane, tetrahydrofuran, etc.; an aromatic hydrocarbon such as benzene, toluene, xylene, etc.; an ester such as ethyl acetate, etc.; a halogenated hydrocarbon such as chloroform, dichloromethane, etc.; a nitrile such as acetonitrile, etc.; an amide such as N,N-dimethylformamide, N,N-dimethylacetamide, etc.; a sulfoxide such as dimethyl sulfoxide, etc.; an organic acid such as trifluoroacetic acid, acetic acid, etc.; or a mixture thereof.

Furthermore, Reduction Reaction 2 is facilitated favorably by addition of an acid. Such acid may be preferably, for example, an inorganic acid such as hydrochloric acid, sulfuric acid, phosphoric acid or the like; an organic acid such as methanesulfonic acid, benzene sulfonic acid, toluene sulfonic acid, oxalic acid, fumaric acid, maleic acid, acetic acid, monofluoroacetic acid, difluoroacetic acid, trifluoroacetic acid, monochloroacetic acid, dichloroacetic acid, trichloroacetic acid or the like; a Lewis acid such as boron trifluoride-ether complex, titanium tetrachloride, aluminum chloride or the like. The amount of the acid used may vary depending on the types of compounds and solvent employed, and other reaction conditions, but it is typically 0.1 to 10 moles, preferably 0.1 to 5 moles, with respect to 1 mole of a compound of the formula [IX] or a salt thereof.

In Reduction Reaction 2, the reaction temperature is -10 to 100°C, and preferably 0 to 50°C. The reaction time is 30 minutes to 24 hours, and preferably 30 minutes to 12 hours.

A compound represented by the formula [IV] or a salt thereof can be produced by deprotecting a compound

represented by the formula [X] or a salt thereof by means of an acid or a base.

An acid employed may be, for example, an inorganic acid such as hydrochloric acid, sulfuric acid, nitric acid, phosphoric acid or the like, and a base may be, for example, an alkali metal hydroxide such as sodium hydroxide, potassium hydroxide, etc.; an alkali metal hydrogen carbonate such as sodium hydrogen carbonate, potassium hydrogen carbonate, etc.; an alkali metal carbonate such as sodium carbonate, potassium carbonate, etc.; an alkali metal amide such as sodium amide, etc.; an alkoxide such as sodium methoxide, sodium ethoxide, etc.; an organic amine such as ammonia, methylamine, ethylamine, etc.

The deprotection reaction is carried out, for example, in an aqueous solution of such inorganic acid in an amount of 5 to 50 volumes (usually at 10 to 30%) with respect to 1 g of a compound of the formula [X] or a salt thereof, or in an aqueous solution containing 3 to 10 moles of such base with respect to 1 mole of the compound or a salt thereof. Since the compound is soluble, the reaction can be carried out in the aqueous solution added with an organic solvent, or in an organic solvent. An organic solvent employed may be an alcohol such as methanol, ethanol, etc.; an organic acid such as acetic acid, etc.; an ether such as dioxane, tetrahydrofuran, etc.; a nitrile such as acetonitrile, etc.; an amide such as N,N-dimethylformamide, N,N-dimethylacetamide, etc.; a sulfoxide such as dimethylsulfoxide, etc.; or the like.

While the reaction temperature may vary depending on compound [X] and other conditions, it is 0 to 200°C, and preferably 20 to 150°C. The reaction time is 30 minutes to 48 hours, and preferably 1 to 24 hours.

(Method E) A compound represented by the following formula [VIII]:

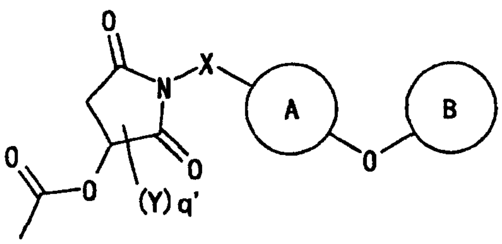

[VIII]

wherein each symbol has the same meaning as defined above,
or a salt thereof can be produced, for example, by reacting a compound represented by the following formula [XI]:

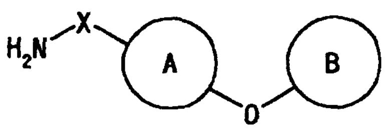

[XI]

wherein each symbol has the same meaning as defined above,
or a salt thereof with a compound represented by the following formula [XII]:

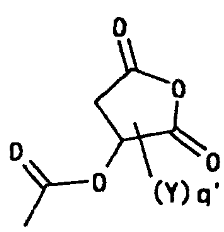

[XII]

wherein each symbol has the same meaning as defined above,
or a salt thereof, or a compound represented by the following formula [XII']:

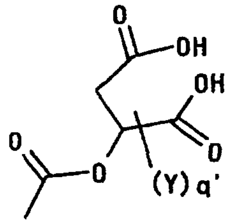

[XII']

wherein each symbol has the same meaning as defined above,
or a salt thereof.

In the reaction, a compound of the formula [XII] or a salt thereof, or a compound of the formula [XII'] or a salt thereof is typically used in an amount of 1 to 5 moles, preferably 1 to 3 moles, with respect to 1 mole of a compound represented by the formula [XI] or a salt thereof.

The reaction solvent may be, for example, an alcohol such as methanol, ethanol, etc.; an ether such as dioxane, tetrahydrofuran, etc.; an aromatic hydrocarbon such as benzene, toluene, xylene, etc.; an ester such as ethyl acetate, etc.; a halogenated hydrocarbon such as chloroform, dichloromethane, etc.; a nitrile such as acetonitrile, etc.; an amide such as N,N-dimethylformamide, N,N-dimethylacetamide, etc.; a sulfoxide such as dimethylsulfoxide, etc.; an organic acid such as acetic acid, etc.; or a mixture thereof.

Further, the reaction is facilitated favorably by addition of an acid. Such acid is preferably an inorganic acid such as hydrochloric acid, sulfuric acid, phosphoric acid or the like, or an organic acid such as methanesulfonic acid, benzene sulfonic acid, toluene sulfonic acid, oxalic acid, fumaric acid, maleic acid or the like. The amount of the acid used may vary depending on the types of compounds or solvent used and other reaction conditions, but is typically 0.01 to 1 mole, preferably 0.05 to 0.1 mole, with respect to 1 mole of the compound.

The reaction temperature is typically 0 to 200°C, and preferably 50 to 150°C.

The reaction time is typically 30 minutes to 48 hours, and preferably 1 to 24 hours.

Compound [XI] can be produced by a method described in WO 97/37990 or a corresponding method, and compound [XII] or [XII'] can be produced by a method known per se or is available commercially.

(Method F) A compound represented by the formula [IV] or a salt thereof can also be produced, for example, by reacting a compound represented by the following formula [XIV] :

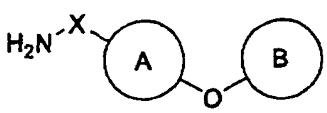

[XIV]

wherein each symbol has the same meaning as defined above,
or a salt thereof with a compound represented by the following formula [XV]:

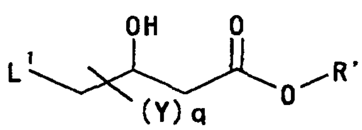

[XV]

wherein R' represents an optionally substituted hydrocarbon group, an acyl group, or an optionally substituted heterocyclic group and $L^1$ represents a leaving group,
or a salt thereof.

An "optionally substituted hydrocarbon group", an "acyl group" and an "optionally substituted heterocyclic group" represented by R' herein may be those exemplified as those represented by $R^1$. A "leaving group" represented by $L^1$ may be one exemplified as a "leaving group" represented by L.

In this reaction, a compound represented by the formula [XV] or a salt thereof is typically used in an amount of 0.1 to 10 moles, preferably 0.5 to 5 moles, with respect to 1 mole of a compound of the formula [XIV] or a salt thereof.

The reaction solvent may be, for example, a halogen-based solvent such as dichloromethane, chloroform, etc.; an ether-based solvent such as tetrahydrofuran, diethyl ether, dimethoxyethane, etc.; an ester-based solvent such

as ethyl acetate, isobutyl acetate, etc.; a ketone-based solvent such as acetone, methyl ethyl ketone, etc.; a hydrocarbon-based solvent such as benzene, toluene, n-hexane, etc.; an alcohol-base solvent such as methanol, ethanol, isopropanol, 1-butanol, etc.; an aprotic polar solvent such as acetonitrile, N,N-dimethylformamide, dimethylsulfoxide, etc.; water, or a mixture thereof. Preference is given to ethanol, isopropanol and 1-butanol.

The reaction is facilitated favorably by addition of a base. Such base may be an amine base such as triethylamine, diisopropylethylamine, etc.; a metal hydroxide such as sodium hydroxide, potassium hydroxide, etc.; a metal carbonate such as sodium carbonate, potassium carbonate, sodium hydrogen carbonate, potassium hydrogen carbonate, etc.; or the like. Preferred are sodium carbonate, potassium carbonate, sodium hydrogen carbonate, and potassium hydrogen carbonate. The amount of the base is typically 0.1 to 10 moles, preferably 0.5 to 5 moles, with respect to 1 mole or a compound of the formula [XIV] or a salt thereof.

Furthermore, additives may also be added. Additives to be added may include sodium iodide, potassium iodide, lithium iodide or the like. The amount of the additives is typically 0.1 to 10 moles, preferably 0.5 to 5 moles, with respect to 1 mole of a compound of the formula [XIV] or a salt thereof.

The reaction temperature is -70 to 200°C, and preferably 20 to 100°C. The reaction time is typically 30 minutes to 24 hours, and preferably 30 minutes to 12 hours.

(Method G): A compound represented by the following formula [Id]:

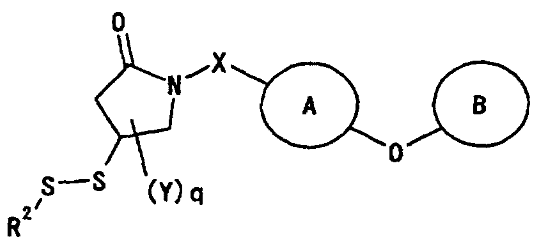

[Id]

wherein each symbol has the same meaning as defined above,

or a salt thereof, which is encompassed within the compound represented by the formula [I] of the invention or a salt thereof, can be produced by, for example, reacting a compound represented by the formula [V] or a salt thereof, with a compound represented by the following formula [XVI]:

$$R^{4c}O\ (C=O)\ S\text{-}L^2 \qquad [XVI]$$

wherein $R^{4c}$ represents an optionally substituted hydrocarbon group, and $L^2$ represents a leaving group,

or a salt thereof (Reaction 1), and reacting the resulting compound of the following formula [XVII]:

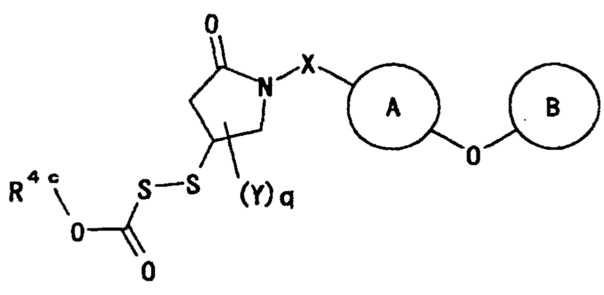

[XVII]

wherein each symbol has the same meaning as defined above, or a salt thereof with a compound represented by the following formula [XVIII]:

$$R^2\text{-SH} \qquad [XVIII]$$

wherein the symbols have the same meaning as defined above,

or a salt thereof (Reaction 2).

An "optionally substituted hydrocarbon group" represented as $R^{4c}$ herein may be exemplified by an "optionally substituted hydrocarbon group" represented by $R^1$. Further, a "leaving group" represented by $L^2$ may be exemplified by a "leaving group" represented by L.

In Reaction 1, a compound of the formula [XVI] or a salt thereof is typically used in an amount of 1 to 10 moles, preferably 1 to 1.5 moles, with respect to 1 mole of a compound of [V] or a salt thereof.

The reaction solvent for Reaction 1 may include, for example, a halogen-based solvent such as dichloromethane, chloroform, etc.; an ether-based solvent such as tetrahydrofuran, diethyl ether, dimethoxy ethane, etc.; an ester-based solvent such as ethyl acetate, isobutyl acetate, etc.; a ketone-based solvent such as acetone, methyl ethyl ketone, etc.; a hydrocarbon-based solvent such as benzene, toluene, n-hexane, etc.; an alcohol-based solvent such as methanol, ethanol, isopropanol, etc.; an aprotic polar solvent such as acetonitrile, N,N-dimethylformamide, dimethylsulfoxide, etc.; water, or a mixture thereof may be used, and preferably methanol is used.

Reaction 1 is facilitated favorably by addition of a base. Such base may be an organic base including an aliphatic amine such as trimethylamine, triethylamine, diisopropylethylamine or the like, or an aromatic amine such as pyridine (usable as a solvent) or the like; or an inorganic base including an alkali metal hydroxide such as sodium hydroxide, potassium hydroxide or the like, an alkali metal hydrogen carbonate such as sodium hydrogen carbonate, potassium hydrogen carbonate or the like, an alkali metal carbonate such as sodium carbonate, potassium carbonate or the like, a halide such as sodium iodide, potassium iodide or the like, an alkali metal hydride such as sodium hydride, potassium hydride or the like, an alkali metal amide such as sodium amide or the like, an alkoxide such as sodium methoxide, sodium ethoxide or the like. The amount of the base is 0.01 to 1 mole, preferably 0.05 to 0.1 mole, with respect to 1 mole of a compound of the formula [V] or a salt thereof.

In Reaction 1, the reaction temperature is -20 to 80°C, and preferably 0 to 30°C. The reaction time is typically 5 to 60 minutes, and preferably 30 minutes.

In Reaction 2, a compound represented by the formula [XVIII] or a salt thereof is typically used in an amount of 1 to 1.2 mole with respect to 1 mole of a compound of the formula [XVII] or a salt thereof.

In Reaction 2, the reaction solvent may be one exemplified as the reaction solvent for Reaction 1, and preference is given to methanol.

Reaction 2 is also facilitated favorably by addition of a base. Such base may be one exemplified as the base for Reaction 1, and preference is given to an aliphatic amine such as triethylamine or the like. The amount of the base is 0.1 to 10 moles, preferably 0.1 to 5 moles, with respect to 1 mole of a compound of the formula [XVII] or a salt thereof.

The reaction temperature for Reaction 2 is -20 to 80°C, and preferably 0 to 30°C. The reaction time is typically 10 minutes to 60 minutes.

(Method H) A compound represented by the formula [I] or a salt thereof according to the invention can be produced, for example, by reaction between a compound of the formula [V] or a salt and a compound represented by the following formula [XIX]:

$$R^1\text{-}L^3 \qquad \text{[XIX]}$$

wherein $L^3$ represents a leaving group, and $R^1$ has the same meaning as defined above, or a salt thereof.

A "leaving group" represented by $L^3$ herein may be exemplified by the "leaving group" represented by L.

In this reaction, a compound of the formula [XIX] or a salt thereof is typically used in an amount of 1 to 1.5 moles with respect to 1 mole of a compound of the formula [V] or a salt thereof. A compound of the formula [XIX] or a salt thereof may also be used as a solvent.

For the reaction solvent, for example, a halogen-based solvent such as dichloromethane, chloroform, etc.; an ether-based solvent such as tetrahydrofuran, diethyl ether, dimethoxy ethane, etc.; an ester-based solvent such as ethyl acetate, isobutyl acetate, etc.; a ketone-based solvent such as acetone, methyl ethyl ketone, etc.; a hydrocarbon-based solvent such as benzene, toluene, n-hexane, etc.; an alcohol-based solvent such as methanol, ethanol, isopropanol, 1-butanol, etc.; an aprotic polar solvent such as acetonitrile, N,N-dimethylformamide, dimethylsulfoxide, etc.; water, or a mixture thereof may be used. Among these, tetrahydrofuran is preferred. However, since a compound represented by the formula [XX] or a salt thereof can be used as a solvent, these reaction solvents may be unnecessary.

The reaction is facilitated favorably by addition of a base and/or an acid. Such base may be an organic base including an aliphatic amine such as trimethylamine, triethylamine, diisopropylethylamine or the like, or an aromatic amine such as pyridine (usable as a solvent) or the like; or an inorganic base including an alkali metal hydroxide such as sodium hydroxide, potassium hydroxide or the like, an alkali metal hydrogen carbonate such as sodium hydrogen carbonate, potassium hydrogen carbonate or the like, an alkali metal carbonate such as sodium carbonate, potassium carbonate or the like, a halide such as sodium iodide, potassium iodide or the like, an alkali metal hydride such as sodium hydride, potassium hydride or the like, an alkali metal amide such as sodium amide or the like, an alkoxide

such as sodium methoxide, sodium ethoxide or the like. Preference is given to an alkali metal hydride such as sodium hydride, potassium hydride or the like, an alkali metal hydroxide such as sodium hydroxide, potassium hydroxide or the like, or an aliphatic amine such as triethylamine or the like.

An acid may be an inorganic acid such as hydrochloric acid, sulfuric acid, phosphoric acid, polyphosphoric acid or the like; or an organic acid such as acetic acid, trifluoroacetic acid, trichloroacetic acid, methanesulfonic acid, benzene sulfonic acid, toluene sulfonic acid, oxalic acid, fumaric acid, maleic acid or the like. Preference is given to trifluoroacetic acid.

The amount of the base is typically 0.1 to 20 moles, preferably 0.1 to 10 moles, with respect to 1 mole of a compound of the formula [V] or a salt thereof. The amount of the acid is typically 0.1 to 5 moles with respect to 1 mole of a compound of the formula [V] or a salt thereof.

Furthermore, an additive such as an ammonium salt, a crown ether or the like may also be added. The amount of the additive is typically 0.1 to 5 moles with respect to 1 mole of a compound of the formula [V] or a salt thereof.

The reaction temperature is -20 to 80°C, and preferably 0 to 30°C. The reaction time is typically 5 minutes to 60 minutes, and preferably 5 minutes to 30 minutes.

(Method I) A compound represented by the following formula [Ie]:

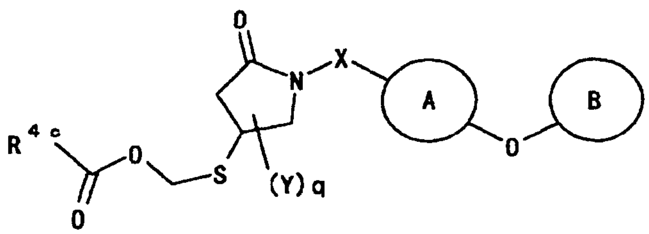

[Ie]

wherein each symbol has the same meaning as defined above, or a salt thereof, which is included in the compound represented by the formula [I] or a salt thereof according to the invention, can be produced, for example, by reacting a compound of the formula [V] or a salt thereof with a compound represented by the following formula [XX]:

$$L^4\text{-}CH_2\text{-}L^5 \qquad [XX]$$

wherein $L^4$ and $L^5$ are the same or different, and each represents a leaving group,
or a salt thereof (Reaction 1), and then reacting the resulting compound represented by the following formula [XXI] :

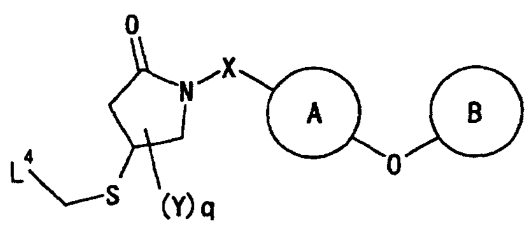

[XXI]

wherein each symbol has the same meaning as defined above,
or a salt thereof with a compound represented by the following formula [XXII]:

$$R^{4c}\text{-COOH} \qquad [XXII]$$

wherein each symbol has the same meaning as defined above, or a salt thereof (Reaction 2).

A "leaving group" represented by $L^4$ or $L^5$ herein may be one exemplified as a "leaving group" represented by L.

In Reaction 1, a compound of the formula [XX] or a salt thereof is typically used in an amount of 1 to 1.5 moles with respect to 1 mole of a compound of the formula [V] or a salt thereof. A compound represented by the formula [XX] or a salt thereof may also be used as a solvent.

For the reaction solvent for Reaction 1, one exemplified as a reaction solvent for Method H may be used.
Reaction 1 is facilitated favorably by addition of a base and/or an acid. Such base or acid may be ones exemplified as the base or acid for Method H, with the amount for each being the same as in Method H.
The reaction temperature is -20 to 80°C, and preferably 0 to 30°C. The reaction time is typically 5 minutes to 60 minutes, and preferably 5 minutes to 30 minutes.
In Reaction 2, a compound represented by the formula [XXII] or a salt thereof is typically used in an amount of 1 to 5 moles, preferably 1 to 2 moles, with respect to 1 mole of a compound represented by the formula [XXI] or a salt thereof.
The reaction solvent for Reaction 2 may be one exemplified as the reaction solvent for Method H, and preference is given to acetonitrile, N,N-dimethylformamide and tetrahydrofuran.
Reaction 2 is facilitated favorably by addition of a base. Such base may be one exemplified as a base for Method H, and preference is given to an aliphatic amine such as pyridine (may be used as a solvent), triethylamine or the like, an alkali metal hydroxide such as sodium hydroxide, potassium hydroxide or the like, an alkali metal hydrogen carbonate such as sodium hydrogen carbonate, potassium hydrogen carbonate or the like, an alkali metal carbonate such as sodium carbonate, potassium carbonate or the like, or an alkali metal hydride such as sodium hydride, potassium hydride or the like. The amount of the base is 1 to 20 moles, preferably 1 to 5 moles, with respect to 1 mole of a compound of the formula [XXI] or a salt thereof.
In Reaction 2, the reaction temperature is -20 to 80°C, and preferably 0 to 30°C. The reaction time is 5 minutes to 60 minutes, and preferably 5 minutes to 30 minutes.
(Method J) A compound represented by the following formula [If]:

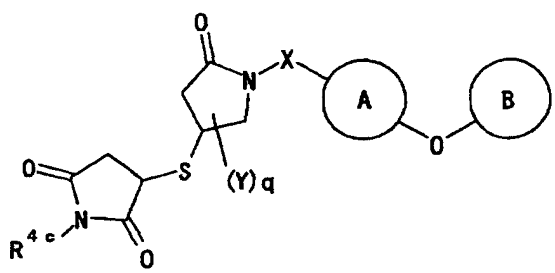

[If]

wherein each symbol has the same meaning as defined above,
or a salt thereof, which is encompassed within the compound represented by the formula [I] of the invention or a salt thereof, can be produced, for example, by reaction between a compound of the formula [V] or a salt thereof and a compound represented by the following formula [XXIII]:

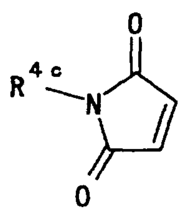

[XXIII]

wherein the symbols have the same meaning as defined above, or a salt thereof.
In this reaction, a compound of the formula [XXIII] or a salt thereof is typically used in an amount of 1 to 5 moles, preferably 1 to 2 moles, with respect to 1 mole of a compound of the formula [V] or a salt thereof.
The reaction solvent may be one exemplified as a reaction solvent for Method H, and preference is given to a halogen-based solvent such as dichloromethane and chloroform, tetrahydrofuran or the like.
This reaction is facilitated favorably by addition of a base. Such base may be one exemplified as a base for Method H, and preference is given to an aliphatic amine such as pyridine (may be used as a solvent), triethylamine or the like. The amount of the base is 0.01 to 5 moles, preferably 0.1 to 2 moles, with respect to 1 mole of a compound of the formula [V] or a salt thereof.
The reaction temperature is -20 to 80°C, and preferably 0 to 30°C. The reaction time is 5 minutes to 60 minutes, and preferably 5 minutes to 30 minutes.
(Method K) A compound represented by the following formula [Ig]:

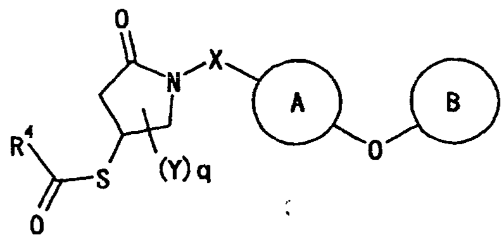

[Ig]

wherein each symbol has the same meaning as defined above,
or a salt thereof, which is encompassed within the compound represented by the formula [I] of the invention or a salt thereof, can be produced, for example, by reacting a compound represented by the formula [VII] or a salt thereof with a compound represented by the following formula [XXIV] :

$$R^4\text{-COOH} \quad [XXIV]$$

wherein each symbol has the same meaning as defined above, or a salt thereof.

In this reaction, a compound of the formula [XXIV] or a salt thereof is typically used in an amount of 1 to 10 moles, preferably 1 to 3 moles, with respect to 1 mole of a compound of the formula [VII] or a salt thereof.

The reaction solvent may be one exemplified as a reaction solvent for Method H, and preference is given to pyridine, tetrahydrofuran, and acetonitrile as well as a halogen-based solvent such as dichloromethane, chloroform or the like.

This reaction is facilitated favorably by addition of a base. Such base may be one exemplified as a base for Method H, and preference is given to an aliphatic amine such as pyridine (may be used as a solvent), triethylamine or the like. The amount of the base is 1 to 5 moles, preferably 1 to 2 moles, with respect to 1 mole of a compound of the formula [VII] or a salt thereof.

Furthermore, the reaction is also facilitated favorably by addition of a reducing agent. Such reducing agent may be one exemplified as a reducing agent for Method B', and preference is given to a trialkylphosphine such as trimethylphosphine, tributylphosphine or the like. The amount of the reducing agent employed is 1 to 5 moles, preferably 1 to 1.5 moles with respect to 1 mole of a compound of the formula [VII] or a salt thereof.

The reaction temperature is -20 to 80°C, and preferably 0 to 30°C. The reaction time is 30 minutes to 240 minutes, and preferably 30 minutes to 120 minutes.

When compound (I) is obtained by any of the methods described above as a free form, it may be converted in accordance with a standard procedure into a pharmacologically acceptable salt with, for example, an inorganic acid (e.g., hydrochloric acid, sulfuric acid and hydrobromic acid, etc.), an organic acid (e.g., methanesulfonic acid, benzenesulfonic acid, toluenesulfonic acid, oxalic acid, fumaric acid, maleic acid and tartaric acid, etc.), an inorganic base (e.g., an alkali metal such as sodium, potassium, etc.; an alkali earth metal such as calcium, magnesium, etc.; aluminum, ammonium, etc.), an organic base (e.g., trimethylamine, triethylamine, pyridine, picoline, ethanolamine, diethanolamine, triethanolamine, dicyclohexylamine and N,N'-dibenzylethylenediamine, etc.), a basic amino acid (e.g., arginine, lysine, ornithine, etc.), an acidic amino acid (e.g., aspartic acid, glutamic acid, etc.) or the like. Likewise, when compound [I] is obtained as a salt, it may be converted into a free form or other salts according to a standard procedure.

Compound [I] or a salt thereof thus obtained can be purified and recovered by means of any of separation/purification methods known per se (e.g., concentration, solvent extraction, column chromatography, recrystallization, etc.).

A starting compound for compound [I] of the present invention may be in the form of a salt, and such salt includes, for example, a salt with an inorganic acid (e.g., hydrochloric acid, phosphoric acid, hydrobromic acid, sulfuric acid, etc.), a salt with an organic acid (e.g., acetic acid, trifluoroacetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) or the like. Furthermore, when any of these compounds carries an acidic group such as -COOH or the like, a salt with an inorganic base (e.g., an alkali metal or an alkali earth metal such as sodium, potassium, calcium or magnesium, ammonia, etc.) or with an organic base (e.g., tri-$C_{1-6}$ alkylamine such as triethylamine, etc.) may be formed.

In each of the reactions described above, when a starting compound carries as a substituent an amino group, a carboxy group or a hydroxy group, such group may be derivatized with a protective group employed ordinarily in

peptide chemistry, which is cleaved after the reaction, if necessary, to yield the desired compound.

A protective group for an amino group may be, for example, an optionally substituted $C_{1-6}$ alkylcarbonyl group (e.g., formyl, methylcarbonyl, ethylcarbonyl, etc.), a phenylcarbonyl group, a $C_{1-6}$ alkyloxycarbonyl group (e.g., methoxycarbonyl, ethoxycarbonyl, etc.), a $C_{6-14}$ aryloxycarbonyl group (e.g., phenyloxycarbonyl, etc.), a $C_{7-10}$ aralkyloxycarbonyl group (for example, benzyloxycarbonyl, etc.), a trityl group, a phthaloyl group or the like. A substituent on each of the groups listed above may be a halogen atom (e.g., fluorine, chlorine, bromine and iodine, etc.), a $C_{1-6}$ alkylcarbonyl group (e.g., methylcarbonyl, ethylcarbonyl, butylcarbonyl, etc.), a nitro group or the like, and the number of such substituents is 1 to about 3.

A protective group for a carboxy group may be, for example, an optionally substituted $C_{1-6}$ alkyl group (e.g., methyl, ethyl, n-propyl, i-propyl, n-butyl, tert-butyl, etc.), a phenyl group, a trityl group, a silyl group or the like. A substituent on each of the groups listed above may be a halogen atom (e.g., fluorine, chlorine, bromine, iodine, etc.), a $C_{1-6}$ alkylcarbonyl group (e.g., formyl, methylcarbonyl, ethylcarbonyl, butylcarbonyl, etc.), or a nitro group, and the number of such substituents is 1 to about 3.

A protective group for a hydroxy group may be, for example, an optionally substituted $C_{1-6}$ alkyl group (e.g., methyl, ethyl, n-propyl, i-propyl, n-butyl, tert-butyl, etc.), a phenyl group, a $C_{7-10}$ aralkyl group (e.g., benzyl, etc.), a $C_{1-6}$ alkylcarbonyl group (e.g., formyl, methylcarbonyl, ethylcarbonyl, etc.), a $C_{6-14}$ aryloxycarbonyl group (e.g., phenyloxycarbonyl, etc.), a $C_{7-10}$ aralkyloxycarbonyl group (e.g., benzyloxycarbonyl, etc.), a pyranyl group, a furanyl group, a silyl group or the like. A substituent on each of the groups listed above may be a halogen atom (e.g., fluorine, chlorine, bromine, iodine, etc. ) , a $C_{1-6}$ alkyl group, a phenyl group, a $C_{7-10}$ aralkyl group, a nitro group or the like, the number of the substituents being 1 to about 4.

For the removal of a protecting group, a method known per se or a method corresponding thereto may be used, such as treatment with an acid, a base, a reducing agent, UV light, hydrazine, phenylhydrazine, sodium N-methyldithiocarbamate, tetrabutylammonium fluoride, palladium acetate or the like.

Moreover, for each of the above-described reactions, when the starting compound carries as a substituent a functionality capable of conversion (e.g., a carboxy group, an amino group, a hydroxy group, a carbonyl group, a thiol group, an ester group, a sulfo group, a halogen atom, etc.), a variety of compounds can be produced by converting the functionality according to a method known per se, for example, a method described in "Organic Functional Group Preparations, 2nd Edition, ACADEMIC PRESS, INC. (1989) " or "Comprehensive Organic Transformations, VCH Publishers, Inc. (1989)", or a method corresponding thereto.

For example, when substituent is a carboxy group, conversion thereof can be carried out by a reaction such as esterification, reduction, amidation, conversion into an optionally protected amino group or the like. When the substituent is an amino group, conversion thereof can be carried out by a reaction such as amidation, sulfonylation, conversion into a nitroso group, alkylation, arylation, imidation or the like. When the substituent is a hydroxy group, conversion thereof can be carried out by a reaction such as esterification, carbamoylation, sulfonylation, alkylation, arylation, oxidation, halogenation or the like. When the substituent is a carbonyl group, conversion thereof can be carried out by a reaction such as reduction, oxidation, conversion into an imino group (including oximation and hydrazonation), (thio)ketalation, conversion into alkylidene, thiocarbonylation or the like. When the substituent is a thiol group, conversion thereof can be carried out by a reaction such as alkylation, oxidation or the like. When the substituent is an ester group, conversion thereof can be carried out by a reaction such as reduction, hydrolysis or the like. When the substituent is a sulfo group, conversion thereof can be carried out by a reaction such as sulfonamidation, reduction or the like. When the substituent is a halogen atom, conversion thereof can be carried out by a reaction such as various nucleophilic substitutions, various coupling reactions or the like.

When compound [I] of the invention may be used in the form of prodrug, and such prodrug denotes a compound that is converted to compound [I] under in vivo physiological conditions by a reaction with an enzyme, gastric acid or the like, that is, a compound that is converted to compound [I] by enzymatic oxidation, reduction, hydrolysis or the like, or a compound that is converted to compound [I] by hydrolysis induced by gastric acid or the like. A prodrug of compound [I] may include, for example, a compound resulting from acylation, alkylation or phophorylation of an amino group of compound [I] (e.g., a compound resulting from conversion of an amino group of compound [I] to an eicosanoyl, alanyl, pentylaminocarbonyl, (5-methyl-2-oxo-1,3-dioxolen-4-yl) methoxycarbonyl, tetrahydrofuranyl, pyrrolidylmethyl, pivaloyloxymethyl or tert-butyl group); a compound resulting from acylation, alkylation, phosphorylation or borylation of a hydroxy group of compound [I] (e.g., a compound resulting from conversion of a hydroxy group of compound [I] to an acetyl, palmitoyl, propanoyl, pivaloyl, succinyl, fumaryl, alanyl or dimethyl aminomethylcarbonyl group); a compound resulting from esterification or amidation of a carboxy group of compound [I] (e.g., a compound resulting from conversion of a carboxy group of compound [I] to an ethyl ester, phenyl ester, carboxymethyl ester, dimethyl aminomethyl ester, pivaloyl oxymethyl ester, ethoxycarbonyl oxyethyl ester, phthalidyl ester, (5-methyl-2-oxo-1,3-dioxolen-4-yl) methyl ester, cyclohexyloxycarbonyl ethyl ester or methylamide group); a compound in which substituent $R^1S$- of compound [I] is $R^1SO$- or $R^1SO_2$-; or the like. Such compound can be produced from compound [I] according to a method known per se.

Furthermore, the prodrug of Compound (I) may be a compound which is converted into Compound (I) under the physiological conditions as described in "Pharmaceutical Research and Development", Vol. 7 (Molecular Design), pages 163-198 published in 1990 by Hirokawa Publishing Co. (Tokyo, Japan).

Compound [I] of the invention has an excellent MMP inhibiting effect, especially an MMP-13 inhibiting effect.

In addition, compound [I] of the invention has low toxicity and is safe.

Accordingly, compound [I] of the invention having an excellent MMP inhibiting effect, especially an MMP-13 inhibiting effect, is useful in a mammal (e.g., mouse, rat, hamster, rabbit, cat, dog, cattle, sheep, monkey, human or the like) as a safe prophylactic and therapeutic agent against all MMP-related diseases, for example, articular diseases (e.g., osteoarthritis, rheumatoid arthritis (chronic rheumatoid arthritis), etc.), osteoporosis, cancer (e.g., primary, metastatic or recurrent cancers in the breast, prostate, pancreas, stomach, lung, large intestine (colon, rectum, anus), esophagus, duodenum and neck (tongue, pharynx, larynx), tumors such as brain tumor, Schwannoma, non-small cell lung carcinoma, pulmonary small cell carcinoma, hepatoma, nephroma, cholangioma, uterine cancer (uterine cancer, cervical cancer), ovarian cancer, bladder cancer, skin cancer, angioma, malignant lymphoma, malignant melanoma, thyroid cancer, bone tumor, hemangioma, angiofibroma, retinal sarcoma, penile cancer, juvenile tumor, malignant skin carcinoma, malignant skin carcinoma induced by AIDS, maxillary sinus carcinoma, fibrous histiocytoma, leiomyosarcoma, rhabdomyosarcoma, liposarcoma, uterine myoma, osteoblastoma, osteosarcoma, chondrosarcoma, cancerous mesothelial tumor, leukemia, etc.), periodontosis, corneal ulcer, chronic ulcer pathologic bone resorption (such as Paget's disease), nephritis, angiogenesis, aneurysm, arteriosclerosis, pulmonary emphysema, chronic obstructive pulmonary diseases (COPD), cirrhosis, autoimmune disease (Crohn's disease, Sjogren's disease, etc.), or infiltration and metastasis of cancers, or as a contraceptive.

A formulation containing compound [I] of the invention may be in various dosage forms such as a solid form such as powder, granule, tablet, capsule or the like, or a liquid form such as syrup, emulsion, solution injection or the like.

A prophylactic and therapeutic formulation according to the invention can be produced by a conventional method such as mixing, kneading, granulation, tabletting, coating, sterilization, emulsification or the like, depending on the type of the formulation. In addition, for the preparation of a formulation, reference may be made, for example, to each section in the General Rules for the formulation under the Japanese Pharmacopeia. Further, the formulation according to the invention may be formed into a sustained-release formulation containing effective components and biodegradable polymer compounds. Such sustained-release formulation can be produced according to a method described in the publication of JP-A-9-263545.

The content of compound [I] in a formulation of the invention may vary depending on the type of the formulation, but typically is 0.01 to 100% by weight, preferably 0.1 to 50% by weight, and more preferably 0.5 to 20% by weight based on the total weight of the formulation.

When compound [I] of the invention is used as a pharmaceutical described above, it can be used as it is, or as a mixture produced by a conventional method with a suitable pharmacologically acceptable carrier such as an excipient (e.g., starch, lactose, sugar, calcium carbonate, calcium phosphate, etc.), a binder (e.g., starch, gum arabic, carboxymethyl cellulose, hydroxypropyl cellulose, crystalline cellulose, alginic acid, gelatin, polyvinylpyrrolidone, etc.), a lubricant (e.g., stearic acid, magnesium stearate, calcium stearate, talc, etc.), a disintegrant (e.g., calcium carboxymethyl cellulose, talc, etc.), a diluent (e.g., water for injection, physiological saline, etc.); appropriate additives, if necessary (e.g., stabilizer, preservative, colorant, flavor, solubilizing agent, emulsifier, buffer, isotonic agent, etc.) or the like, in a solid dosage form such as powder, fine powder, granules, tablets, capsules and the like, as well as a liquid form such as an injection formulation, such formulation being administered orally or parenterally. Further, compound [I] can be administered in the form of a formulation for local administration or directly on the diseased part having an articular disease. In the latter case, injection is preferred. There can also be administrated as a parenteral formulation for local administration (e.g., a formulation for injection for intramuscular, subcutaneous, intraorgan and on-site (in the vicinity of a joint) routes, a solid form such as capsule, granule, powder, a liquid form such as suspension, an ointment, etc.).

For example, a practical formulation for injection can be obtained by mixing compound [I] with a dispersant (e.g., surfactant such as Tween 80, HCO-60, etc., carboxymethyl cellulose, sodium alginate, polysaccharide such as hyaluronic acid, polysorbate, etc.), a preservative (e.g., methylparaben, propylparaben, etc.), an isotonic agent (e.g., sodium chloride, mannitol, sorbitol, glucose, etc.), a buffer (e.g., calcium carbonate, etc.), a pH adjusting agent (e.g., sodium phosphate, potassium phosphate, etc.) or the like, into an aqueous suspension. Further, thus obtained formulation is made into a practically usable formulation for injection by dispersing it with an oil of plant origin such as sesame oil or corn oil, or with a mixture thereof with phospholipids such as lecithin, or with medium chain triglyceride (e.g., Miglyol 812, etc.) as in a suspension in oil.

In particular, when such formulation is administered directly into the joint cavity of a patient suffering from an articular disease for local administration, the formulation can be produced by dispersing compound [I] in a hyaluronic acid preparation for injection (for example, product by Kaken Pharmaceutical Co., Ltd.; Arts ) as dispersing medium. The hyaluronic acid used in the dispersing medium may be a non-toxic salt thereof, and examples may include an alkali metal salt such as sodium, potassium or the like, or an alkali earth metal salt such as magnesium, calcium or

the like, a sodium salt being particularly preferred. The molecular weight of hyaluronic acid or a non-toxic salt thereof is about 200,000 to about 5,000,000 (measured by the viscosity method), preferably about 500,000 to about 3,000,000, and more preferably about 700,000 to 2,500,000.

The final concentration of hyaluronic acid or sodium hyaluronate in the dispersion is suitably less than 1% (W/V) in the aspects of easy handlability and administration, preferably from about 0.02 to less than 1%, and even more preferably from about 0.1 to less than 1% (W/V) .

The dispersing medium may contain a pH adjusting agent, local anesthetic, antibiotics, a solubilizing agent, an isotonic agent, anti-adsorption agent, glycosaminoglycan, polysaccharides or the like by a method known per se. Preferred examples may include mannitol, sorbitol, table salt, glycine, ammonium acetate, or an aqueous protein that can be administered into body fluid without exhibiting any substantial pharmacological activity. The glycosaminoglycan may include hyaluronic acid, chondroitin, chondroitin sulfate A, chondroitin sulfate C, dermatan sulfate, heparin, heparan sulfate, keratan sulfate or the like. The polysaccharide may include an acidic polysaccharide such as arginic acid.

The above-mentioned aqueous protein may be anything that is soluble in water, a physiological saline or a buffer, and examples include human serum albumin, human serum globulin, collagen, gelatin or the like. The pH adjusting agent may include, for example, glycine, ammonium acetate, citric acid, hydrochloric acid, sodium hydroxide or the like. The local anesthetic may include, for example, chlorobutanol, xylocaine hydrochloride or the like. The antibiotic may include, for example, gentamicin or the like. The solubilizing agent may include, for example, glycerin, polyethylene glycol 400 or the like. The isotonic agent may include, for example, mannitol, sorbitol, sodium chloride or the like. The anti-adsorption agent may include, for example, polyoxyethylenesorbitan monooleate or the like.

Furthermore, when the dispersing medium contains an aqueous protein, the content of the aqueous protein in the preparation for a single dose is preferably 0.05 to 50 mg, more preferably 0.5 to 20 mg, and even more preferably 0.75 to 10 mg. Such preparation may contain phosphoric acid or a salt thereof (e.g., sodium phosphate, potassium phosphate, etc.).

When a preparation for injection contains phosphoric acid or a salt thereof, the concentration of sodium phosphate or potassium phosphate in the preparation for injection is about 0.1 mM to 500 mM, and preferably about 1 mM to 100 mM.

Sterilization of a preparation may be carried out by operating the entire production process under aseptic conditions, sterilizing with $\gamma$-rays, adding a preservative, or the like, without being particularly limited.

The prophylactic and therapeutic agent of the invention can be used in combination with other agents. For example, when compound [I] is used as a therapeutic agent for articular diseases, it can be used in combination with (i) a cyclooxygenase inhibitor (Cox-I or Cox-II inhibitor), (ii) a disease-modified anti-rheumatoid drug and immune suppressant, (iii) a biological formulation, (iv) an analgesic and anti-inflammatory agent, (v) a therapeutic agent for bone diseases, (vi) p38MAP kinase inhibitor and/or an inhibitor of biosynthesis of polymer substances by TNF-$\alpha$, or (vii) c-JUN N-terminal kinase (JNK) inhibitor. Further, when compound [I] is used as an anticancer agent, it can be used in combination with (viii) other anticancer agents.

A cyclooxygenase inhibitor (i) (Cox-I or Cox-II inhibitor) may include, for example, a salicylic acid derivative such as aspirin, Celecoxib, Rofecoxib, or the like, Diclofenac, Indomethacin, Loxoprofen or the like.

A disease-modified anti-rheumatoid drug and immune suppressant (ii) may include, for example, methotrexate, Leflunomid, Prograf, sulfasalazine, D-penicilamine, oral metallic agent, T-cell differentiation controlling agent or the like.

A biological formulation (iii) may include, for example, a monoclonal antibody (e.g., anti-TNF-$\alpha$ antibody, anti-IL-12 antibody, anti-IL-6 antibody, anti-ICAM-I antibody, ani-CD4 antibody, etc.), soluble receptor (e.g., soluble TNF-$\alpha$ receptor, etc.), proteinaceous ligand (IL-1 receptor antagonist, etc.) or the like.

An analgesic and anti-inflammatory agent (iv) may include, for example, centrally active analgesic (e.g., morphine, codeine, pentazocine, etc.), steroids (e.g., prednisolone, hydrocortisone, methylprednisolone, dexamethasone, betamethasone, etc.), anti-inflammatory enzymes (e.g., bromelain, lysozyme, proctase, etc.) or the like.

A therapeutic agent for bone diseases (v) (e.g., fracture, refracture, osteoporosis, osteomalacia, Paget's disease, spastic myelitis, chronic articular rheumatism, destruction of articular tissues in modified gonarthritis and its analogous diseases, etc.) may include, for example, a calcium preparation (e.g., calcium carbonate, etc.), a calcitonin preparation, a vitamin D preparation (e.g., alpha-calcidol, etc.), sex hormones (e.g., estrogen, estradiol, etc.), prostaglandin $A_1$, bisphosphonic acids, ipriflavones, fluorine compounds (e.g., sodium fluoride, etc.), vitamin $K_2$, bone morphogenic proteins (BMP), fibroblast growth factor (FGF), platelet-derived growth factor (PDGF), transforming growth factor (TGF-$\beta$), insulin-like growth factor-1 and -2 (IGF-1 and -2), parathyroid hormone (PTH) or the like.

A p38MAP kinase inhibitor and/or an inhibitor of biosynthesis of polymer substances by TNF-$\alpha$ (vi) may include compounds described in the publications of, for example, WO98/57966, WO98/56377, WO98/25619, WO98/07425, WO98/06715, US5739143, WO97/35855, WO97/33883, WO97/32583, WO97/25048, WO97/25046, WO96/10143, WO96/21654, WO95/07922, WO00/09525, WO99/17776, WO99/01131, WO98/28292, WO97/25047, WO97/25045,

US5658903, WO96/21452, WO99/18942, US5756499, US5864036, US6046208, US5716955, US5811549, US5670527, US5969184, WO00/31072, WO00/31063, WO00/20402, WO00/18738, WO00/17175, WO00/12497, WO00/12074, WO00/07991, WO00/07980, WO00/02561, US6096711, WO99/64400, WO99/61440, WO99/59959, WO99/58523, WO99/58502, WO99/57101, WO99/32111, WO99/32110, WO99/26657, WO99/20624, WO99/18942, WO99/15164, WO99/00357, WO98/52940, WO98/52937, WO98/52558, WO98/06715, WO97/22256, WO96/21452, WO00/43366, WO00/42003, WO00/42002, WO00/41698, WO00/41505, WO00/40243, WO00/34303, WO00/25791, WO00/17204, WO00/10563, US6080546, WO99/61426, WO99/32463, WO99/32121, WO99/17776, WO98/28292, WO98/27098, WO98/25619, WO98/20868, WO97/35855, WO97/32583, WO97/25048, WO97/25047, WO97/25046, WO97/25045, US5658903, WO96/40143, WO96/21654, WO00/55153, WO00/55120, WO00/26209, US6046208, US5756499, US5864036, JP-A-2000-86657, WO99/59960, WO99/21859, WO99/03837, WO99/01449, WO99/01136, WO99/01130, US5905089, WO98/57966, WO98/52941, WO98/47899, WO98/07425, WO97/33883, WO00/42213, WO99/58128, WO00/04025, WO00/40235, WO00/31106, WO97/46228, WO00/59904, WO00/42003, WO00/42002, WO00/41698, WO00/10563, WO00/64894, WO99/61426, WO99/32463, US6002008, WO98/43960, WO98/27098, WO97/35856, WO97/35855, WO96/22985 and JP-A-61-145167.

A JNK inhibitor (vii) may include compounds described in the publications of, for example, WO00/35906, WO00/35909, WO00/35921, WO00/64872, WO00/75118 and WO02/62792.

An anticancer agent (viii) may include, for example, 6-O-(N-chloroacetylcarbamoyl)fumagillol, bleomycin, methotrexate, actinomycin D, mitomycin C, daunorubicin, adriamycin, neocartinostatin, cytosine arabinoside, fluorouracil, tetrahydrofuryl-5-fluorouracil, picibanil, lentinan, levamisole, bestatin, azimexone, glytilitin, doxorubicin hydrochloride, acrarubicin hydrochloride, bleomycin hydrochloride, hepromycin sulfate, vincristine sulfate, vinblastine sulfate, irinotecan hydrochloride, cyclophosphamide, melphalan, busulphan, thiotepa, procarbazine hydrochloride, cisplatin, azathioprine, mercaptopurine, tegafur, carmofur, cytarabine, methyl testosterone, propionic acid testosterone, testosterone enanthate, mepitiostan, fosfestrol, chlormadinone acetate, leuprorelin acetate, buserelin acetate or the like.

When used in combination, the administration interval for compound [I] and the combination drug is not particularly limited. Compound [I] or a pharmaceutical composition thereof, and the drug for combination or a pharmaceutical composition thereof, may be administered to a subject of administration either simultaneously or with a time interval. The dose of the drug for combination may be determined to be in accordance with the clinically used dose, and can be suitably selected according to the subject of administration, administration route, type of disease, organization of the like.

The form of administration for combination is not particularly limited, and compound [I] and the drug for combination may be combined at the time of administration. Such form of administration may include, for example, (1) administration of a single preparation which can be obtained by simultaneously formulating compound [I] or a pharmaceutical composition thereof with the drug for combination or a pharmaceutical composition thereof; (2) simultaneous administration by an identical route of administration, of two preparations which can be obtained by separately formulating compound [I] or a pharmaceutical composition thereof and the drug for combination or a pharmaceutical composition thereof; (3) administration with a time interval by an identical route of administration, of two preparations which can be obtained by separately formulating compound [I] or a pharmaceutical composition thereof with the drug for combination or a pharmaceutical composition thereof; (4) simultaneous administration by different routes of administration, of two preparations which can be obtained by separately formulating compound [I] or a pharmaceutical composition thereof with the drug for combination or a pharmaceutical composition thereof; (5) administration with a time interval by different routes of administration, of two preparations which can be obtained by separately formulating compound [I] or a pharmaceutical composition thereof with the drug for combination or a pharmaceutical composition thereof (for example, administration in the order of compound [I] or a pharmaceutical thereof and then the drug for combination or a pharmaceutical thereof, or administration in the reverse order), or the like.

The mixing ratio for compound [I] with the drug for combination in the combination of the invention can be appropriately selected according to the subject of administration, the administration route, type of disease or the like.

For example, the content of compound [I] present in the combination of the invention may vary depending on the type of preparation, but it is typically about 0.01 to 100% by weight, preferably about 0.1 to about 50% by weight, and more preferably about 0.5 to about 20% by weight, with respect to the whole preparation.

The content of the drug for combination present in the combination of the invention may vary depending on the type of preparation, but it is typically about 0.01 to 100% by weight, preferably about 0.1 to about 50% by weight, and more preferably about 0.5 to about 20% by weight, with respect to the whole preparation.

The content of an additive such as carrier present in the combination of the invention may vary depending on the type of preparation, but it is typically about 1 to 99.99% by weight, and preferably about 10 to 90% by weight, with respect to the whole preparation.

When compound [I] and the drug for combination are each separately formulated, the same contents may be used.

The dose may vary depending on the type of compound [I] or a pharmaceutically acceptable salt thereof, the administration route; symptoms, age of the patient, but in the case of administering orally to an adult patient having osteoarthritis, for example, the daily dose of compound [I] per kg of body weight is about 0.005 to 50 mg, preferably about 0.05 to 10 mg, and more preferably about 0.2 to 4 mg, which can be administered in portions in 1 to 3 times.
When the pharmaceutical composition of the invention is a sustained-release formulation, the dose may vary depending on the type and content of compound [I], type of formulation, the duration of drug release, the animal subject of administration (e.g., mammals such as human, rat, mouse, cat, dog, rabbit, cattle, pig, etc.) and purpose of administration, but in the case of parenteral administration, the release amount from the preparation is preferably from about 0.1 mg to about 100 mg of compound [I] per week.
For a drug for combination, the dose can be set to any level within the scope that side-effects do not occur. The daily dose for a drug for combination may vary depending on the extent of symptoms, the age, gender, body weight, sensitivity of the subject of administration, the time and interval of administration, the properties, formulation, type of the pharmaceutical preparation, type of the active ingredient, or the like and may not be particularly limited. However, the dose of the drug , for example, in oral administration to a mammal is typically about 0.001 to 2000 mg, preferably about 0.01 to 500 mg, and more preferably about 0.1 to 100 mg per kg of body weight, which is typically administered in portions in 1 to 4 times.
When an agent for combination of the invention is administered, compound [I] and a drug for combination may be administered simultaneously; a drug for combination may be first administered, followed by compound [I]; or compound [I] may be administered first, followed by a drug for combination. When the administration is carried out with a time interval, the time interval may vary depending on the effective component to be administered, formulation, route of administration or the like. For example, when a drug for combination is first administered, compound [I] can be administered within 1 minute to 3 days from the point of administration of the drug for combination, preferably within 10 minutes to 1 day, and more preferably within 15 minutes to 1 hour. When compound [I] is administered first, a drug for combination can be administered within 1 minute to 1 day from the point of administration of compound [I], preferably within 10 minutes to 6 hours, and more preferably within 15 minutes to 1 hour.
The pharmaceutical composition of the invention is low in toxicity and thus can be used safely. In particular, the compounds of the Examples shown below exhibit excellent absorption when administered orally, and thus can be used advantageously as an oral preparation.

## Best Mode for Carrying Out the Invention

## EXAMPLES

The present invention will be further described in detail in the following Examples and Comparative Examples, which are not intended to restrict the invention and may be modified without departing from the scope of the invention.
An elution in column chromatography in the each Example was performed under the observation by a TLC (Thin Layer Chromatography), unless otherwise specified. TLC was performed using a $60F_{254}$ from Merck as a TLC plate. Detection was made by an UV detector or by means of a color development with a phosphomolybdic acid or a ninhydrin reagent. Silica gel 60 (70 to 230 mesh size) from Merck was employed as silica gel for column chromatography. A $60F_{254}$ plate from Merck was employed as preparative TLC plate. A room temperature referred herein typically means a temperature from about 10°C to 35°C.
NMR (Nuclear Magnetic Resonance) spectra were measured using a VARIAN model Gemini-200 spectrometer ($^1$H-NMR: 200 MHz or 300 MHz) or a BRUKER model DPX-300 spectrometer ($^1$H-NMR: 300 MHz). An internal standard was tetramethylsilane and all δ values are represented in ppm. Abbreviations employed here are described below.
$CDCl_3$: deutero chloroform, DMSO-$d_6$: deutero dimethylsulfoxide, Hz: Herz, J: Coupling constant, m: Multiplet, q: Quartet, t: Triplet, d: Doublet, s: Singlet, br: Broad, dd: Double doublet, dq: Double quartet.

Example 1

S-((3R)-1-{[6-(4-fluorophenoxy)pyridin-3-yl]methyl}-5-oxopyrrolidin-3-yl)ethanethioate

[Process 1]

Step 1

Synthesis was performed with reference to the method described in WO97/37990.
0.14 g (1.0 mmol) of 6-chloronicotinonitrile was added to a mixture of 0.11 g (1.0 mmol) of 4-fluorophenol,

0.12 g (1.1 mmol) of potassium tert-butoxide and 2 mL of N,N-dimethylformamide (DMF). The reaction mixture was stirred at room temperature for 0.5 hour, at 60°C for 0.5 hour, at 100°C for 1 hour, and at 150°C for 2 hours. It was then cooled to room temperature and diluted by addition of water, followed by extraction with ethyl acetate-n-hexane (10:1). The organic layer was washed with saturated brine and then dried over anhydrous magnesium sulfate. The solvent was distilled off under reduced pressure to give a residue which was then purified by silica gel column chromatography (n-hexane:ethyl acetate = 5:1) to yield 0.18 g (84%) of 6-(4-fluorophenoxy)nicotinonitrile.

Melting Point 120 - 121°C.

Step 2

A mixture of 21.4 g (100.0 mmol) of 6-(4-fluorophenoxy)nicotinonitrile, 2.0 g of Raney nickel, 30 mL of 28% aqueous ammonia, 200 mL of ethanol and 400 mL of tetrahydrofuran (THF) was stirred vigorously for 64 hours under hydrogen atmosphere. The insolubles were filtered off, and the solvent was distilled off under reduced pressure to give a residue. Thus obtained residue was dissolved in ethyl acetate and dried over anhydrous magnesium sulfate. The solvent was distilled off under reduced pressure to yield 21.82 g (99%) of 1-[6-(4-fluorophenoxy)pyridin-3-yl]methaneamine.

Melting Point 46 - 47°C.

Step 3

0.44 g (2.0 mmol) of 1-[6-(4-fluorophenoxy)pyridin-3-yl]methaneamine was added to a mixture of 0.38 g (2.2 mmol) of (S)-O-acetyl malic anhydride in 1 mL of THF and 4 mL of acetonitrile, and the reaction mixture was stirred at room temperature for 1.5 hours. Then, the solvent was distilled off under reduced pressure to give a residue which was then dissolved in 4 mL of acetyl chloride and stirred at 60°C for 0.5 hour. The solvent was distilled off under reduced pressure to give a residue to which was added ice-water, and the mixture was extracted with ethyl acetate, washed with saturated brine and dried over magnesium sulfate. The solvent was distilled off under reduced pressure to give a residue which was purified by silica gel column chromatography (n-hexane:ethyl acetate = 1:1) to yield 0.70 g (98%) of (3S)-1-{[6-(4-fluorophenoxy)pyridin-3-yl]methyl}-2,5-dioxopyrrolidin-3-yl acetate.

IR(KBr): 1748, 1717, 1607, 1505, 1478 $cm^{-1}$.

Step 4

To a mixed solution of 0.69 g (1.93 mmol) of (3S)-1-{[6-(4-fluorophenoxy)pyridin-3-yl]methyl}-2,5-dioxopyrrolidin-3-yl acetate in 6 mL of THF-6 mL of ethanol, 0.073 g (1.93 mmol) of sodium borohydride was added at -19°C. The reaction mixture was stirred at -19 to -13°C for 15 hours, followed by addition of 16 mL of saturated aqueous sodium hydrogen carbonate, and the mixture was extracted with ethyl acetate. The organic layer was washed with saturated brine and dried over anhydrous magnesium sulfate, and the solvent was distilled off under reduced pressure. Thus obtained residue was dissolved in 4 mL of trifluoroacetic acid, followed by addition of 0.22 g (1.93 mmol) of triethylsilane, and the mixture was stirred for 1 hour, followed by addition of 0.22 g (1.93 mmol) of triethylsilane. After stirring at room temperature for 1 hour, the solvent was distilled off under reduced pressure to give a residue which was then dissolved in ethyl acetate, and ice-water and saturated aqueous sodium hydrogen carbonate were added thereto for neutralization. The organic layer was washed with saturated brine and dried over anhydrous magnesium sulfate, and the solvent was distilled off under reduced pressure. Thus obtained residue was dissolved in 6 mL of ethanol, and 38.6 mL (2.74 mmol) of acetyl chloride was added dropwise thereto. The reaction mixture was stirred at 60°C for 1 hour, the solvent was distilled off under reduced pressure to give a residue, to which was added ice-water, and the mixture was extracted with ethyl acetate. The organic layer was washed with saturated brine and dried over anhydrous magnesium sulfate, and the solvent was distilled off under reduced pressure to give a residue which was purified by silica gel column chromatography (chloroform:methanol = 10:1) to yield 0.27 g (46%) of (4S)-1-{[6-(4-fluorophenoxy)pyridin-3-yl]methyl}-4-hydroxypyrrolidin-2-one.

Melting point 104 - 105°C.

Step 5

To a solution of 0.26 g (0.86 mmol) of (4S)-1-{[6-(4-fluorophenoxy)pyridin-3-yl]methyl}-4-hydroxypyrrolidin-2-one, 0.14 g (1.20 mmol) of methanesulfonyl chloride in 1 mL of chloroform, a solution of 0.12 g (1.20 mmol) of triethylamine in 1 mL of chloroform was added dropwise at 0°C, the mixture was stirred at 0°C for 10 minutes, and the solvent was distilled off under reduced pressure to give a residue to which was then added ice-water and the mixture was extracted with ethyl acetate. The organic layer was washed with saturated brine and dried over anhydrous magnesium sulfate, and the solvent was distilled off under reduced pressure to yield 0.36 g of (3S)-1-{[6-(4-flourophenoxy) pyridin-3-yl]methyl}-5-oxopyrrolidin-3-yl methanesulfonate.

IR(KBr): 1696, 1607, 1578, 1505, 1478 $cm^{-1}$.

Step 6

To a solution of 0.36 g of (3S)-1-{[6-(4-fluorophenoxy)pyridin-3-yl]methyl}-5-oxopyrrolidin-3-yl methanesulfonate in 3 mL of DMF, a solution of 0.38 g (5 mmol) of thioacetic acid and 0.20 g (0.60 mmol) of cesium carbonate in 2 mL of DMF was added dropwise. After completion of the dropwise addition, the reaction mixture was stirred for 4 days. A solution of 0.38 g (5 mmol) of thioacetic acid and 0.20 g (0.60 mmol) of cesium carbonate in 2 mL of DMF was added dropwise again thereto, and after completion of the dropwise addition, the reaction mixture was stirred for one day, to which was added water, and the mixture was extracted with ethyl acetate-n-hexane (10:1). The organic layer was washed with saturated brine and dried over anhydrous magnesium sulfate, and then the solvent was distilled off under reduced pressure to give a residue which was then purified by silica gel column chromatography (n-hexane: ethyl acetate = 1:5) and crystallized from n-hexane-ethyl acetate to yield 0.14 g (45%) of the title compound.

Melting Point 107 - 108°C.

[Process 2]

Step 1

In the same manner as in Step 3 of Example 38, 164 mg (54%) of (4S)-1-{[6-(4-fluorophenoxy)pyridin-3-yl] methyl}-4-hydroxypyrrolidin-2-one was obtained from 1-[(6-fluorophenoxy)pyridin-3-yl]methanamine (218 mg)obtained in the same manner as in Step 1 and Step 2 of Example 38 and ethyl (S)-4-chloro-3-hydroxybutanoate (content 90%, 200 mg). Step 2

In the same manner as in Step 4 of Example 38, the title compound (15.2 g) was obtained in a crude form from (4S)-1-{[6-(4-fluorophenoxy)pyridin-3-yl]methyl}-4-hydroxypyrrolidin-2-one (15.0 g). The title compound in a crude form (7.6 g) was recrystallized from toluene/n-heptane (36.5 mL/61 mL) to yield 6.8 g (76%) of the title compound.

Example 2

(4R)-1-{[6-(4-fluorophenoxy)pyridin-3-yl]methyl}-4-mercaptopyrrolidin-2-one hydrochloride

To a solution obtained by adding dropwise 5.6 mL (79.4 mmol) of acetyl chloride to 20 mL of ethanol under icecooling, 1.43 g (3.97 mmol) of S-((3R)-1-{[6-(4-fluorophenoxy)pyridin-3-yl]methyl)-5-oxopyrrolidin-3-yl)ethanethioate obtained in Example 1 was added in small portions. The reaction mixture was stirred at 50°C for 2 hours, and the solvent was distilled off under reduced pressure to yield 1.56 g (quantitative yield) of the title compound

Melting Point 92 - 93°C.

Example 3

S-((3R)-1-{[6-(4-fluorophenoxy)pyridin-3-yl]methyl}-5-oxopyrrolidin-3-yl)methylthiocarbamate

[Process 1]

To a solution of 6.74 g (17.1 mmol) of (4R)-1-{[6-(4-fluorophenoxy)pyridin-3-yl]methyl}-4-mercaptopyrrolidin-2-one hydrochloride obtained Example 2 and 1.07 g (18.8 mmol) of methyl isocyanate in 40 mL of dichloromethane, a solution of 2.16 g (35.9 mmol) of acetic acid and 3.63 g (35.9 mmol) of triethylamine in 20 mL of dichloromethane was added dropwise. After completion of the dropwise addition, the reaction mixture was stirred at room temperature for 1 hour, and the solvent was distilled off under reduced pressure to give a residue, to which was added ethyl acetate. The mixture was washed twice and dried over magnesium sulfate, and the solvent was distilled off under reduced pressure to give a residue. The residue thus obtained was crystallized from n-hexane-ethyl acetate to yield 4.84 g (76%) of the title compound.

Melting Point 167 - 168°C.

[Process 2]

In the same manner as in Example 39, 9.7 g (93%) of the title compound was obtained from S-((3R)-1-{[6-(4-fluorophenoxy)pyridin-3-yl]methyl}-5-oxopyrrolidin-3-yl)ethanethioate (10.0 g) obtained in Example 1.

Example 4

S-((3R)-1-{[6-(4-fluorophenoxy)pyridin-3-yl]methyl}-5-oxopyrrolidin-3-yl)ethylthiocarbamate

In the same manner as in Example 3, 309 mg (59%) of the title compound was obtained from 480 mg (1.35 mmol) of (4R)-1-( [6-(4-fluorophenoxy)pyridin-3-yl]methyl)-4-mercaptopyrrolidin-2-one hydrochloride obtained in Example 2 and 0.12 mL (1.52 mmol) of ethyl isocyanate.
Melting Point 157°C.

Example 5

S-((3R)-1-{[6-(4-fluorophenoxy)pyridin-3-yl]methyl}-5-oxopyrrolidin-3-yl)propylthiocarbamate

In the same manner as in Example 3, 295 mg (55%) of the title compound was obtained from 478 mg (1.35 mmol) of (4R)-1-{[6-(4-fluorophenoxy)pyridin-3-yl]methyl}-4-mercaptopyrrolidin-2-one hydrochloride obtained in Example 2 and 0.14 mL (1.49 mmol) of propyl isocyanate.
Melting Point 169°C.

Example 6

S-((3R)-1-{[6-(4-fluorophenoxy)pyridin-3-yl]methyl}-5-oxopyrrolidin-3-yl)butylthiocarbamate

In the same manner as in Example 3, 347 mg (54%) of the title compound was obtained from 546 mg (1.54 mmol) of (4R)-1-{(6-(4-fluorophenoxy)pyridin-3-yl]methyl}-4-mercaptopyrrolidin-2-one hydrochloride obtained in Example 2 and 0.19 mL (1.69 mmol) of butyl isocyanate.
Melting Point 163°C.

Example 7

S-((3R)-1-{[6-(4-fluorophenoxy)pyridin-3-yl]methyl}-5-oxopyrrolidin-3-yl)isopropylthiocarbamate

In the same manner as in Example 3, 341 mg (52%) of the title compound was obtained from 577 mg (1.63 mmol) of (4R)-1-{[6-(4-fluorophenoxy)pyridin-3-yl]methyl}-4-mercaptopyrrolidin-2-one hydrochloride obtained in Example 2 and 0.18 mL (1.79 mmol) of isopropyl isocyanate.
Melting Point 181 - 182°C.

Example 8

Ethyl N-{[((3R)-1-{[6-(4-fluorophenoxy)pyridin-3-yl]methyl}-5-oxopyrrolidin-3-yl)thio]carbonyl}glycinate

In the same manner as in Example 3, 383 mg (63%) of the title compound was obtained from 483 mg (1.36 mmol) of (4R)-1-{[6-(4-fluorophenoxy)pyridin-3-yl]methyl}-4-mercaptopyrrolidin-2-one hydrochloride obtained in Example 2 and 0.17 mL (1.50 mmol) of ethyl isocyanatoacetate.
Melting Point 133 - 134°C.

Example 9

(3R)-1-{[6-(4-fluorophenoxy)pyridin-3-yl]methyl}-5-oxopyrrolidin-3-ylmethyl dithiocarbamate

In the same manner as in Example 3, 28.1 mg (10%) of the title compound was obtained from 250 mg (0.705 mmol) of (4R)-1-{[6-(4-fluorophenoxy)pyridin-3-yl]methyl}-4-mercaptopyrrolidin-2-one hydrochloride obtained in Example 2 and 56.7 mg (0.776 mmol) of methyl isothiocyanate.
Melting Point 160 - 161°C.

Example 10

(4R,4'R)-4,4'-dithiobis[1-{[6-(4-fluorophenoxy) pyridin-3-yl]methyl}pyrrolidin-2-one]

In the same manner as in Example 3, 6.08 g (9%) of the title compound was obtained from 76.4 g (211.1 mmol) of (4R)-1-{[6-(4-fluorophenoxy)pyridin-3-yl]methyl}-4-mercaptopyrrolidin-2-one hydrochloride which was purified by silica gel column chromatography (acetone) and then crystallized from ethyl acetate.

Melting Point 190 - 191°C.

Example 11

S-((3R)-1-{[6-(4-ethylphenoxy)pyridin-3-yl]methyl}-5-oxopyrrolidin-3-yl)ethanethioate

[Process 1]

Step 1

27.7 g (200 mmol) of 6-chloronicotinonitrile, 24.4 g (200 mmol) of 4-ethylphenol and 29.0 g (210 mmol) of potassium carbonate in DMF were stirred at 100°C for 4 hours, and the solvent was distilled off under reduced pressure. To the residue thus obtained was added water, and the mixture was extracted from ethyl acetate. The organic layer was washed with saturated brine and dried over anhydrous magnesium sulfate, and the solvent was distilled off under reduced pressure to give a residue. The residue thus obtained was purified by silica gel column chromatography (n-hexane:ethyl acetate = 10:1) to yield 48.93 g (quantitative yield) of 6-(4-ethylphenoxy)nicotinonitrile.

$^1$H-NMR ($CDCl_3$) δ: 1. 27 (3 H, t, J=7.5 Hz) , 2.69 (2 H, q, J=7. 5 Hz) , 6.97-7.08(3 H, m), 7.27 (2 H, d, J=8.8 Hz), 7.90 (1 H, dd, J=2.4, 8.6 Hz), 8.47(1 H, d, J=2.2 Hz).

Step 2

48.93 g (200 mmol) of 6-(4-ethylphenoxy)nicotinonitrile, 60 mL of 28% aqueous ammonia and 20 g of Raney nickel were stirred in a mixed solvent of 350 mL of THF-700 mL of ethanol under hydrogen atmosphere for 2 days. The insolubles were filtered off, and the filtrate was concentrated under reduced pressure to give a residue which was then dissolved in ethyl acetate and dried over anhydrous magnesium sulfate. The solvent was again distilled off under reduced pressure to give [6-(4-ethylphenoxy)pyridin-3-yl]methylamine which was dissolved in 80 mL of THF. This solution was added dropwise to a solution of (S)-O-acetylmalic anhydride in 240 mL of acetonitrile at 0°C. After completion of the dropwise addition, the reaction mixture was stirred at room temperature for 1.5 hours, and the solvent was then distilled off under reduced pressure. Thus obtained residue was dissolved in 200 mL of acetyl chloride and stirred at 60°C for 0.5 hour, and the solvent was distilled off under reduced pressure. Thus obtained residue was dissolved in ethyl acetate, washed with saturated aqueous sodium hydrogen carbonate and saturated brine, and dried over anhydrous magnesium sulfate. The solvent was distilled off under reduced pressure to give a residue which was then purified by silica gel column chromatography (n-hexane:ethyl acetate = 1:1) to yield 58.3 g (79%) of (3S)-1-{[6-(4-ethylphenoxy) pyridin-3-yl]methyl}-2,5-dioxopyrrolidin-3-yl acetate.

| Elemental analysis (%) : $C_{20}H_{20}N_2O_5$ | | | |
|---|---|---|---|
| Calcd. | C, 65.21 ; | H, 5.47 ; | N, 7.60 |
| Found | C, 64.84 ; | H, 5. 55 ; | N 7.48. |

Step 3

In the same manner as in Step 4 of Example 1, 9.9 g (20%) of (4S)-1-{[6-(4-ethylphenoxy)pyridin-3-yl]methyl}-4-hydroxypyrrolidin-2-one was obtained from 58.2 g (158 mmol) of (3S)-1-{[6-(4-ethylphenoxy)pyridin-3-yl]methyl}-2,5-dioxopyrrolidin-3-yl acetate.

$^1$H-NMR ($CDCl_3$) δ: 1.25 (3 H, t, J=7.7 Hz) , 2.17 (1 H, brs) , 2.42(1 H, dd, J=2.4, 17.2 Hz), 2.66(2 H, q, J=7.8 Hz), 2.73 (1 H, dd, J=6.6, 17.6 Hz), 3.19(1 H, dd, J=2.2, 10.6 Hz), 3.52(1 H, dd, J=5.5, 10.7 Hz), 4.42(2 H, dd, J=14.8, 22.6 Hz), 4.50(1 H, brs), 6.86(1 H, d, J=8.4 Hz), 7.03(2 H, d, J=8.4 Hz), 7.23(2 H, d, J=8.0 Hz), 7.60(1 H, dd, J=2.6, 8.4 Hz), 8.05 (1 H, d, J=2.4 Hz).

Step 4

In the same manner as in Steps 5 and 6 of Example 1, 5.3 g of (3S)-1-{[6-(4-ethylphenoxy)pyridin-3-yl]methyl)-5-oxopyrrolidin-3-yl methanesulfonate was obtained from 9.9 g (31.6 mmol) of (4S)-1-{[6-(4-ethylphenoxy)pyridin-3-yl]methyl)-4-hydroxypyrrolidin-2-one. Using this, 10.2 g (87%) of the title compound was obtained.

| Elemental analysis(%) : $C_{20}H_{22}N_2O_3S \cdot 0.5H_2O$ | | | |
|---|---|---|---|
| Calcd. | C, 63.30 ; | H, 6.10; | N, 7.38 |
| Found | C, 63.35 ; | H, 6.12 ; | N, 7.16. |

[Process 2]

Step 1

In the same manner as in Step 3 of Example 38, 7.9 g (58%) of (4S)-1-{[6-(4-ethylphenoxy)pyridin-3-yl]methyl}-4-hydroxypyrrolidin-2-one was obtained from 1-([6-ethylphenoxy)pyridin-3-yl]methaneamine (10.0 g) which was obtained in the same manner as in Steps 1 and 2 of Example 38 and ethyl(S)-4-cloro-3-hydroxybutanoate (content 90%, 11.7 g).

Step 2

In the same manner as in Step 4 of Example 38, 4.4 g (62%) of the title compound was obtained from (4S)-1-{[6-(4-ethylphenoxy)pyridin-3-yl]methyl}-4-hydroxypyrrolidin-2-one (6.0 g).

Example 12

(4R)-1-{[6-(4-ethylphenoxy)pyridin-3-yl]methyl}-4-mercaptopyrrolidin-2-one

In the same manner as in Example 2, 11.1 g (quantitative yield) of the title compound was obtained from 10.0 g (27.0 mmol) of S-((3R)-1-{[6-(4-ethylphenoxy)pyridin-3-yl]methyl)-5-oxopyrrolidin-3-yl)ethanethioate obtained in Example 11.

Melting Point 85 - 88°C.

Example 13

S-((3R)-1-{[6-(4-ethylphenoxy)pyridin-3-yl]methyl}-5-oxopyrrolidin-3-yl)methylthiocarbamate

[Process 1]

In the same manner as in Example 3, 5.34 g (51%) of the title compound was obtained from 11.1 g (27 mmol) of (4R)-1-{[6-(4-ethylphenoxy)pyridin-3-yl]methyl}-4-mercaptopyrrolidin-2-one obtained in Example 12.

Melting Point 103 - 104°C.

[Process 2]

In the same manner as in Example 39, 0.7 g (69%) of the title compound was obtained from S-((3R)-1-([6-(4-ethylphenoxy)pyridin-3-yl]methyl)-5-oxopyrrolidin-3-yl)ethanethioate (1.0 g) obtained in Example 11.

Example 14

S-{(3R)-1-[(6-{4-[(methylsulfonyl)amino]phenoxy}pyridin-3-yl)methyl]-5-oxopyrrolidin-3-yl)ethanethioate

Step 1

In the same manner as in Step 3 of Example 1, 13.2 g (57%) of (3S)-1-[ (6-{4-[(methylsulfonyl)amino]phenoxy}pyridin-3-yl)methyl]-2,5-dioxopyrrolidin-3-yl acetate was obtained from 16.8 g (53 mmol) of N-(4-{[5-(aminomethyl)pyridin-2-yl]oxy}phenyl)methanesulfonamide.

Melting Point 148 - 149°C.

Step 2

In the same manner as in Step 4 of Example 1, 2.19 g (57%) of (3S)-1-[(6-{4-[(methylsulfonyl)amino]phenoxy} pyridin-3-yl)methyl]-5-oxopyrrolidin-3-yl acetate was obtained from 4.0 g (9.23 mmol) of (3S)-1-[(6-{4-[(methylsulfonyl) amino]phenoxy}pyridin-3-yl)methyl]-2,5-dioxopyrrolidin-3-yl acetate.
Melting Point 127 - 128°C.

Step 3

In the same manner as in Step 4 of Example 1, 1.95 g (quantitative yield) of N-{4-[(5-{[(4S)-4-hydroxy-2-oxopyrrolidin-1-yl]methyl}pyridin-2-yl)oxy]phenyl)methanesulfonamide was obtained from 2.0 g (4.77 mmol) of (3S)-1-[(6-{4-[(methylsulfonyl)amino]phenoxy}pyridin-3-yl)methyl]-5-oxopyrrolidin-3-yl acetate.

| Elemental analysis (%) : $C_{17}H_{19}N_3O5S \cdot 1.9H_2O$ | | | |
|---|---|---|---|
| Calcd. | C, 49.60 ; | H, 5.58; | N, 10.21 |
| Found | C, 49.75; | H, 5.29 ; | N, 9.84. |

Step 4

In the same manner as in Step 5 of Example 1, 1.67 g (77%) of (3S)-1-[(6-{4-[(methylsulfonyl)amino]phenoxy} pyridin-3-yl)methyl]-5-oxopyrrolidin-3-yl methanesulfonate was obtained from 1.85 g (4.90 mmol) of N-{4-[(5-{[(4S)-4-hydroxy-2-oxopyrrolidin-1-yl]methyl}pyridin-2-yl)oxy]phenyl}methanesulfonamide.
Melting Point 155 - 157°C.

Step 5

In the same manner as in Step 6 of Example 1, 1.01 g (63%) of the title compound was obtained from 1.55 g (3.66 mmol) of (3S)-1-[(6-{4-[(methylsulfonyl)amino]phenoxy}pyridin-3-yl)methyl]-5-oxopyrrolidin-3-yl methanesulfonate.
Melting Point 135 - 136°C.

Example 15

N-{4-[(5-[(4R)-4-mercapto-2-oxopyrrolidin-1-yl]methyl)pyridin-2-yl]oxy}phenyl}methanesulfonamide

In the same manner as in Example 2, 770 mg (94%) of the title compound was obtained from 910 mg (2.09 mmol) of S-{(3R)-1-[(6-(4-[(methylsulfonyl)amino]phenoxy}pyridin-3-yl)methyl]-5-oxopyrrolidin-3-yl}ethanethioate obtained in Example 14.
$^1$H-NMR ($CDCl_3$) δ: 1.89(1 H, d, J=6.6 Hz), 2.42(1 H, dd, J=5.8, 17.2 Hz), 2.93(1 H, dd, J=8.0, 17.2 Hz), 3.03(3 H,S), 3.17(1 H, dd, J=4.8, 10.0 Hz), 3.58(1 H, m), 3.68(1 H, dd, J=7.0, 10.0 Hz), 4.37 (1 H, d, J=15.0 Hz), 4.50(1 H, d, J=15.0 Hz), 6.93 (1 H, d, J=8.4 Hz), 6.97(1 H,S), 7.13(2 H, d, J=9.0 Hz), 7.28(2 H, d, J=9.0 Hz), 7.66(1 H, dd, J=2.6, 8.4 Hz), 8.05(1 H, d, J=2.6 Hz).

Example 16

S-{(3R)-1-[(6-{4-[(methylsulfonyl)amino]phenoxy}pyridin-3-yl)methyl]-5-oxopyrrolidin-3-yl}methylthiocarbamate

In the same manner as in Example 3, 396 mg (49%) of the title compound was obtained from 710 mg (1.80 mmol) of N-{4-[(5-([(4R)-4-mercapto-2-oxopyrrolidin-1-yl]methyl}pyridin-2-yl)oxy]phenyl}methanesulfonamide obtained in Example 15 and 118 μL (1.98 mmol) of methyl isocyanate.
Melting Point 172 - 173°C.

Example 17

(4R)-1-{[6-(4-fluorophenoxy)pyridin-3-yl]methyl}-4-mercaptopyrrolidin-2-one dihydrochloride

To a solution obtained by adding dropwise 217 mL (3052 mmol) of acetyl chloride to 500 mL of ethanol under icecooling, 55.0 g (153 mmol) of S-((3R)-1-{[6-(4-fluorophenoxy)pyridin-3-yl]methyl}-5-oxopyrrolidin-3-yl)ethanethioate obtained in Example 1 was added in small portions. The reaction mixture was stirred at 50°C for 2 hours, and then the solvent was distilled off to yield 59.4 g (97%) of the title compound.

| Elemental analysis (%) : $C_{16}H_{15}N_2O_2SF \cdot 2HCl \cdot 0.5H_2O$ | | | |
|---|---|---|---|
| Calcd. | C, 48.00 ; | H, 4. 53 ; | N, 6.99 |
| Found | C, 47. 88 ; | H, 4.68 ; | N, 6.86. |

Example 18

(4R)-1-((6-(4-fluorophenoxy)-3-pyridinyl)methyl)-4-(methylthio)-2-pyrrolidinone

To a solution of 250 mg (0.63 mmol) of (4R)-1-{[6-(4-fluorophenoxy)pyridin-3-yl]methyl}-4-mercaptopyrrolidin-2-one dihydrochloride obtained in Example 17 and 0.043 mL (0.69 mmol) of iodomethane in 2 mL of THF, 180 mg (4.50 mmol) of sodium hydride (60% in oil) which had been previously washed with n-hexane was added, and the reaction mixture was stirred at room temperature for 30 minutes. Then, the reaction was stopped by addition of water, and the solvent was distilled off under reduced pressure. To the residue thus obtained was added ethyl acetate, and the mixture was washed with water and then saturated brine and dried over anhydrous sodium sulfate. The solvent was distilled off under reduced pressure, and the residue obtained was purified by preparative TLC (n-hexane:ethyl acetate = 1:2) and crystallized from ethyl acetate to yield 143 mg (69%) of the title compound.

Melting Point 94 - 95°C.

Example 19

(2R)-2-((tert-butoxycarbonyl)amino)-3-(((3R)-1-((6-(4-fluorophenoxy)-3-pyridinyl)methyl)-5-oxo-3-pyrrolidinyl) thio) propanoic acid

To a mixture of 1.50 g (3.75 mmol) of (4R)-1-{[6-(4-fluorophenoxy)pyridin-3-yl]methyl}-4-mercaptopyrrolidin-2-one dihydrochloride obtained in Example 17, 772 mg (4.12 mmol) of tert-butyl[(3S)-2-oxooxetan-3-yl]carbamate and 15 mL of DMF, 5.74 g (17.6 mmol) of cesium carbonate was added, the reaction mixture was stirred at room temperature for 30 minutes, and then the solvent was distilled off under reduced pressure. To the residue thus obtained was added ethyl acetate, and the mixture washed with water and then saturated brine. The organic layer was dried over anhydrous sodium sulfate. The solvent was distilled off under reduced pressure, and the residue obtained was purified by preparative TLC (chloroform:methanol = 6:1) to yield 1.18 g (62%) of the title compound.

| Elemental analysis (%) : $C_{24}H_{28}N_3O_6SF \cdot H_2O$ | | | |
|---|---|---|---|
| Calcd. | C, 55.06; | H, 5.78; | N, 8.03 |
| Found | C, 54.98 ; | H, 5.42 ; | N, 7.96. |

Example 20

(2R)-2-(acetylamino)-3-(((3R)-1-((6-(4-fluorophenoxy)-3-pyridinyl)methyl)-5-oxo-3-pyrrolidinyl) thio)propanoic acid

To a solution of 200 mg (0.50 mmol) of (2R)-2-amino-3-(((3R)-1-((6-(4-fluorophenoxy)-3-pyridinyl)methyl)-5-oxo-3-pyrrolidinyl)thio)propanoic acid dihydrochloride obtained in Example 21 in 3 mL of pyridine, 0.47 mL (0.50 mmol) of anhydrous acetic acid was added, the reaction mixture was stirred at room temperature for 30 minutes, and then the solvent was distilled off under reduced pressure. To the residue thus obtained was added ethyl acetate, the mixture was washed with 0.1 N hydrochloric acid, and then the organic layer was dried over anhydrous sodium sulfate. The solvent was distilled off under reduced pressure, and the residue obtained was purified by preparative TLC (chloroform:methanol = 3:1) to yield 99 mg (49%) of the title compound.

| Elemental analysis (%) : $C_{21}H_{22}N_3O_5SF \cdot 1.25\ H_2O$ | | | |
|---|---|---|---|
| Calcd. | C, 53.66 ; | H, 5.25; | N, 8.94 |
| Found | C, 53.55 ; | H, 4. 89 ; | N, 8.87. |

Example 21

(2R)-2-amino-3-(((3R)-1-((6-(4-fluorophenoxy)-3-pyridinyl)methyl)-5-oxo-3-pyrrolidinyl)thio)propanoic acid dihydrochloride

400 mg (0.79 mmol) of (2R)-2-((tert-butoxycarbonyl) amino)-3-(((3R)-1-((6-(4-fluorophenoxy)-3-pyridinyl)methyl)-5-oxo-3-pyrrolidinyl)thio)propanoic acid obtained in Example 19 was dissolved in a 4 N solution of hydrogen chloride in ethyl acetate, the reaction mixture was stirred at room temperature for 30 minutes, and then the solvent was distilled off to yield 342 mg (98%) of the title compound.

| Elemental analysis (%) : $C_{19}H_{20}N_3O_4SF \cdot 2HCl \cdot 2.2\ H_2O$ | | | |
|---|---|---|---|
| Calcd. | C, 44.05; | H, 5. 14 ; | N, 8.11 |
| Found | C, 44.35 ; | H, 4.99 ; | N, 8.30. |

Example 22

(((3R)-1-((6-(4-fluorophenoxy)-3-pyridinyl)methyl)-5-oxo-3-pyrrolidinyl)thio)methyl acetate

Step 1

To a mixture of 300 mg (0.75 mmol) of (4R)-1-{[6-(4-fluorophenoxy)pyridin-3-yl]methyl}-4-mercaptopyrrolidin-2-one dihydrochloride obtained in Example 17 and 1 mL of bromochloromethane, 286 mg (5.09 mmol) of pulverized potassium hydroxide and 17 mg (0.08 mmol) of benzyltriethylammonium chloride were added, the reaction mixture was stirred at room temperature for 15 minutes, and then the solvent was distilled off under reduced pressure. To the residue thus obtained was added ethyl acetate, the mixture was washed with water and then saturated brine, and the organic layer was dried over anhydrous sodium sulfate. The solvent was distilled off under reduced pressure, and the residue obtained was crystallized from ethyl acetate to yield 92 mg (34%) of (4R)-4-[(chloromethyl)thio]-1-{[6-(4-fluorophenoxy)pyridin-3-yl]methyl}pyrrolidin-2-one.

Step 2

To a solution of 219 mg (0.60 mmol) of (4R)-4-[(chloromethyl)thio]-1-{[6-(4-fluorophenoxy)pyridin-3-yl]methyl} pyrrolidin-2-one in 1 mL of DMF, 117 mg (1.19 mmol) of potassium acetate was added, the reaction mixture was stirred at room temperature for 30 hours, and then the solvent was distilled off under reduced pressure. To the residue thus obtained was added ethyl acetate, and the mixture was washed with water and then saturated brine, and dried over anhydrous sodium sulfate. The solvent was distilled off under reduced pressure, and the residue obtained was purified by preparative TLC (n-hexane:ethyl acetate = 1:2) to yield 151 mg (65%) of the title compound.

| Elemental analysis (%) : $C_{19}H_{19}N_2O_4SF$ | | | |
|---|---|---|---|
| Calcd. | C, 58.45 ; | H, 4.91; | N, 7.18 |
| Found | C, 58.27 ; | H, 4.75 ; | N, 7.08. |

Example 23

N-((((3R)-1-((6-(4-fluorophenoxy)-3-pyridinyl)methyl)-5-oxo-3-pyrrolidinyl)thio)methyl)acetamide

To a solution of 200 mg (0.50 mmol) of (4R)-1-{[6-(4-fluorophenoxy)pyridin-3-yl)methyl}-4-mercaptopyrrolidin-2-one dihydrochloride obtained in Example 17 in 1 mL of trifluoroacetic acid, 49 mg (0.55 mmol) of acetamidemethanol was added, the reaction mixture was stirred at room temperature for 10 minutes, and then the solvent was distilled off under reduced pressure. To the residue thus obtained was added ethyl acetate, and the mixture was washed with

saturated aqueous sodium hydrogen carbonate and then saturated brine, and dried over anhydrous sodium sulfate. The solvent was distilled off under reduced pressure, and the residue obtained was purified by preparative TLC (chloroform:methanol = 10:1) to yield 154 mg (79%) of the title compound.

Melting Point 94 - 95°C.

Example 24

3-(((3R)-1-((6-(4-fluorophenoxy)-3-pyridinyl)methyl)-5-oxo-3-pyrrolidinyl)thio)-1-methyl-2,5-pyrrolidin-2-one

To a solution of 200 mg (0.50 mmol) of (4R)-1-{[6-(4-fluorophenoxy)pyridin-3-yl]methyl}-4-mercaptopyrrolidin-2-one dihydrochloride obtained in Example 17 and 61 mg (0.55 mmol) of N-methyl maleimide in 2 mL of chloroform, 0.18 mL (1.3 mmol) of triethylamine was added, the reaction mixture was stirred at room temperature for 10 minutes, and then the solvent was distilled off under reduced pressure. To the residue thus obtained was added ethyl acetate, and the mixture was washed with saturated aqueous sodium hydrogen carbonate and then saturated brine, and dried over anhydrous sodium sulfate. The solvent was distilled off under reduced pressure, and the residue obtained was purified by preparative TLC (n-hexane:ethyl acetate = 1:3) to yield 199 mg (93%) of the title compound.

| Elemental analysis (%) : $C_{21}H_{20}N_3O_4SF \cdot 0.5H_2O$ | | | |
|---|---|---|---|
| Calcd. | C, 57.52; | H, 4.83 ; | N, 9.58 |
| Found | C, 57.24 ; | H, 4.69 ; | N, 9.38. |

Example 25

1-ethyl-3-(((3R)-1-((6-(4-fluorophenoxy)-3-pyridinyl)methyl)-5-oxo-3-pyrrolidinyl)thio)-2,5-pyrrolidindione

In the same manner as in Example 24, 204 mg (92%) of the title compound was obtained from 200 mg (0.50 mmol) of (4R)-1-{[6-(4-fluorophenoxy)pyridin-3-yl]methyl}-4-mercaptopyrrolidin-2-one dihydrochloride obtained in Example 17 and 69 mg (0.55 mmol) of N-ethyl maleimide.

| Elemental analysis (%) : $C_{22}H_{22}N_3O_4SF \cdot 0.5H_2O$ | | | |
|---|---|---|---|
| Calcd. | C, 58.39 ; | H, 5.12; | N, 9.29 |
| Found | C, 58.53 ; | H, 5.21; | N, 9.28. |

Example 26

2-((((3R)-1-((6-(4-fluorophenoxy)-3-pyridinyl)methyl)-5-oxo-3-pyrrolidinyl)thio)carbonyl)phenyl acetate

To a solution of 200 mg (0.50 mmol) of (4R)-1-{[6-(4-fluorophenoxy)pyridin-3-yl}methyl}-4-mercaptopyrrolidin-2-one dihydrochloride obtained in Example 17 and 109 mg (0.55 mmol) of O-acetylsalicyloyl chloride in 2 mL of chloroform, 0.25 mL (1.8 mmol) of triethylamine was added, the reaction mixture was stirred at room temperature for 30 minutes, and then the solvent was distilled off under reduced pressure. To the residue thus obtained was added ethyl acetate, and the mixture was washed with saturated aqueous sodium hydrogen carbonate and then saturated brine, and dried over anhydrous sodium sulfate. The solvent was distilled off under reduced pressure, and the residue obtained was purified by preparative TLC (n-hexane:ethyl acetate = 1:1) to yield 222 mg (92%) of the title compound.

| Elemental analysis (%) : $C_{25}H_{21}N_2O_5SF \cdot 0.2 H_2O$ | | | |
|---|---|---|---|
| Calcd. | C, 62.02 ; | H, 4.46 ; | N, 5.79 |
| Found | C, 61.92 ; | H, 4.49 ; | N, 5. 76. |

Example 27

5-(((3R)-1-((6-(4-fluorophenoxy)-3-pyridinyl)methyl)-5-oxo-3-pyrrolidinyl)thio)-5-oxopentanoic acid

To a solution of 220 mg (0.55 mmol) of (4R)-1-{[6-(4-fluorophenoxy)pyridin-3-yl]methyl}-4-mercaptopyrrolidin-2-one dihydrochloride obtained in Example 17 and 75 mg (0.66 mmol) of glutaric anhydride in 2 mL of THF, 101 mg (2.53 mmol) of sodium hydride (60% in oil) which had been previously washed with n-hexane was added, and the

reaction mixture was stirred at room temperature for 12 hours. The reaction mixture was added dropwise to 50 mL of 0.1 N hydrochloric acid solution and extracted by adding ethyl acetate, and the organic layer was dried over anhydrous sodium sulfate. The solvent was distilled off under reduced pressure, and the residue obtained was purified by preparative TLC (chloroform:methanol = 15:1) to yield 85 mg (34%) of the title compound.
Melting Point 132 - 133°C.

Example 28

S-((3R)-1-((6-(4-fluorophenoxy)-3-pyridinyl)methyl)-5-oxo-3-pyrrolidinyl)(acetylamino)ethanethioate

To a solution of 500 mg (0.788 mmol) of (4R, 4'R)-4, 4'-dithiobis[1-{[6-(4-fluorophenoxy)pyridin-3-yl]methyl} pyrrolidin-2-one] obtained in Example 10 and 222 mg (1.89 mmol) of N-acetylglycine in 5 mL of pyridine, 0.29 mL (1.18 mmol) of tributylphosphine was added, the reaction mixture was stirred at room temperature for 1.5 hours, and then the solvent was distilled off under reduced pressure. To the residue thus obtained was added ethyl acetate, and the mixture washed with 0.1 hydrochloric acid and then saturated brine, and dried over anhydrous sodium sulfate. The solvent was distilled off under reduced pressure, and the residue obtained was purified by preparative TLC (chloroform: methanol = 10:1) to yield 217 mg (33%) of the title compound.
Melting Point 157 - 158°C.

Example 29

4-(((((3R)-1-((6-(4-fluorophenoxy)-3-pyridinyl)methyl)-5-oxo-3-pyrrolidinyl)thio)carbonyl)amino)ethylbutanoic acid

To a solution of 500 mg (1.25 mmol) of (4R)-1-{[6-(4-fluorophenoxy)pyridin-3-yl]methyl}-4-mercaptopyrrolidin-2-one dihydrochloride obtained in Example 17 and 0.24 mL (1.63 mmol) of ethyl 4-isocyanatobutyric acid in 3 mL of chloroform, a solution of 0.24 mL (4.13 mmol) of acetic acid and 0.57 mL (4.13 mmol) of triethylamine in 2 mL of chloroform was added dropwise. After completion of the dropwise addition, the reaction mixture was stirred at room temperature for 10 minutes, and then the solvent was distilled off under reduced pressure. To the residue thus obtained was added ethyl acetate, and the mixture washed with 0.1 hydrochloric acid and then saturated brine, and dried over anhydrous sodium sulfate. The solvent was distilled off under reduced pressure, and the residue obtained was crystallized from n-hexane-ethyl acetate to yield 531 mg (89%) of the title compound.
Melting Point 129 - 130°C.

Example 30

4-(((((3R)-1-((6-(4-fluorophenoxy)-3-pyridinyl)methyl)-5-oxo-3-pyrrolidinyl)thio)carbonyl)amino)butanoic acid

A mixture of 225 mg (0.47 mmol) of 4-(((((3R)-1-((6-(4-fluorophenoxy)-3-pyridinyl)methyl)-5-oxo-3-pyrrolidinyl)thio)carbonyl)amino)ethylbutanoic acid obtained in Example 29, 3 mL of acetic acid and 3 mL of concentrated hydrochloric acid was stirred at 100°C for 25 minutes, the solvent was distilled off under reduced pressure. To the residue thus obtained was added ethyl acetate, and the mixture was washed with water, and dried over anhydrous sodium sulfate. The solvent was distilled off under reduced pressure, and the residue obtained was crystallized from ethyl acetate to yield 139 mg (66%) of the title compound.
Melting Point 131 - 132°C.

Example 31

(3R)-1-((6-(4-fluorophenoxy)-3-pyridinyl)methyl)-5-oxopyrrolidin-3-yl allyldithiocarbamate

To a solution of 200 mg (0.51 mmol) of (4R)-1-{[6-(4-fluorophenoxy)pyridin-3-yl]methyl}-4-mercaptopyrrolidin-2-one dihydrochloride obtained in Example 17 in 3 mL of pyridine, 0.64 mL (0.26 mmol) of tributylphosphine and 0.75 mL (0.77 mmol) of allylisothiocyanate were added, and the reaction mixture was stirred at room temperature for 12 hours. The solvent was distilled off under reduced pressure, and the residue obtained was purified by preparative TLC (n-hexane:ethyl acetate = 1:2) to yield 167 mg (78%) of the title compound.
Melting Pont 64 - 65°C.

Example 32

2-(4-fluorophenoxy)-5-(((4R)-4-((methoxycarbonyl)dithio)-2-oxo-1-pyrrolidinyl)methyl)pyridine

To a solution of 200 mg (0.51 mmol) of (4R)-1-{[6-(4-fluorophenoxy)pyridin-3-yl]methyl}-4-mercaptopyrrolidin-2-one dihydrochloride obtained in Example 17 in 2 mL of methanol, 0.50 mL (0.55 mmol) of methoxycarbonylsulfenyl chloride was added, and the reaction mixture was stirred at room temperature for 30 minutes. The solvent was distilled off under reduced pressure to yield 225 mg (quantitative yield) of the title compound.
$^1$H-NMR ($CDCl_3$) δ: 2.51(1 H, dd, J=18.0, 3.7 Hz), 2.87 (1 H, dd, J=17.9, 7.6 Hz), 3.36(1 H, m), 3.71(2 H, m), 3.91(3 H,S), 4.37 (1 H, d, J=15.0 Hz), 4.62 (1 H, d, J=15.1 Hz), 6.93(1 H, d, J=8.8 Hz), 7.16(2 H,S), 7.18 (2 H,S), 7.99(1 H, dd, J=8.8, 2.2 Hz), 8.31(1 H, d, J=2.2 Hz) .

Example 33

2-(((3R)-1-((6-(4-fluorophenoxy)-3-pyridinyl)methyl)-5-oxo-3-pyrrolidinyl)dithio)benzoate

In the same manner as in Example 32, to a solution of 200 mg (0.51 mmol) of (4R)-1-{[6-(4-fluorophenoxy) pyridin-3-yl]methyl}-4-mercaptopyrrolidin-2-one dihydrochloride obtained in Example 17 in 2 mL of methanol, 0.50 mL (0.55 mmol) of methoxycarbonylsulfenyl chloride was added, and then the reaction mixture was stirred at room temperature for 30 minutes to prepare a solution of 2-(4-fluorophenoxy)-5-(((4R)-4-((methoxycarbonyl)dithio)-2-oxo-1-pyrrolidinyl)methyl)pyridine. To this solution, 81 mg (0.53 mmol) of thiosalicylic acid and 0.29 mL (2.10 mmol) of triethylamine were added, and then the reaction mixture was stirred at room temperature for 10 minutes. The solvent was distilled off under reduced pressure, to the residue thus obtained was added ethyl acetate, and the mixture was washed with 0.1 N hydrochloric acid and dried over anhydrous sodium sulfate. The solvent was distilled off under reduced pressure to give a residue which was then purified by preparative TLC (chloroform:methanol = 9:1) to yield 209 mg (89%) of the title compound.

| Elemental analysis (%) : $C_{23}H_{19}N_2O_4S_2F \cdot 0.5H_2O$ | | | |
|---|---|---|---|
| Calcd. | C, 57.61; | H, 4.20; | N, 5.84 |
| Found | C, 57.73 ; | H, 4.00; | N, 5.79. |

Example 34

3-(((3R)-1-((6-(4-fluorophenoxy)-3-pyridinyl)methyl)-5-oxo-3-pyrrolidinyl)dithio)benzoic acid

In the same manner as in Example 32, to a solution of 200 mg (0.51 mmol) of (4R)-1-{[6-(4-fluorophenoxy) pyridin-3-yl]methyl}-4-mercaptopyrrolidin-2-one dihydrochloride obtained in Example 17 in 2 mL of methanol, 0.50 mL (0.55 mmol) of methoxycarbonylsulfenyl chloride was added, and then the reaction mixture was stirred at room temperature for 30 minutes to prepare a solution of 2-(4-fluorophenoxy)-5-(((4R)-4-((methoxycarbonyl)dithio)-2-oxo-1-pyrrolidinyl)methyl)pyridine. From this solution together with 81 mg (0.53 mmol) of 3-mercaptobenzoic acid and 0.29 mL (2.10 mmol) of triethylamine, 188 mg (80%) of the title compound was obtained.
Melting Point 119 - 120°C.

Example 35

4-(((3R)-1-((6-(4-fluorophenoxy)-3-pyridinyl)methyl)-5-oxo-3-pyrrolidinyl)dithio)benzoic acid

In the same manner as in Example 32, to a solution of 200 mg (0.51 mmol) of (4R)-1-{[6-(4-fluorophenoxy) pyridin-3-yl]methyl}-4-mercaptopyrrolidin-2-one dihydrochloride obtained in Example 17 in 2 mL of methanol, 0.50 mL (0.55 mmol) of methoxycarbonylsulfenyl chloride was added, and then the reaction mixture was stirred at room temperature for 30 minutes to prepare a solution of 2-(4-fluorophenoxy)-5-(((4R)-4-((methoxycarbonyl)dithio)-2-oxo-1-pyrrolidinyl)methyl)pyridine. From this solution together with 81 mg (0.53 mmol) of 4-mercaptobenzoic acid and 0.29 mL (2.10 mmol) of triethylamine, 45 mg (19%) of the title compound was obtained.
Melting Point 184 - 185°C.

Example 36

(2R)-2-(acetylamino)-3-(((3R)-1-((6-(4-fluorophenoxy)-3-pyridinyl)methyl)-5-oxo-3-pyrrolidinyl) dithio)propanoic acid

In the same manner as in Example 32, to a solution of 400 mg (1.00 mmol) of (4R)-1-{[6-(4-fluorophenoxy) pyridin-3-yl]methyl)-4-mercaptopyrrolidin-2-one dihydrochloride obtained in Example 17 in 4 mL of methanol, 0.1 mL (0.50 mmol) of methoxycarbonylsulfenyl chloride was added, and then the reaction mixture was stirred at room temperature for 30 minutes to prepare a solution of 2-(4-fluorophenoxy)-5-(((4R)-4-((methoxycarbonyl)dithio)-2-oxo-1-pyrrolidinyl)methyl)pyridine. From this solution together with 163 mg (1.00 mmol) of N-acetyl-L-cysteine and 0.43 mL (3.10 mmol) of triethylamine, 110 mg (23%) of the title compound was obtained.
$^{1}$H-NMR (DMSO-$d_6$) δ: 1.81(3 H,S), 2.26(1 H, dd, J=17.4, 3.4 Hz), 2.80(1 H, dd, J=17.4, 7.8 Hz), 2.94(1 H, dd, J=13.0, 7.4 Hz), 3.14(1 H, dd, J=12.8, 4.0 Hz), 3.24(1 H, dd, J=10.6, 2.5 Hz), 3.65(2H, m), 4.17 (1 H, m), 4.32(1 H, d, J=14.8 Hz), 4.39 (1 H, d, J=15.8 Hz), 7.01 (1 H, d, J=8.5 Hz), 7.19(4 H, m), 7.69 (1 H, dd, J=8.5, 2.4 Hz), 7.87(1 H, m), 8.01 (1 H, d, J=2.2 Hz).

Example 37

(2R)-2-amino-3-(((3R)-1-((6-(4-fluorophenoxy)-3-pyridinyl)methyl)-5-oxo-3-pyrrolidinyl)dithio)propanoic acid hydrochloride

Step 1

In the same manner as in Example 32, to a solution of 1.8 g (4.50 mmol) of (4R)-1-{[6-(4-fluorophenoxy) pyridin-3-yl]methyl}-4-mercaptopyrrolidin-2-one dihydrochloride obtained in Example 17 in 18 mL of methanol, 0.45 mL (5.00 mmol) of methoxycarbonylsulfenyl chloride was added, and then the reaction mixture was stirred at room temperature for 10 minutes to prepare a solution of 2-(4-fluorophenoxy)-5-(((4R)-4-((methoxycarbonyl)dithio)-2-oxo-1-pyrrolidinyl)methyl)pyridine. From this solution together with 995 mg (4.50 mmol) of N-Boc-L-cysteine and 1.94 mL (14.0 mmol) of triethylamine, 118 mg (5%) of (2R)-2-((tert-butoxycarbonyl)amino)-3-(((3R)-1-((6-(4-fluorophenoxy)-3-pyridinyl)methyl)-5-oxo-3-pyrrolidinyl)dithio)propanoate was obtained.

Step 2

117 mg (0.22 mmol) of (2R)-2-((tert-butoxycarbonyl) amino)-3-(((3R)-1-((6-(4-fluorophenoxy)-3-pyridinyl)methyl)-5-oxo-3-pyrrolidinyl)dithio)propanoic acid was stirred in a solution of 4 N hydrogen chloride in ethyl acetate at room temperature for 30 minutes. The solvent was distilled off under reduced pressure, and thus obtained residue was crystallized from ethyl acetate to yield 104 mg (quantitative yield) of the title compound.
$^{1}$H-NMR (DMSO-$d_6$) δ: 2.33 (1 H, dd, J=17.4, 3.9 Hz) , 2. 86 (1 H, dd, J=17.4, 8.0 Hz), 3.33(3H, m), 3.69(2 H, m), 3.84 (1 H, m), 4.17 (2 H, m), 4.36 (1 H, d, J=15.1 Hz), 4.42 (1 H, d, J=15.0 Hz), 7.03(1 H, d, J=8.5 Hz), 7.21(4 H, m), 7.72 (1 H, dd, J=8.4, 2.5 Hz), 8.03(1 H, d, J=2.1 Hz), 8,89(1 H, m).

Example 38

S-{(3R)-5-oxo-1-[(6-phenoxypyridin-3-yl)methyl]pyrrolidin-3-yl}ethanethioate

Step 1

Sodium hydroxide (4.4 g) was added to a mixture of 6-chloronicotinonitrile (13.9 g), phenol (12.3 g) and N, N-dimethylformamide (DMF) at room temperature, and the mixture was stirred at the same temperature for 2 hours. To the reaction mixture was added water and the mixture was stirred at room temperature for another 2 hours. The precipitated crystals were collected by filtration, washed with water and dried under reduced pressure until the weight became constant to yield 18.0 g (92%) of 6-phenoxynicotinonitrile.
$^{1}$H-NMR ($CDCl_3$) δ: 7.02(1 H, d, J=8.6 Hz), 7.13-7.17(2 H, m), 7.29-7.32(1 H, m), 7.43-7.48(1 H, m) 7.90-7.94(1 H, m), 8.11(1 H, d, J=2.2 Hz)

Step 2

Raney nickel (15 mL) was added to a mixture of 6-phenoxynicotinonitrile (15.0 g), 28% aqueous ammonia (44 mL), tetrahydrofuran (THF, 300 mL) and ethanol (150 mL), and the mixture was stirred at room temperature under

the condition of 0.3 MPa of hydrogen pressure for 4 hours. The insolubles were filtered off, and the solvent was completely distilled off under reduced pressure to yield 15.1 g (99%) of 1-(6-phenoxypyridin-3-yl)methanamine.
$^1$H-NMR ($CDCl_3$) δ: 1.44(2 H, brs) , 3.83(2 H,S), 6.87(1 H, d, J=8.4 Hz), 7.10-7.20(3 H, m), 7.36-7.41(1 H, m), 7.66-7.69 (1 H, m), 8.11(1 H, d, J=2.1 Hz).

Step 3

Potassium hydrogen carbonate (9.9 g) was added to a mixture of 1-(6-phenoxypyridin-3-yl)methaneamine (10.0 g), ethyl (S)-4-chloro-3-hydroxybutanoate (content 90%, 13.3 g) and potassium iodide (2.7 g) in 1-butanol (100 mL), and the mixture was stirred under reflux with heating for 6 hours. The solvent was distilled off under reduced pressure, and ethyl acetate and water were added to the residue. After partition, the solvent was distilled off from the organic layer under reduced pressure, and thus obtained residue was purified by silica gel chromatography (ethyl acetate → n-hexane:acetone = 1:4) to yield 7.6 g (54%) of (4S)-4-hydroxy-1-[(6-phenoxypyridin-3-yl)methyl]pyrrolidin-2-one.
$^1$H-NMR ($CDCl_3$) δ: 1.71(1 H, brs) , 2.38-2.45(1 H, m), 2.67-2.75(1 H, m), 3.17-3.21(1 H, m), 3.49-3.54(1 H, m), 4.34-4.50(3 H, m), 6.86(1 H, d, J=8.4 Hz), 7.10-7.23(3 H, m), 7.37-7.40(2 H, m), 7.59-7.63(1 H, m), 8.05(1 H, d, J=2.1 Hz).

Step 4

Methanesulfonyl chloride (2.7 mL) was added to a mixed solution of (4S)-4-hydroxy-1-[(6-phenoxypyridin-3-yl)methyl]pyrrolidin-2-one (6.0 g), triethylamine (3.8 mL) and THF (30 mL) at 0 to 10°C, and the mixture was stirred at the same temperature for 0.5 hour. To the reaction mixture was added water and the mixture was extracted with ethyl acetate. The organic layer was washed with saturated aqueous sodium hydrogen carbonate and water, and the solvent was distilled off under reduced pressure. To the concentrate, DMF (30 mL) and potassium thioacetate (4.8 g) were added, and the mixture was stirred at 50°C for 2 hours. The reaction mixture was cooled to room temperature, to which was added water, and the mixture was extracted with ethyl acetate. The organic layer was washed with saturated brine and dried over anhydrous sodium sulfate. The solvent was distilled off under reduced pressure, and thus obtained residue was purified by silica gel chromatography (n-hexane:ethyl acetate = 1:1) to yield 3.6 g (50%) of the title compound.
$^1$H-NMR ($CDCl_3$) δ: 2.31(3 H, s), 2.33-2.37 (1 H, m), 2.85-2.94(1 H, m), 3.14-3.19(1 H, m), 3.72-3.78(1 H, m), 4.00-4.07 (1 H, m), 4.33-4.48(2 H, m), 6.88(1 H, d, J=8.4 Hz), 7.12-7.26(3 H, m), 7.37-7.43(2 H, m), 7.58-7.62(1 H, m), 8.05(1 H, d, J=2.2 Hz).

Example 39

S-((3R)-5-oxo-1-[(6-phenoxypyridin-3-yl)methyl]pyrrolidin-3-yl)methylthiocarbamate

Methane sulfonic acid (1.4 g) was added to a mixture of S-{(3R)-5-oxo-1-[(6-phenoxypyridin-3-yl)methyl]pyrrolidin-3-yl)ethanethioate (1.0 g) obtained in Example 38 and isopropyl alcohol (5 mL), and the mixture was stirred at 90°C for 5 hours. The reaction mixture was cooled to 60°C, potassium carbonate (2.0 g) and isopropyl alcohol (5 mL) were added thereto, and the mixture was stirred at the same temperature for 1 hour. Tri-n-butylphosphine (59 mg) and phenyl methylcarbamate (0.7 g) were added to the mixture, and stirring was continued at the same temperature for another 1 hour. The mixture was cooled to 0 to 10°C, citric acid mono hydrate (1.5 g) was added thereto, and then water was added to the mixture while maintaining the same temperature. The mixture was extracted with ethyl acetate and washed with saturated brine. The organic layer was dried over anhydrous sodium sulfate. The solvent was distilled off under reduced pressure, and thus obtained residue was purified by silica gel chromatography (ethyl acetate) to yield 0.7 g (69%) of the title compound.
$^1$H-NMR ($CDCl_3$) δ: 2.40-2.48(1 H, m), 2.82-2.94(3 H, m), 3.23-3.29 (1 H, m), 3.75-3.81(1 H, m), 4.04-4.08(1 H, m), 4.40-4.49(2 H, m), 5.60 (1 H, brs), 6.87 (1 H, d, J=8.4 Hz), 7, 11-7.22(3 H, m), 7.37-7.42(2 H, m), 7.58-7.62(1 H, m), 8.05(1 H, d, J=2.2 Hz).

Reference Example 1

Phenyl methylcarbamate

In an ice bath, sodium hydrogen carbonate (1.6 g) was added to a mixture of an aqueous solution of 40% methylamine (6.2 g) and acetonitrile (10 mL), and then a solution of phenyl chlorocarbonate (5 mL) in acetonitrile was

added dropwise to the mixture under temperature control at 0 to 12°C for 15 minutes. After completion of the dropwise addition, the mixture was stirred at room temperature for 30 minutes. To the reaction mixture was added water (30 mL) and the mixture was extracted with ethyl acetate (15 mL) twice. The organic layer was washed with 1N hydrochloric acid, saturated aqueous sodium hydrogen carbonate and saturated brine. The organic layer was dried over anhydrous magnesium sulfate and then concentrated under reduced pressure. Thus obtained white crystals were recrystallized from diisopropyl ether/n-heptane and dried to yield 5.5 g (91%) of the title compound.

Melting Point 84 - 85°C.

The structures of the compounds from the Examples are summarized in the following table.

[Table 1]

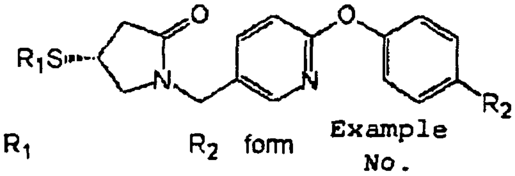

| Example No. | $R_1$ | $R_2$ | form |
|---|---|---|---|
| 1 | Ac | F | |
| 2 | H | F | HCl |
| 3 | MeNHCO | F | |
| 4 | EtNHCO | F | |
| 5 | PrNHCO | F | |
| 6 | BuNHCO | F | |
| 7 | iPrNHCO | F | |
| 8 | $EtOCOCH_2NHCO$ | F | |
| 9 | MeNHCS | F | |
| 10 | 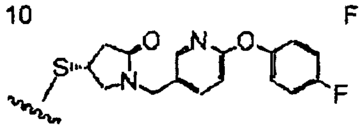 | F | |
| 11 | Ac | Et | |
| 12 | H | Et | HCl |
| 13 | MeNHCO | Et | |
| 14 | Ac | $MeSO_2NH$ | |
| 15 | H | $MeSO_2NH$ | |
| 16 | MeNHCO | $MeSO_2NH$ | |
| 17 | H | F | 2HCl |
| 18 | Me | F | |
| 19 | 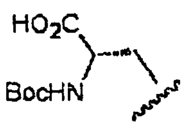 | F | |
| 20 | 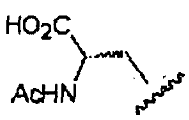 | F | |
| 21 | 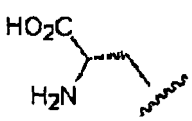 | F | 2HCl |
| 22 | $AcOCH_2$ | F | |
| 23 | $AcNHCH_2$ | F | |
| 24 | 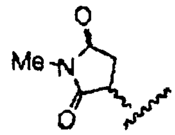 | F | |
| 25 | 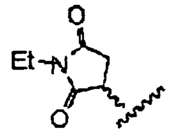 | F | |
| 26 | 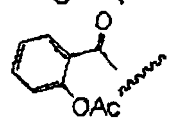 | F | |
| 27 | $HO_2C(CH_2)_3CO$ | F | |
| 28 | $AcNHCH_2CO$ | F | |
| 29 | $EtO_2C(CH_2)_3NHCO$ | F | |
| 30 | $HO_2C(CH_2)_3NHCO$ | F | |
| 31 | AllylNHCS | F | |
| 32 | MeOCOS | F | |
| 33 | 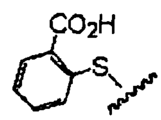 | F | |
| 34 | 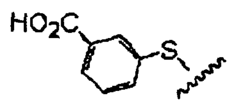 | F | |
| 35 | 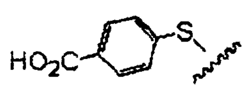 | F | |
| 36 | 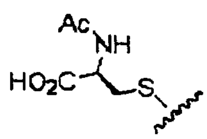 | F | |
| 37 | 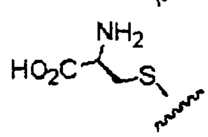 | F | HCl |
| 38 | Ac | H | |
| 39 | MeNHCO | H | |

Preparation Example 1

| | | |
|---|---|---|
| (1) | Compound of Example 1 | 50 mg |
| (2) | Lactose | 34 mg |
| (3) | Maize starch | 10.6 mg |

(continued)

| | | |
|---|---|---|
| (4) | Maize starch (paste) | 5 mg |
| (5) | Magnesium stearate | 0.4 mg |
| (6) | Calcium carboxymethyl cellulose | 20 mg |
| | Total | 120 mg |

The above components (1) to (6) were mixed by a conventional method, and the mixture was tabletted by means of a tabletting machine to give tablets.

Preparative Example 2

| | | |
|---|---|---|
| (1) | Compound of Example 2 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of 10.0 mg of Compound of Example 2 with 60.0 mg of lactose and 35.0 mg of corn starch was treated with 0.03 mL of a 10% aqueous solution of gelatin (gelatin 3.0 mg) , and the mixture was subjected to granulation through a 1-mm mesh sieve, then dried at 40°C and sieved again. Thus obtained granules were mixed with 2.0 mg of magnesium stearate and compacted. Thus obtained core tablets were coated with an aqueous suspension of sucrose, titanium dioxide, talc and gum Arabic. The coated tablets were covered with yellow beeswax to obtain coated tablets.
Preparative Example 3

| | | |
|---|---|---|
| (1) | Compound of Example 3 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound of Example 3 and 3.0 mg of magnesium stearate were mixed with 0.07 mL of an aqueous solution of soluble starch (soluble starch 7.0 mg), and the mixture was subjected to granulation, dried and then mixed with 70.0 mg of lactose and 50.0 mg of corn starch. The mixture was compacted to obtain tablets.

Experimental Example 1 MMP-13 inhibitory activity [Method]

The MMP-13 inhibitory activity was measured by a method using a fluorescent synthetic substrate. To a buffer solution for measurement (100 mM Tris-HCl, pH 7.4, 100 mM NaCl, 10 mM $CaCl_2$) containing Recombinant Human MMP-13 (manufactured by Takeda Pharmaceuticals, Inc.) activated by APMA (1 mM) in a concentration of 8 nM, the compound dissolved in DMSO was added in a predetermined concentration. Subsequently, a fluorescent synthetic substrate (7-methoxycoumarin-4-yl)acetyl-Pro-Leu-Gly-Leu-(3-[2,4-dinitrophenyl]-L-2,3-diaminopropionyl)-Ala-Arg-$NH_2$ was added thereto in a concentration of 10 μM to initiate the reaction. After incubation at 37°C for 1 hour, a 500 mM acetate buffer (pH 3.0) was added to stop the reaction. The increase in the fluorescence intensity due to substrate decomposition was measured employing a CORONA MTP-32 plate reader under the condition of Ex/Em = 340/400. The rate of inhibition of the enzymatic inhibitory activity was determined from the following equation:

$$\text{Rate of Inhibition (\%)} = 100- (X-C) / (T-C) \times 100$$

T: Fluorescence intensity of the control with addition of enzyme and without addition of the compound
C: Fluorescence intensity of the control without addition of enzyme and without addition of the compound
X: Fluorescence intensity with addition of enzyme and with addition of the compound

[Results]

The compound (3 nM) described in Example 2, the compound (1.5 nM) described in Example 12 and the compound (1.5 nM) described in Example 15 showed a rate of inhibition for MMP-13 activity of 63%, 50% and 67%, respectively.

## Industrial Applicability

Compound [I] of the present invention has excellent MMP inhibitory activity, especially MMP-13 inhibitory activity, and is useful as a safe prophylactic and therapeutic agent against MMP-related diseases, for example, articular diseases (e.g., osteoarthritis, rheumatoid arthritis, etc.), osteoporosis, cancers, periodontosis, corneal ulcer, chronic ulcers, pathologic bone resorption (Paget's disease, etc.), nephritis, angiogenesis, aneurysm, arterial sclerosis, pulmonary emphysema, chronic obstructive pulmonary diseases (COPD), cirrhosis, autoimmune diseases (Crohn's disease, Sjogren's disease, etc.), or infiltration and metastasis of cancers, or as a contraceptive.

## Claims

**1.** A compound represented by the formula [I]:

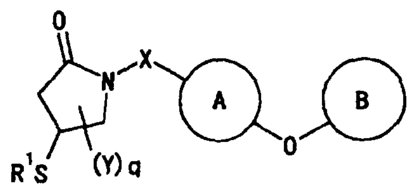

[I]

wherein ring A represents an optionally substituted aromatic heterocyclic ring;
ring B represents an optionally substituted homocyclic or heterocyclic ring;
$R^1$ represents a hydrogen atom, an optionally substituted hydrocarbon group, an acyl group, an optionally substituted heterocyclic group or $SR^2$ (wherein $R^2$ represents a hydrogen atom, an optionally substituted hydrocarbon group, an acyl group or an optionally substituted heterocyclic group);
X represents an optionally substituted divalent $C_{1-3}$ aliphatic hydrocarbon group;
each Y may be the same or different and represents an optionally substituted hydrocarbon group, a halogen atom, a carboxy group, an acyl group, an optionally substituted hydroxy group, an optionally substituted amino group, $SR^3$ (wherein $R^3$ represents a hydrogen atom, an optionally substituted hydrocarbon group, an acyl group or an optionally substituted heterocyclic group), an oxo group, a thioxo group, an optionally substituted imino group, a nitro group or a cyano group; and
q represents an integer of 0 to 5,
or a salt thereof.

**2.** The compound according to claim 1, wherein X is an optionally substituted methylene group.

**3.** The compound according to claim 1, wherein $R^1$ represents a hydrogen atom, an optionally substituted lower alkyl group, - (C=O) -$R^4$ or -(C=S)-$R^4$ (wherein $R^4$ represents a hydrogen atom, an optionally substituted hydrocarbon group, an optionally substituted amino group or an optionally substituted hydroxy group) or $SR^2$ (wherein $R^2$ has the same meaning as defined in claim 1).

**4.** The compound according to claim 1, wherein $R^1$ is (1) a hydrogen atom, (2) a $C_{1-6}$ alkyl group optionally substituted by 1 to 3 substituents selected from (i) a $C_{1-6}$ alkoxy-carbonyl group, (ii) a $C_{6-14}$ aryl group, (iii) a carboxy group, (iv) an amino group, (v) a mono- or di-$C_{1-6}$ alkylamino group, (vi) a $C_{1-6}$ alkyl-carbonylamino group, (vii) a $C_{1-6}$ alkoxy-carbonylamino group, (viii) a $C_{1-6}$ alkyl-carbonyloxy group and (ix) a heterocyclic group, (3) -(C=O) -$R^{6a}$ or - (C=S) -$R^{6a}$ (wherein $R^{6a}$ represents a $C_{1-6}$ alkyl group, a $C_{2-6}$ alkenyl group or a $C_{6-14}$ aryl group each optionally substituted by 1 to 3 substituents selected from (i) a carboxy group, (ii) a $C_{1-6}$ alkyl-carbonylamino group, (iii) a $C_{1-6}$ alkyl-carbonyloxy group and (iv) a $C_{1-6}$ alkoxy-carbonyl group), (4) - (C=O) -$NR^{6a}R^{27a}$ or - (C=S) -$NR^{6a}R^{27a}$ (wherein $R^{6a}$ has the same meaning as defined above and $R^{27a}$ represents a hydrogen atom or a $C_{1-6}$ alkyl group),

or (5) $SR^{2a}$ (wherein $R^{2a}$ represents (i) a $C_{1-6}$ alkyl group or a $C_{6-14}$ aryl group each optionally substituted by 1 to 3 substituents selected from (a) a carboxy group, (b) an amino group and (c) a $C_{1-6}$ alkyl-carbonylamino group, or (ii) a $C_{1-6}$ alkoxy-carbonyl group).

**5.** The compound according to claim 1, wherein ring A is an optionally substituted pyridine ring.

**6.** The compound according to claim 1, wherein ring A is the following formula:

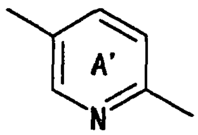
or
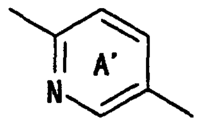

wherein ring A' represents an optionally substituted pyridine ring.

**7.** The compound according to claim 1, wherein X is a methylene group optionally substituted by 1 or 2 substituents selected from (1) a halogen atom, (2) a $C_{1-4}$ alkyl group or $C_{1-4}$ alkoxy group each optionally substituted by 1 or 2 substituents selected from (i) a halogen atom, (ii) $C_{1-4}$ alkoxy group, (iii) a nitro group, (iv) a cyano group, (v) a hydroxy group, (vi) an amino group, (vii) a mono- or di-$C_{1-4}$ alkylamino group, (viii) a carboxy group, (ix) an $C_{1-4}$ alkoxy-carbonyl group and (x) $C_{1-4}$ alkyl-carbonyl group, (3) a nitro group, (4) a cyano group, (5) a hydroxy group, (6) an amino group, (7) a mono- or di-$C_{1-4}$ alkylamino group, (8) a carboxy group, (9) a $C_{1-4}$ alkoxy-carbonyl group and (10) a $C_{1-4}$ alkyl-carbonyl group, an oxo group or a thioxo group;

$R^1$ is (1) a hydrogen atom, (2) a $C_{1-6}$ alkyl group optionally substituted by 1 to 3 substituents selected from (i) a $C_{1-6}$ alkoxy-carbonyl group, (ii) a $C_{6-14}$ aryl group, (iii) a carboxy group, (iv) an amino group, (v) a mono- or di-$C_{1-6}$ alkylamino group, (vi) a $C_{1-6}$ alkyl-carbonylamino group, (vii) a $C_{1-6}$ alkoxy-carbonylamino group, (viii) a $C_{1-6}$ alkyl-carbonyloxy group and (ix) a heterocyclic group, (3) - (C=O) -$R^{6a}$ or - (C=S) -$R^{6a}$ (wherein $R^{6a}$ represents a $C_{1-6}$ alkyl group, a $C_{2-6}$ alkenyl group or a $C_{6-14}$ aryl group each optionally substituted by 1 to 3 substituents selected from (i) a carboxy group, (ii) a $C_{1-6}$ alkyl-carbonylamino group, (iii) a $C_{1-6}$ alkyl-carbonyloxy group and (iv) a $C_{1-6}$ alkoxy-carbonyl group, (4 ) - (C=O) -$NR^{6a}R^{27a}$ or - (C=S) -$NR^{6a}R^{27a}$ (wherein $R^{6a}$ has the same meaning as defined above and $R^{27a}$ represents a hydrogen atom or a $C_{1-6}$ alkyl group) , or (5) $SR^{2a}$ (wherein $R^{2a}$ represents (i) a $C_{1-6}$ alkyl group or a $C_{6-14}$ aryl group each optionally substituted by 1 to 3 substituents selected from (a) a carboxy group, (b) an amino group and (c) a $C_{1-6}$ alkyl-carbonylamino group, or (ii) a $C_{1-6}$ alkoxy-carbonyl group) ;

ring A is a pyridine ring optionally substituted by 1 to 3 substituents selected from (i) a halogen atom, (ii) an optionally halogenated $C_{1-4}$ alkyl group and (iii) an optionally halogenated $C_{1-4}$ alkoxy group;

ring B is a benzene ring optionally substituted by 1 to 3 substituents selected from (i) a halogen atom, (ii) a $C_{1-6}$ alkyl group and (iii) a $C_{1-6}$ alkylsulfonylamino group;

Y is (1) a $C_{1-6}$ alkyl group optionally substituted by (i) a hydroxy group or (ii) a $C_{1-6}$ alkoxy group, or (2) an oxo group; and

q is 0 or 1.

**8.** The compound according to claim 1, wherein the formula [I] is represented by the following formula:

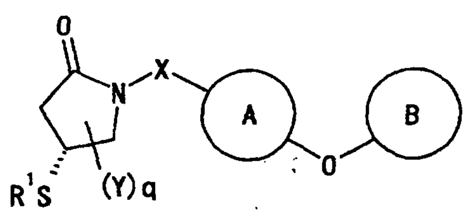

wherein each symbol has the same meaning as defined in claim 1.

**9.** (4R)-1-{[6-(4-fluorophenoxy)pyridin-3-yl]methyl}-4-mercaptopyrrolidin-2-one or a salt thereof,

S-((3R)-1-{[6-(4-fluorophenoxy)pyridin-3-yl]methyl}-5-oxopyrrolidin-3-yl)methylthiocarbamate or a salt

thereof,

(3R)-1-{[6-(4-fluorophenoxy)pyridin-3-yl]methyl}-5-oxopyrrolidin-3-yl methyl-(dithiocarbamate) or a salt thereof,

(4R)-1-{[6-(4-ethylphenoxy)pyridin-3-yl]methyl}-4-mercaptopyrrolidin-2-one or a salt thereof,

S-((3R)-1-{[6-(4-ethylphenoxy)pyridin-3-yl]methyl}-5-oxopyrrolidin-3-yl)methylthiocarbamate or a salt thereof,

N-{4-[(5-[(4R)-4-mercapto-2-oxopyrrolidin-1-yl]methyl)pyridin-2-yl]oxy}phenyl}methanesulfonamide or a salt thereof,

S-{(3R)-1-[(6-(4-[(methylsulfonyl)amino]phenoxy}pyridin-3-yl)methyl]-5-oxopyrrolidin-3-yl}methylthiocarbamate or a salt thereof, or

(3R)-1-((6-(4-fluorophenoxy)-3-pyridinyl)methyl)-5-oxo-3-pyrrolidinyl aryldithiocarbamate or a salt thereof.

**10.** A method for producing a compound represented by the following formula:

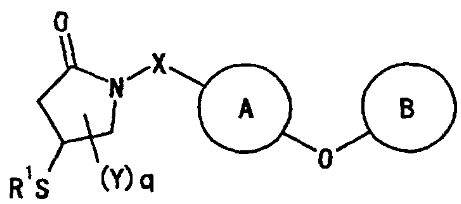

wherein each symbol has the same meaning as defined in claim 1,
or a salt thereof, comprising reacting a compound represented by the following formula:

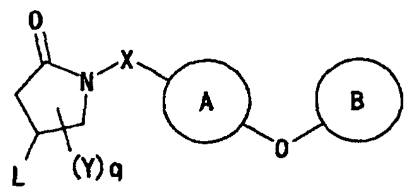

wherein L represents a leaving group and each of the other symbols has the same meaning as defined claim 1, or a salt thereof with a compound represented by the formula:

$$R^1SH$$

wherein $R^1$ has the same meaning as defined in claim 1, or a salt thereof.

**11.** A method for producing a compound represented by the following formula:

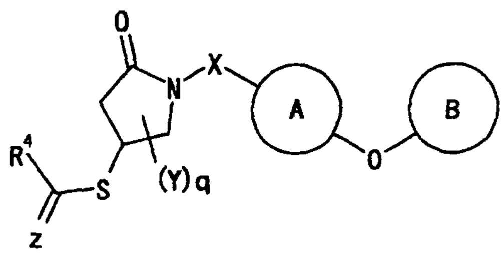

wherein Z represents O or S and each of the other symbols has the same meaning as defined in claims 1 and 3, or a salt thereof, comprising reacting a compound represented by the following formula:

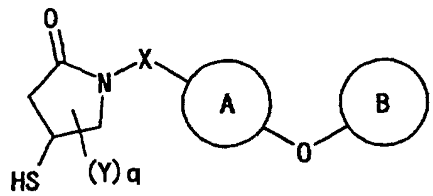

wherein each symbol has the same meaning as defined in claim 1,
or a salt thereof with a compound represented by the formula:

$$R^4\text{-}CO_2H,\ R^4\text{-}COSH \text{ or } R^4\text{-}CSOH$$

wherein $R^4$ has the same meaning as defined in claim 3, or a salt or a reactive derivative thereof.

**12.** A method for producing a compound represented by the following formula:

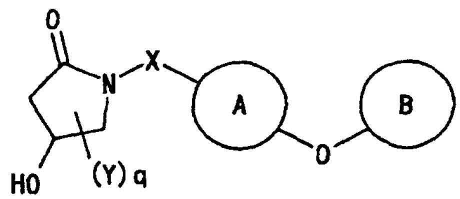

wherein each symbol has the same meaning as defined above,
or a salt thereof, comprising reacting a compound represented by the following formula:

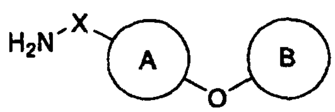

wherein each symbol has the same meaning as defined in claim 1,
or a salt thereof with a compound represented by the following formula:

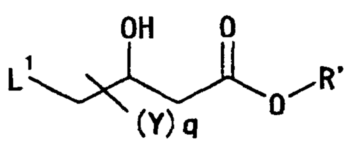

wherein $L^1$ represents a leaving group and R' represents an optionally substituted hydrocarbon group, an acyl group or an optionally substituted heterocyclic group,
or a salt thereof.

**13.** A compound represented by the following formula:

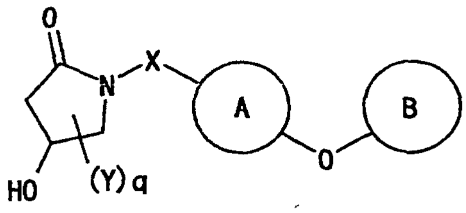

wherein each symbol has the same meaning as defined in claim 1, or a salt thereof.

**14.** A prodrug of the compound according to claim 1.

**15.** A pharmaceutical composition comprising the compound according to claim 1 or a prodrug thereof.

**16.** The pharmaceutical composition according to claim 15, which is a matrix metalloprotease inhibitor.

**17.** The pharmaceutical composition according to claim 15, which is a prophylactic and therapeutic agent for matrix metalloprotease-related diseases.

**18.** The pharmaceutical composition according to claim 15, which is a prophylactic and therapeutic agent for articular disease, osteoporosis, cancer, periodontosis, corneal ulcer, chronic ulcer, pathologic bone resorption, nephritis, angiogenesis, aneurysm, arteriosclerosis, pulmonary emphysema, chronic obstructive pulmonary disease (COPD), cirrhosis, autoimmune disease, or infiltration and metastasis of cancer, or a contraceptive.

**19.** The pharmaceutical composition according to claim 18, wherein the articular disease include osteoarthritis or rheumatoid arthritis.

**20.** A method for preventing and treating articular disease, osteoporosis, cancer, periodontosis, corneal ulcer, chronic ulcer, pathologic bone resorption, nephritis, angiogenesis, aneurysm, arteriosclerosis, pulmonary emphysema, chronic obstructive pulmonary disease (COPD), cirrhosis, autoimmune disease, or infiltration and metastasis of cancer, or for contraception, comprising administering an effective amount of the compound according to claim 1 or a prodrug thereof to a mammal; and

**21.** Use of the compound according to claim 1 or a prodrug thereof for producing a prophylactic and therapeutic agent for articular disease, osteoporosis, cancer, periodontosis, corneal ulcer, chronic ulcer, pathologic bone resorption, nephritis, angiogenesis, aneurysm, arteriosclerosis, pulmonary emphysema, chronic obstructive pulmonary disease (COPD), cirrhosis, autoimmune disease, or infiltration and metastasis of cancer, or a contraceptive.

## INTERNATIONAL SEARCH REPORT

International application No.
PCT/JP03/05255

**A. CLASSIFICATION OF SUBJECT MATTER**

Int.Cl[7] C07D401/06, 401/14, A61K31/4439, A61P1/02, 1/04, 1/16, 9/10, 11/00, 13/12, 19/02, 19/10, 27/02, 29/00, 35/00, 37/02, 43/00

According to International Patent Classification (IPC) or to both national classification and IPC

**B. FIELDS SEARCHED**

Minimum documentation searched (classification system followed by classification symbols)

Int.Cl[7] C07D401/06, 401/14, A61K31/4439, A61P1/02, 1/04, 1/16, 9/10, 11/00, 13/12, 19/02, 19/10, 27/02, 29/00, 35/00, 37/02, 43/00

Documentation searched other than minimum documentation to the extent that such documents are included in the fields searched

| | | | |
|---|---|---|---|
| Jitsuyo Shinan Koho | 1926-1996 | Jitsuyo Shinan Toroku Koho | 1996-2003 |
| Kokai Jitsuyo Shinan Koho | 1971-2003 | Toroku Jitsuyo Shinan Koho | 1994-2003 |

Electronic data base consulted during the international search (name of data base and, where practicable, search terms used)

CAPLUS, REGISTRY(STN), JSTPluS/JMEDPlus(JOIS)

**C. DOCUMENTS CONSIDERED TO BE RELEVANT**

| Category* | Citation of document, with indication, where appropriate, of the relevant passages | Relevant to claim No. |
|---|---|---|
| X<br><br>Y | EP 1132379 A1 (Takada Chemical Industries, Ltd.), 12 September, 2001 (12.09.01), Full text; particularly, Par. No. [0014]<br>& WO 00/17162 A1 & CA 2344412 A<br>& AU 9956637 A1 & JP 2000-159747 A<br>& US 6420415 B1 & US 2003/078253 A1 | 1-11,13-19, 21<br><br>12 |
| Y | JP 01-045360 A (Denki Kagaku Kogyo Kabushiki Kaisha), 17 February, 1989 (17.02.89), Claim 2<br>(Family: none) | 12 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 May, 2003 (27.05.03) | 10 June, 2003 (10.06.03) |
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP03/05255

**Box I Observations where certain claims were found unsearchable (Continuation of item 2 of first sheet)**

This international search report has not been established in respect of certain claims under Article 17(2)(a) for the following reasons:

1. ☒ Claims Nos.: 20
because they relate to subject matter not required to be searched by this Authority, namely:
Claim 20 pertains to method for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)